# EUROPÄISCHE PATENTANMELDUNG

(11) **EP 3 456 830 A1**
(43) Veröffentlichungstag der Anmeldung: **20.03.2019**
(21) Anmeldenummer: 18197772.9
(22) Anmeldetag: 06.08.2013
(51) Int. Cl.: C12N 15/82

(54) **TRANSGENE PFLANZE DER ART SOLANUM TUBEROSUM MIT RESISTENZ GEGENÜBER PHYTOPHTHORA**

(30) Priorität: 08.08.2012 DE 102012016009
(62) Teilanmeldung aus: 13759425.5
(71) Anmelder: KWS SAAT SE, 37574 Einbeck (DE)
(72) Erfinder: STAHL, Dietmar, 37574 Einbeck (DE); TEMME, Nora, 37574 Einbeck (DE)

(57) **Zusammenfassung**

Die vorliegende Erfindung betrifft eine transgene Pflanze der Art *Solanum tuberosum* mit einer Resistenz gegenüber einem Oomyzeten der Gattung *Phytophthora,* transgene Teile einer solchen Pflanze, Verfahren zu deren Herstellung sowie ein Mittel zur äußeren Applikation bei Pflanzen. Zum einen werden Nukleotidsequenzen gemäß den SEQ ID NOS: 1 - 43 aus *P*. *infestans* in einem Wirtspflanzen-vermittelten Gensilencing-Ansatz in Kartoffelpflanzen, zum anderen als Fungizid zur Pflanzenbehandlung bereitgestellt.

SEQUENCE LISTING

<110>  KWS SAAT SE

<120>  TRANSGENE PFLANZE DER ART SOLANUM TUBEROSUM MIT RESISTENZ
       GEGENÜBER PHYTOPHTHORA

<130>  KWS0199PCT/EP/D1

<150>  DE 10 2012 016 009.7
<151>  2012-08-08

<150>  EP13759425.5
<151>  2013-08-06

<160>  50

<170>  PatentIn version 3.5

<210>  1
<211>  1042
<212>  DNA
<213>  Phytophthora infestans

<400>  1
cgtcaagatg ctgcggaggt ctgtgctgcc gttggcacgt cgtgccttcc cgcggtccgc      60

ggctagccta gcgccgtcct gtgctgctcc caagtacttt tcacgtgcct tcagcgttgc     120

ggagcagagt cagcggcatg tgatcgccgc gctggttgtc aaccagcctg gttgtctggc     180

cgagatcgcc aacctcttcg ccgctcgagg taactatcga tacgcaaaga cgctggccac     240

aggagatcct aaacatggtg tgatgcaggc tacaatattg acagtctggt tgtgggtcgc     300

acggaggtcg aggagctctc ccgtatgacg gttgtcgtca acggcactgc gcagagtgtt     360

gtcaacgtac gtatagtaat tacagagaca ggagcagtag gtgattgaca ctcaatgtgt     420

ctttgcagat gaagaagcag ctcgaggatg tcgtgtatgt tgctgtggtc aatatcctga     480

gcagtggcaa gaacgctgaa aagaactacg tcgagcgcga cctgatgctg gccaaggtgt     540

ccacggccgg tacgtcggac gctgatctga taccctacca ccaccggaat tcgtgaacat     600

ggacctgacg taaacatttg ctggtgtgtg tgctggctac agaggctgga tctcgcgctg     660

aggtggtgga gcttgccaac ctgttcgacg ccaaggtgat tgacgtgcga ccgcaccagg     720

ttatggtaca actggctggc acccctggtc gcattgaggc atttttggac ctgctaaagc     780

cgctgggtat cacagagatc caccgcagtg gggtcattgc gatggctcgc agcaccagtg     840

tgaccgacga cctgggcgat ctctcgacgt ttgagggcgc cacgcgaacg cttctggacg     900

aggccgatga cgaagagttc gatgtctctc gccttccgcc tggataatat gttggtagag     960

ttatgatcat gccatcacct tttcttcgaa taggacttgt gcctgatgac aattgcagca    1020

aggaacgtgc tagcgaagat tg                                             1042

<210>  2

<211>   3072
<212>   DNA
<213>   Phytophthora infestans

<400>   2
atggtgctcc gagcggttcg gctgatcgtc caggcatctc tgctcctgca agtgctccag      60

tgtggcgcct ccgtaactgg tgctcaagta aatgaaaact ttgagaacac tgagcttacc     120

tcgaacgata aactgggaac agtggctccg gccgacatcc cgtcttcaca agacgagaat     180

ctcaagaaac aggaagagcg tggcttcttt gactggtttg gcaatggcga cgacacgcct     240

gccccggctg ctgacgacaa ctccggtaaa tatgcgacta cgacaccgat ccctggcacg     300

gctgcgcggc cgtctacgag tagtctgatg tcaaagtatg gtagtatgct cggtgacttt     360

actaaagaca cgacgccggc ctcggaagcc gatgagcgca caacgagcgc acgtagcgct     420

gccacccgtg ccacctcgga ctcctcttca ggtagtacct ctggcagcgc ggcagcagaa     480

gtcaagccga agagaaagaa gaccaagaag cctgtggttg ccgccgcgtc tgaaagcgga     540

gagagtgcaa gtgacgacga tgccagcgag gcgagcgctt cgggcagtga ggcaaagccc     600

aagaagagga caaccaagaa gacgaggaag ccggtcgtag ctgctgcgtc gacgtctgaa     660

tccgaggatg cctcgacgtc tgaatccgag gatgcttcgg gcagtgaggc aaagcctaag     720

aagaagacaa ccaagaagac tagaaggccg gtagcagctg ctgcatcgac gtctgaatcg     780

gaggatgctt cgggcagctc ggacgagtcg ggaagcgacg atctgtcgga tctgctcggc     840

agctcggcgg gttccggaag cgaagatgac atgtcggccc ttctcggtag cgcgggaggt     900

tccgggactg acgacgcctt atcagctctg ctggggtcgg gagtcggtgg ctcgggtagt     960

atgcttagtt tcgaagactt tatgaagaat tacggtagca tgttcggttc gggaagtagt    1020

gctgtcgacc tgttcggaga cccgagcacg ccccagaaca acacgattga cgacgaagat    1080

atcatcctcg gcgaactgta cggtggcaag gagcacggcg acgccttctc ggacatcagg    1140

aacatcaagt ttggtcagat gatcctgaac attacagttc gcggtcaaga gcgcgtggat    1200

tccattggta ttacagtgat gactcaagaa gctgttggta accttgtgca cggtggtgaa    1260

ggtggtaccg aaggattcat tgaaccggag atgggtgaca ctattgatac cgtcgaagta    1320

cactgggaca agaacaaggg caagacgtgc atcttctacc tcaagatggc gacttctggc    1380

ggtaagacga ttgcgacggg aacgaagacg gccaacagcg ccgtcatcaa gccgcccaag    1440

ggctaccagc ttgctggatt ccatggtcgt gccagtagtt ctggtatctt ttgtattggt    1500

ggaatcttca ccaagcaaga cgcgacggat ctcgcagtca cggacgtgat ggccatctcc    1560

agtaagggct ctcctgatat ctacaactac gacaccacca ttcgtaactg ggtgggacct    1620

ctagagacag cgagtgacaa cgcctgttac cagaagagag tcgacgtcag cagcaaggga    1680

atgtgtccgt cgggtttcaa caaggacgac gacaggtgca tcacccaatg tcctctcaac    1740

taccccattg actgcttgat ggagtgcatg cctcaaaaca gtgactgcac ccagttgatt 1800

gtcgctaagg tttccgccgt cgttgctgtc gctttaaatg ccgctacgat gggtatcttc 1860

ggtacgctgg tggctgccta cagaaccgct aactttgctc tcacctgcgc catcaacgtt 1920

gtgaacgctg tcaagtcgct gatttactac ctgcgttaca agcagacctt gatcccgact 1980

acggacacgg agaagttgat ggacaaggcg ttccagctgc agattgtcat tcttgacttg 2040

cctctggcta tctgttcttg cctgggcatc aagattcctc ctaagctcca gttctcggct 2100

accattctgg ctgtcgtgtc ggccattgtc atgatggctg tcatggtcgg tgaggccctc 2160

ttcgcgtcgt cgaacaacgt catgctcatg cttcgtgagt cgggcgcttt taacacttct 2220

gctctgaacg gagacaccat tgagcttgat acgttcctca acaccaagaa cggcacgtgt 2280

ggttacgaga tgagaactct cactaaccgc gtcatgggca aggtctacga aatccgtaac 2340

aacacgccga atgctgatgc cgatgatgta cgtgttgagg tgagcaagtc gtccatcatt 2400

acggatgaca tccccattgt gaccaaccac tgcatgggtg atatctggac caacaagacg 2460

ggcgcgtcgt cgtacaagac gcgcaacctg ctgcgtaaga ccctcagtgt aattgttgac 2520

cagctcgttg aggacggtac gaccgatatg ggtaagcatg tgaccaagaa ggagaaggct 2580

ctcgaatact cgaatatggg tcttttcgtg ctgtccatgt tcgatccgac gggtattgcc 2640

tggatggctt ccgagttcgt gcagcccatt tgtggaccca ctgagtacct gggtgagatc 2700

gatgatggta cgctgtacga cgctctgggt ctaaacacgg tcgaccaggc gttcctggga 2760

agctacggtg tgtggaagaa gaagggtgat ggctccgtca cggtttactt cgagagtgtt 2820

gacaagttcc ctgtgtctgt ggtgatcacg tccggtggtg acaagctcaa ggaggtcaag 2880

gtgcctgcta acggcaacgt cacgtggaca tctacggtgg aggagcttgg tgacaagact 2940

ctttaccttg accgctggcg tcctggtctc tttggcctgc ctggtacggg cggtggttcg 3000

ctactgatgt ggatcccgcg atcgtctgag ggtggccagc ttgttcttca tgctcgcttg 3060

aatgttagct aa 3072

<210> 3
<211> 2405
<212> DNA
<213> Phytophthora infestans

<220>
<221> misc_feature
<222> (949)..(1048)
<223> n is a, c, g, or t

<400> 3
atgtcatccg tagacttgag tatcctgcgc aacggcatcc cggccgagct gccggcgcac 60

ccgggcaacc accccgaccc gacgctgccc aaggctccgc accgcaacat cgacggtctg 120

tccaaggacg atctcgtgct ggccgtgcag aacgcgttac ggtacttccc cgagaggttc 180

cacgccactc tggctcccga gttcgcgcag gaactcaagg acgagggcca catctacatg 240

caccgcttcc gtcccgtgca gtacgagatg aaggcgtacc ccattgacca atacccggcc 300

aaatgcaaac aggcagctag tatcatgctt atgatcatga acaacctcga ccgtcgcgtt 360

gcacagttcc ccaaccacct ggtcacttac ggtggcaacg gtagtgtctt ccagaactgg 420

gcacagtact tggtggccat gcgctacctg tccgaactca cggaacagca gacgctcgtc 480

atgtactctg gtcaccctat gggtttgttc cctagtcgtc ctgcagctcc acgtatggta 540

gtgaccaacg gtctaatgat ccccaactac tccacacgcg ctcagtacga caaggcctac 600

gctatgggta acacccagta cggtcagatg actgctggca gctactgcta cattggtccg 660

cagggtattg tacacggtac gaccattact gtactgaaaa cctactgacg aggtctatgg 720

cggttcagat gagagcaatc taccttcctt ctagaaaaat gctaaacaat tccagtggat 780

gttgtatagg tcttgacaat caatattgcc ccctacgatt ttgtcaatat taacaatcag 840

tttatttctt ccaatccctg attttacagt gaggatttcg agagtgggaa cgtggaaaca 900

aaatcattgt caaatttgag cgagcctttc tcgccgatgc cagactggnn nnnnnnnnnn 960

nnnnnnnnnn nnnnnnnnnn nnnnnnnnnn nnnnnnnnnn nnnnnnnnnn nnnnnnnnnn 1020

nnnnnnnnnn nnnnnnnnnn nnnnnnnngc gtcatctccg gtgtcatctc cgtcactgca 1080

gagatcgacg aatctgcagt gaagaagcgt cacgaacagg gctgggttga cgaggtcgtg 1140

agcgacctgg acgcctgtgt cgttcgtatc cgtgaggcaa aagccaaggg cgaggtggtg 1200

agcttggcgt accacggcaa cgtcgtcact ctttgggaac gtctcgccga cgaggctgaa 1260

gctacgggcg agctgctcgt ggagctcggc tcggaccaga cttccctaca caacccgttc 1320

aacggcggct actacccggt ccagttgtcg ttcgaggagt ctcagcgcgt catggctgaa 1380

gacccggagc gcttccagga actggtacag gagtctctgc gtcgtcacgc tgtcgctatc 1440

aaccgtctga cagccaaggg catgcggttc tgggactacg gtaactcgtt cctattggag 1500

gcacaacgtg ccggcgcgga cgtgttgcgt cccggtgtta agcccgagga ggctgctgtc 1560

tcgactactg cctttaagta ccctagttac gttcaggaca tcatgggcga cattttctcg 1620

cttggtttcg gtccgttccg ctgggtctgt acgtcgggtg accacgcgga cctgcagaag 1680

acagacgcca ttgctgcccg tgtgatgcgc gagctgctgg ccgaaccgga agtgccggac 1740

cgggtcgcgg ctcagctgcg tgacaacttg cgctggattg aggctgcaga ggagaacaaa 1800

ctggtcgtgg gatcggaggc gcgtatcctg tacgctgacc gtgtgggtcg cggcacgatc 1860

gccatggcgt tcaacgccgc tgtggctagc ggcgagctct cggcacccgt cgtgctcagc 1920

cgcgaccacc acgacgtcag cggcaccgac agtccgttcc gtgagacgtc gaatgtgacg 1980

gacggctcgg ctttctgtgc cgatatggcg gtgcagaacg cgctgggcga cgccgctcgc 2040

ggagctacgt ggatcgcact gcacaacggc ggcggtgtcg gctggggcga ggtcatgaac 2100

ggtggcttcg gaatggtact ggacggctcg gatgacgcgc gcgagaaggc ggcgtgcatg 2160

ctcggctggg acgtcaacaa cggcgtggca cgtcgtgcgt gggcgcgcaa cgccaacgcg 2220

cgcttcgcta ttgagcgcga gatgaaggcg gaccctttgg tacgtttgtt tggttacgag 2280

cttgagactt tggaagcgtt agattttaat gtgtgtgtat ctttgtggta ttgcagctga 2340

cggtgacgct ggctaacgag gccgacgacg cgagcgtgcg tgacgctgtg agcaagctct 2400

tctag 2405

<210> 4
<211> 1735
<212> DNA
<213> Phytophthora infestans

<400> 4
atgctctcgc agcttaaacg cagattcacc ctgggacgac gttgtcagac cgaagaagaa 60

gaccactcga ggatcaccat gagcctttcc aacagcaaca gtaccagctc cttgccccct 120

gtggacgagc accacggctg tgagtacctg gatacggcgc tgacgatctt tgtaatcggc 180

gcgtcgggcg atctggccaa gaagaagacg tatccgtcgc tctttgcgct ctacaccatg 240

ggctacctgc ctgaacacgc ggtcatcgtg ggctacgctc gcagcgccaa gaatgatgcc 300

gacttccgcg cgcaaattgc gccctggatc aagcctaaga cacccgaggc cgaggctcgc 360

aaggaggcct tcctcaacaa gtgcatctac cgcagcggca aatacgactc aactgaagat 420

gtgggcaagg taagcaagga gatggaggca ttggaagaag cccatggatc gcctgtggcc 480

aaccgcctct tctacttcgc catcccgccc acagtcttcg tgcccatcgg cacgagcatc 540

aagaaggcgg cactgaccac gcgtggttgg aaccgtctca tcgtcgagaa gccatttggc 600

cacgatctcg actcgttcga caagttgtct caggacatgg gcgcgctgta cagcgaagac 660

gagatctacc gtatcgatca ctacttgggc aaggaaatgg tgcagaactt gctcgtgttg 720

cgcttcggca atgcaatctt cgagcccatt tggaaccgca actacgtgtc cagtgtgacc 780

atcactttca aggaagacat cggcactcag ggccgcggtg gctacttcga ctcgttcggt 840

atcatccgtg acgtcatgca gaaccacctg cttcaggtgc tgtcacttgt ggccatggag 900

ccaccaatcc aagctgctgg tgacaactac tccaactata tccgtgatga gaaggtcaag 960

gtgcttaact gcattgagcc tatcaagatc gagaacaccg tcctgggcca gtatgaaggc 1020

agcaaggagc tcaacgagcc gggctacctc gaggacccga cggtgcccaa gggatcagtg 1080

acccccacct tcgccacagc tgttatgtat gtcaacaacc cgcgttggtc tggtgttccg 1140

ttcatcatga aggctggtaa ggccttgaac gaacgcaagg gtgagatccg tgtgcaattc 1200

cgcccgcctc ctggagcgca gcacttgttc ccaggtgtca agatcccagt acaagagttg 1260

gtgctgcgtc tacagccgga ggaagccgtc tacttgaaga tgaacgtcaa gagtcctggt 1320

ctgcagaccc aggcgatctc aagcgagctg gacttgtcgt acgccgagcg ttacgagggt 1380

gcagaggtgc cggacgccta cactcgcttg atcctggacg tgctgcgtgg taagcaggcc 1440

gcattcgtgc gtgatgacga gctccgtgct gcatggaaga tcttcacgcc attgctgaac 1500

gagattgaga cgcagaaggt gaagccgctg cagtatacgt tcggctcgcg tggccccaag 1560

gagagcgacg agctggtgaa cagagctggc ttccagtacc accagggcga ataccagtgg 1620

cagccgcgtg tgcgcactac cagtgcacta taggttgtct ctttggttga gcacaaagat 1680

gcccggaagt ctggcataaa tatgatggtt gagccagcta gtcttcaata aggga 1735

<210> 5
<211> 1245
<212> DNA
<213> Phytophthora infestans

<400> 5
gggagcttaa aagtgctaca ggaagtcgct atcatggcgt caagaagtct cgtccgccgc 60

tcggcgcgtc tcgccactgc catcgctact aagcgcagct tcgttcgcgc cgcttccgcg 120

tcggtgaacc tccagtgctt tcagacagca cccgccactc gccagacccg tcttcagagt 180

acgcagacaa gctccagcga tgagccgccc aaaacggagt ccccagtaga cccagagcag 240

ctcattctgg ccaaggctct ggaccatgtg atttcccacg ggtacgttat ggtttaagtg 300

ctataaaagc gaagatatcc taggttgtaa cgctctcgaa tgcaaggtgg gccattgagg 360

ctctagcagc tggcgccacg gatcttggct acccttcggt ggctcacggc atgttccagc 420

gcggagctat tgatttggtg gattatttta tggactcgtg tctcgccaag ctacgcgaga 480

cgctgattgt caacacggaa aagttgcagg ccatgacggt gactgaacgt ctcaagtttg 540

gcgtctgcac gcgtctgcag atgctggagc ctgtgttggc tacatggcca caggctatgg 600

ccattggcgc tctaccgcag aacgcacctg gtactgcaaa gagactggcc cagctctcgg 660

atgaaatctg gtacttcgct ggtgacaagt ccacggatct atcatggtac accaagcgcg 720

ctattctcac gggcatctac gctagcacgg agctgtttat gctgaatgac aagtcgccga 780

acttccagga cacgtgggat ttcttggacc gccgtgtgga cgaaacgatc caactcggag 840

aactgcctca gaacgtacgt gaagctgttt gagtctctta taaggcttag ctgttgacgt 900

gactaactac ttgtgtgctg gtctgtgtct gagcagctga acgatgtggc tgggatggcc 960

agtattgggc tgcagtcggt gttttcggct gtgacgtcgc ttgcgggtcc tctggccagt 1020

cagatcatct cgaactcgcc tttgagtcaa gttccgaacc cgatttcagc tgtgggcagt 1080

gtggttcctc cctcggttgt atcggctgtt gcctctggaa tgccatttag caatcctact 1140

tctgctggac atgacggtat ggcgttcaag tcgaaggatc tggacgaaat taaccaagag 1200

cttgagaagc tcggcggcct tgacgcgagt gaacgacgga actaa 1245

<210> 6
<211> 1461
<212> DNA
<213> Phytophthora infestans

<400> 6
ccacgtccag ctcaagccaa ttgccatgct gtccgctact cgccgcctta cgcgatctct 60

gcgtcgccct tctgcactgg gatgccgcct cgagtcctca ttcgctcccc tcactcagct 120

atccgaggag gaaaccatgt tcaaggacac agtcgcccgg ttcgcggctg acgtggtagc 180

tccgaatgtc cgcgccatgg acacagcggg ggagatggac catgctatca cacgaggact 240

attcgagaat ggactgctgt cggtagagat cccagcagac tacggaggta gcgaggcctc 300

gttcatgaac ctctgcttga ctattgagga gctgtccaaa gtggatcccg tcgtgggact 360

gctcgtggat ctacagaaca cagttgtgaa caacgtcttc ctggtgagtg aagagtacga 420

ttggtattct aaattctgta gctgaccatt gtgttgcagg tgcacggcac ggacgagcag 480

aaagaaaagt acctgccgag actcagcgcg gacatggtgc gtgcatcaat atttggaaat 540

attgtgataa tactaatatt gtacggtgtt gtagatcggc agtttctgtt tgtcagaggc 600

tggatcaggt agtgacgcgt tcgcgctgaa gacacgggcg gaggcctcgc cggacgggag 660

ctactactcc atcactggtc agaagatgtg gatctccaac gccgagtact ctggcgtgta 720

cttggtgttc gccaatgtgg acccgtccaa gggctacaag ggtatcacgt gctttatcgt 780

ggaccgagac atggaaggac tggagatcgg taagcccgaa gagaagctcg gcatccgcgc 840

atcgtcgaca tgtcccgtca cactgacgga tgtgaaggtc ccgaaggaga acattctggg 900

agagctgggc aagggataca agatcgcaat cagcacgttg aatgaaggac gcattggcat 960

tgcgtcgcag atgctgggac tggctcaggg agtctacgac cagacgttgc cgtacttgtt 1020

cgagcgccaa cagttcggct ctcccatcgg agagttccag gcgatgcagc accaatatgc 1080

ggaggcagcg ctcgatatcg agacggcgcg tttgttggtg tataacgcag cgcgtctcaa 1140

ggatgctggc caaccgttcg tcaaacaggc tgctatggcg aagcttcatg cctcgcgtgt 1200

ggcagagaag acggcatcca agtgcatcga gctgcttgga ggcattggct ttaccaagta 1260

cttgcttgcg gagaaattct atcgtgacgc aaagatcgga gctatctacg aaggaaccag 1320

taacatgcag ctgacgacga ttgcgaagct tgtgtcggag gaatacaaga ggtaattgaa 1380

tgtcgtgatc ctgtactcag acttgatcct tgtagcaaac caaacaagta acatttccgg 1440

agaacgagcg ttagaaccag a 1461

<210> 7
<211> 1580

<212>  DNA
<213>  Phytophthora infestans

<400>  7
atgctgcgtc gtcttgccct ccgtaccgtg aagccttctg tgcgcaactt cgcctccgcc      60

tcggagctgt acaagaaccc gcgcagctcc aagttcagca tgaccaagat cgtggccact     120

atcggccccg tgtcggagca gctggacatg ctacagaaga tcacggacga aggtttgcgt     180

atcatgcgca ttaacttctc acacgccgag tacgacgagg ccttgctgcg cgtcactaac     240

cttcgtgcct gtcgcggcgt gcacgccgag ggtcaggcca aattcaacct acgtgccgtg     300

ctacacgaca ccaaaggacc cgagatccgt acgggtaaga tgcgtgacgg caagaaaatc     360

acgttagaga gtggcaagtc ggtggtgctc acgaccgacc cggctttcga gcttgagggt     420

accgccgaca agttctacgt tacgtatcag cagttagccg aaaccgttaa ggtgggcgat     480

acagtgttac tatctgacgg ccttatccgt ctcacctgta cgtcagtggg taaggatgag     540

attacgtgcc acatccacaa taccgaggaa atcggtaacc gtaagggtgt aaacctcccg     600

ggtttgatcg tcgagttgcc tgctctatct gataaggaca aacgcgacct ggattggggt     660

gtcgagcacg atatcgactt tatcgcggcc tcgttcatcc gtaaggccag cgacgtgaac     720

tcgatccgtt cgttcgtggg agagtgcgtt aagaaacact ggggtaacca acctggttac     780

attgccccta agatcatctc taagatcgag aacctcgaag gtatccagaa ctttgaggag     840

atcctcgagg cctcggatgg catcatgtgt gcccgtggcg atttgggtgt cgaagtgccg     900

gcacagaagg tcctgacata ccagaagatg atggtggacc gctgtaacgc cgtcggaaag     960

ccggttattg tggcgacgca gatgctcgaa tccatgcaga acaacccgcg ccccacgcgt    1020

gccgaagtgt cggatgtcgg caatgccgtg ctggacggcg cggactgcgt catgctcagt    1080

ggcgagtcgg ctcagggcaa gtacccgatc gagtctgtgg ccacgatgaa cacggtgatc    1140

aaggaggccg accagctcct actcaagcct aactaccagg ccaagttcca gttcgagccg    1200

cccacgtcgg acgtcgagtc agccgtgtcg tcggcagtca agacggccaa cgagatgcac    1260

gcgcagctcc tgattgtgct cacccgtacc ggttacacgg cccgaaaggt cgccaagtac    1320

aaaccgactg tgccggtcat gtgcttcacc accgacctta aggtcggccg tcagctccag    1380

atccaccgcg gtctgtaccc ggtggtgccg gactaccttg accgtgcccc gactaccgcg    1440

gaggccatcg ctcacgccaa gaagatggga tggttgtctg ccggcgaccg tgtcgtcgtg    1500

atcagcggcg acaagctgtc ggacgatctc ggccgggaaa tcctcatcgg cgtcgctgac    1560

gtccagtaag acggagacaa                                                1580

<210>  8
<211>  900
<212>  DNA
<213>  Phytophthora infestans

<400>  8
atgtggtcga cgatgatcat gcggttccct acgcaggcca agtccgtgtc gcgcagcgcg      60

cttgtggcgg caggtatggc tgcgttcgct gccttctcca gtgtttcctc tactcgctgc     120

gaagaggaga agtccaaggt ggcgttgagc cccaaggagt tccgttcttt cactgtgcgt     180

aaagttgaga ccgtgaacta caacacgaag cgcgtgacgt tcgctcttcc cacacccgag     240

cacgagatgg gtctcacgac gcctagttgc cttatggctc gtgccaaggt agacggcaag     300

acagtggtgc gcccctacac gcctgtcaac gtgaacgacg agaagggttt cttggagctc     360

gtagtcaagg gttacccaca gggaaagctc agcaagcaca tcgtgcagct caaagaagga     420

gactctcttg acatgaaagg tccctttccc aagttcaatt actaccccaa caggtacaag     480

agcatcggca tgatcgctgg cggctccggt atcaccccca tgctgcagct catcaaggcc     540

atttgccgca acccggagga ccgcaccgag atcacgctgc tgtactgcag tgtctcggaa     600

gaagatatca tcctgcgtga agaagtggag gccatgatgt acctgtaccc gcagatctcc     660

gtgatccacg tgctcagcaa cccgtccgcc gagtggaagg gtcttacagg cttcgtgtcc     720

aaggagatga tcgaaaagta catgccggag ccgtcagacg acaacctcgt gtgtgtgtgc     780

ggtcctccgc caatgatgta ccacgtctcg ggtgacaagg cgaaggacag gtctcagggc     840

gaactgcaag gtctgctgaa agacatgaac tacacctcca ctcaagtgtt caagttttaa     900

<210>  9
<211>  1245
<212>  DNA
<213>  Phytophthora infestans

<400>  9
gtcgagcaca agaccaacac tcctgcacca tgactaccct caagatcgtc gtttccggcg      60

ccgccggcca aattgcgtac tcgttgctgc ctctcagtac gtccgtctcc ttccctcttg     120

tgtcttctcc ataccctaac gtctcaccat gttgcagtct gcatcggcca cgtcttcggc     180

cccaaccagc gcgtggagct gcgcctgctg gacatcgaac ccgcccagga agcgcttgag     240

ggcgtcaaga tggagctcca ggactgtgcc ttcaacctag tggacgctat catccccaca     300

gccgatctgg agactgcttt caaggacgcg gacgtcgcaa tcctcgtggg cggcttcccg     360

cgcaagcaag gcatgcagcg caaggatctg attgagaaga atgtagccat ttttaaggct     420

cagggagctg ctatcgacca gttcgccagt cgcgacgtta aggtgcttgt tgtggccaat     480

ccagccaata cgaactgcct cattgccatg gagaacgcac ccagcatccc gcggcgcaat     540

ttctcggcac tgacgcgtct ggaccatgag cgtttgcgct cgttcctagt ggagaaggtc     600

aacgagaccc aaagcccgaa agttacgtcg aaggacgtca acaaggtcgt gatctggggc     660

aatcactcga gcacgcaagt gcccgacgtc acgaacgcag aagtgaaggg ccagccgctt     720

gataagatcg tctcggacaa ggattgggct gagaagaagc tcgtcaagga cgtgcaggag 780

cgcggtgcag ccatcattaa ggctcgcaag ctctcgagcg ccatgtcggc tgccgccgct 840

atcggcgccc acctgcgcga ctggttcaat ggctccaagg acggcgagct cgtgtctatg 900

gccatttgct cggacggcaa caagtacggt gtgcccgagg ggctcattta ctcgttccca 960

gtcaagtgcg cgggcaacgg cgcgtacgag gtggtgaacg gtcttcccat ctcgccgcgt 1020

atcgacgcaa tgatgaaagc cactgcgcag gagctgacag aggagaaggc cgacgctgtg 1080

gagatcctgt cgcgccagtg agcagtattc agctaatagc tggacgatgg caacgtcgtc 1140

tacaccccat tcgatttttt tctcccatta tgagctgctt caatcgttta tttgaccatc 1200

taaaccaatt aatttcttgt tattacgatt tcttattgaa aaaaa 1245

<210> 10
<211> 1280
<212> DNA
<213> Phytophthora infestans

<400> 10
atgtcgtctt cattttgtca gctatccaag cgcgtgctgc agtatcttcg acatgagcat 60

ccacatcgtc tatttccctc catcggtcga agacgcaaac tcccaggcat aaacctcgac 120

ggaagatctc agcggtgatg gcgcgtggtt agccgcaagg tgttccgatg cacaccacac 180

actactcgcg aaaacgacca tacttctgtc ggccagggta acctctgggg agctgcacaa 240

cggccgtctt tttagcttat tttagcgagc caacaggttg tgcctcattg gccacgtgtt 300

cacgcacgta cgtcacgtgt gtgctcattt taatattaaa gatcccgttg aagacgaaaa 360

gcttctttgc tggcatcatg cgcttccaga cgttgttgct cgtccttgtc gcactagtca 420

tcgccccgtt tcaggccctg tctccaccca gcgagtccaa cgtgccgttc actcttcggt 480

acgtcccggg cttctttaaa caaggtaccc cgtccgtgga gattccacct tcaaattcaa 540

ggcatctcgg gctacttgac aacgtcacgt ggacggatgt agacgcctac attgctgcac 600

gagccgctca aggcgtggac gtcaagttgt tcctcttctt acgtcatgga gaaggcctcc 660

acaacgtcgc cgaagccact tacggcaccg aagcctggga cagattctac agcaaactag 720

cgaaatatac tgacgccaag ttgacgaaac ttgggatgca acaggctgtc aaagcgtcgg 780

aaaggatcga cgaggagctt aagagaggac tgagcctgga ggaagtcgtg gtttccccgt 840

tggagcgtac actacatacg gcaatgatcg cgtgccagaa tcaccacgag atcccgaagc 900

ggtcgatgga atggccccgc gagaccatcg gcgtctgcac gtgcgactta cgcggcacca 960

tctccgccaa ggccgagctg tacccaagta tcgacttcag tgatatctgg agtgatgcag 1020

acccgtggtg gacgcctgat catcgtgaga ccgagctgca catcaacgac cgcgctcgca 1080

tcttcctgaa ccgcgtcttc tacggtcaca agtcagtgcg tgttggtgtg gtgacgcaca 1140

gtggactaac caccgccgcg atgcgtgtca ttggccatcg taagtacagt gttgcgacgg     1200

cggaagtgat accgttcctg cttgaagaca ccacggtgca cacgatcctg tcgatcttct     1260

ctggtaaaga agacatgtag                                                 1280

<210>  11
<211>  1016
<212>  DNA
<213>  Phytophthora infestans

<400>  11
atgtcgtcct atcgtgctgt ccaagttgta aagttgacta aggacttccg cgccgctacc      60

agaatcgtga gtgtccccga actacccacc gctgggcccg acagcatcgt tgttaagaac     120

cgcttcttgg gaattaactc gaccgacgtc aatcttacga atggcatgta tcatgacaag     180

ctgccgtttc tcgcgggcgt tgaaggtgca ggtctcgtca ctgaggtcgg gagtaacgtc     240

acgactgtga aagtgggcga cgctgtcatg taccagacgc tgggagccta tgcggactac     300

gtacaagtgc ccgtgtcggc cgccatcaag atcccagagc tgtcgaagaa cgctctaccg     360

gttgcagtgg gcggcgtctc tgcgtccatc tgccttgaat gtctcggtga gatgaagtcg     420

aatgagaccg ttcttgtgac tgctgctgct gggggaaccg gacagtttgt cgtacagctc     480

gccaaacttg ctggcaacca tgttatcggc actaccagct ctgacgagaa agtggaggct     540

ttgacgaaac tgggctgcga ccgtgtaata aattacaaca aggagaatgt cggcgatgtg     600

ctaaagaagg agtatccgaa tggagtagac atcgttttcg agtccattgg aggcgacatg     660

ttccgtgctg ctgtcgataa cattgcagtc cgtggtcgta tcatcaagat tggcttcatc     720

tcgcgagtat tggagaccaa agacaagacg actccccaag gccgcctccg ctgctatcgt     780

gggaagcgaa caagaagatt ctgatgaagt ctgcttccat ccgagggctc ttcatgtatc     840

actttgagga acacattcga gagcacacgg agcgattgct gaagctcatt agcgagggca     900

cgttgaagcc tggcgttgac cccactacgt acaagaaatt cgagtccatt ccgaatgcaa     960

tcgaccgcat gttcgcccgt gaaaatgtag gcaaactcat cgtagagctc gagtag        1016

<210>  12
<211>  267
<212>  DNA
<213>  Phytophthora infestans

<400>  12
atgggcaagc acacggagcg agccaaggag agtccattca agcagcagtc gatgatcctc      60

gtgcctatgg acacaccagg cgtgcaggtc gtgaagccca tgcatgtctt tggctacgac     120

gacgcaccgc acggacacat ggatatgctg ttcaaggacg tgcgtgtgcc gttcagcaac     180

gtgttgctcg gtgaaggtcg cggctttgag atcgctcagg atcgtcttgg acctggccgt     240

atccaccact gtatgcgagc catctga                                         267

<210>  13
<211>  1147
<212>  DNA
<213>  Phytophthora infestans

<400>  13
atgcgcaaaa tattgaatat tgattggtta aaacctggtc ttggcatcct tatgaccaat      60

tacagttatt gagtgcttta agcggggcag acgtccacta cgtcacactc agaatctcca     120

cacaagcaac agaccgacac aatgagcagg tgcgagtggc atcccagcaa cttcgtgcaa     180

atttagcctt gttatattaa ttgtataacg tggcatgttg cagaccgacg atctcagagg     240

ttatcgccga gcggaaggcc aacaacactg cggctttcat caccttcgtg ccctgcggct     300

tcaagactaa ggctgacaca gtggacattc ttctgggact gcagcgcggc ggcgcgaaca     360

tcatagaggt gggcattccg tactcggacc cacaggccga tggcccgacc atccagcgcg     420

cacaccaggt gggcgtggac cagggcatca cgctgcatga cgtcctggcc acagtgagcg     480

aggcgcgaac taagggcctt actacgcctg tggtgcttat gggctactac aacaacattt     540

tgcagtacgg cgaagtcaaa atctgccccg acgcccagaa aggtacgcct gtaatcgacc     600

acaggagaga tttgtgggct caaaaactga caataaacgt atttgtagct ggggtggacg     660

gattcattat tgtggacctg ccgcctgagg aggcaaagac gctcagtgac gacgcagcta     720

agcacggact tgcgtacatc ccgttggtgt ccccaacgac gacggaggag cgtatgaagc     780

tcatcgactc ggtggcacat ggttttgtgt attgcgtgag tctcaccggt gtcacgggcg     840

ctcgtaacga gctgcctccg aacctcgacg cgttcatggc caagagtaag ttgcttttcg     900

cgctttagtt tattttaatt gtcgagctaa cgtggtccct tgattcgctg cagttcgtgc     960

caacgtgaag caccctctcg ctcttggctt cggtctgtcg acccgccagc acttcgttca    1020

agccagcgcg cttgcggacg gcgttgtgat cggctcgaag attgtcaaga tcatcgagga    1080

cgcgcctgac acagccacgc gtgctgccaa tgtggaggca tttgccaaga gcatcgtgaa    1140

cccttag                                                              1147

<210>  14
<211>  1917
<212>  DNA
<213>  Phytophthora infestans

<400>  14
atgggtaaca tcctggagga gatcgcagcg cagcgccgtc tggacgtggc ggccgccaag      60

caggtcatat ccgccgatga tctggccaag aagatcgagc acgcggaggt cgttttcggc     120

tcgaccctgc ccgtgcttga ccgccttaac gcacccacgg tgcgtccggc agtgcaggga     180

atctggagaa gaatctgcgt ctaacgcagc taccgctgta ctgtattttt tccttgtatt     240

tatggcagag agaggggtgg tccgacgtgg ctctggctgc cgagtttaag cgtgcgagcc 300

ccagcaaagg tgacatcgcc acggagctca atctgcgtga gcaagtgcag gcctacgcca 360

acgcaggtgc cagtatgatc tccgtactga cagagcccaa gtggtttaag ggatcgctag 420

acgacatgag ggcagcccgg gaggtggtcg agggcatgag tcagcgtcct gccatcctgc 480

gcaaggattt catcatcgac gtgtaccagt tgctggaggc ccgcgcttac ggagcggatt 540

gcgtgttgct catcgttgcg ctgctgtccc aggagcagct tatcgagctc attgacgtac 600

gtcgccccgc ttgcactaca tgtagtgggt ttactgtaca ctgacaagcc tcttgttggt 660

ttgtaggcca cccacaatct cggtatgtgc gctctggtcg aggtgaacag catccaggag 720

ctggacatcg ctctggctgc gagggctcga ctcattggcg tcaacaaccg cgatctccgc 780

acgtttaagg tagacatgaa cacgacggct cgcgttgcag acgctattcg tgagcgtggg 840

ctttcgctgg gacgtgacgg cgttacgctc tttgctctca gtggcattcg ctcgcacgca 900

gacgtggtca agtacgagaa gtgcggcgcc cggggcatct tggtgggcga gtacttgatg 960

aagagtggtg atattgccgc gacggtgaag gatctcttgc agaatgtgac gcgtcacacc 1020

gagtcgggcg aattcgcttt ggctcctccg cttgccaaag tgtgcggcgt cacgacagtg 1080

gagtacgcgc tggcggcctt gcgtaatggt gccaatatga ttggtatcat catggcggaa 1140

cactcacccc gctatgtcca agtggaggaa gccaaggcca ttgcccaggc tgtgtgggag 1200

tatggggagc gcacgggccc tattctctca gacattgtgg agactcactt ggacggcaag 1260

aacgactggt ttcatagaaa tgtccttgcg ttgcgtgaag cttgctcgcg tgcacctctc 1320

gtggtcggag tgtttgtcaa caagacagct gccgagatga acgcgactgc aaaggaaatc 1380

ggactggact tggtacagct acacggcgac gaggggtttc agatctgcag ggacattaag 1440

taccccacca ttcgcgcgtt gcatctgccc gacaccacgc tctgcgacgg cgtggacgca 1500

gaagccgttc tacagcaggt tcaggaaggc cttgccaact acattctgct tgatacgacc 1560

gtgaagggtc agcagggcgg cactggcgtc acattcgact ggaagattgc agccatcttt 1620

gcgcaggcac gacttccctg cctcatggct ggtggcctta cccctgagaa cgtggtgaag 1680

gctctatcgg tcggtcaccc cgttggggtg gatgttagca gtggagtgga agtgaaaggt 1740

tcacctggtg tgaaggatat ggacaaggtg actgcgttcc taaaggctgt gaaggattac 1800

ctctcgattg ctacgcttaa gatcgaggag gagacggaaa cctagagaga gaccctcgga 1860

gtgttctaat caatgagggc cactaatgaa gtgcttttac gttacgagct tggatgc 1917

<210> 15
<211> 1494
<212> DNA
<213> Phytophthora infestans

<400> 15

atggtgcact atcgtagtac tcgcggcggc gtccgcggcc ttagctttga gcaggcggtg 60

ctgacgggct tggcttccga ccgtggcctg ctgatacccg aagctgacga gttccctacg 120

cttccagctg atgcgctgga gaagtgggcg tctctttcgt accaggaatt ggccgtggag 180

gtcatgcgcc ttttcattga cgaaagcgag atctcgcgtg accagctgcg tgagcttgtg 240

accaagagtt acaacgcgac cacgttccgc tccgatgagg tggcgccggt ggtcaaggtg 300

acggatcaaa tgctggtgct tgagctcttc catggcccca cgtttgcctt taaggatatc 360

gctctccagt ttctaggcaa ccttttcgag tttttcttaa agcgcaagaa cgaggcgctg 420

ccttctgacg ctcctaaaca ccagatcaca gtcgtgggcg ccacctcagg cgacaccggt 480

agctcggcca tatacggtct ccgcggcaag gagaacgtcg aggtgttcat cctcttcccc 540

gagggtcgtg tgagcgccat tcagcagcga cagatgacta ctgtattgga ccagaacatc 600

cacaacgtgg ctgtgaaggg cacgttcgac gactgccagg ctatcgtcaa ggaccttttt 660

gccaacgctg acttcaaggc caagtacaac ctaggtgccg tcaactcgat taactttgcg 720

cgtatcttgg cgcagatcgt gtactacgtc tgggcctact tccgtgctca cgacgagggt 780

gttagcggcg aggttgcctt ctccgtccca acaggcaact tcggtgacat ccttgccggt 840

ttctacgcca agaagctcgg tgttcccatt ggcaagctga ttgtggctac caacgagaac 900

gatatcctgc accgcttttt ctccacagga aaataccacc gccgcgacat cgagcacacg 960

atttccccgt ccatggacat ttgcgtgtcg agtaactttg aacgttatct cttcgctctg 1020

tccggcgaga accacgacat tctacgcggc tggatgcagg ctttcgagca gactaatgag 1080

ctcacgatct cgggcgagct gctctccaag gcacaagacg agatggcatc gtatgcggtg 1140

cttcaggagc aggtgcgctc cactattgcg gagtacaaaa cgatgcatca gtacctcttc 1200

gacccgcaca gcgctattgg cgcagcagct gccatgcact ttgtccagga taaccttgca 1260

gacaagccaa actcggcggt agttgtagtc ggcactgccc actacggcaa gttcctcccc 1320

gtggtgtcga aggcactagg tgtcgctgag tcagagattg agcaacaccc gatcctcaag 1380

gcgctggaat cactgcccac ccggctttcc gttgccagca actcgagtga aactgtggct 1440

gagcatattc gcaagattat tgctgagaag aacgacgaag cctgtagtga ttga 1494

<210> 16
<211> 1299
<212> DNA
<213> Phytophthora infestans

<400> 16
atgagctcgt tccgtcttcg tattgccaac gcgcgccaga tcgttcaagt ttgtgcgaac 60

ggcgagccgt ttaagaccgg cgcggcccaa aaggatctgg ctgtgctcga gaatgcgtca 120

cttgtcgtgg acaaggcagg taagatcgcc ttcgttggtc ctgccgctga cgtagagaag 180

tggctgagca cgcagctgca gcccgtgagc tttgagaagg acgtggacgc tcgtgacatg 240

gtcgtactgc cgggttttgt ggatgcgcac acgcacccag tgtggagcgg caaccgtgtc 300

aacgagtttg ccatgaagct ggctggcgct acctacatgg aagttcacga gagcggcggc 360

ggcatcaaca acacggtgcg tcacaccaag aagagttcag aaactgagct gttggagctg 420

ctgcgtacgc gactagaccg catgttgcgt agcggtacga cgcttatcga agcgaagagc 480

ggatacggtc tcgagactga cactgagctc aagatgctgc gtgttttaca ctccgcatcg 540

acgggtgaca acgcacaccc tgtggagatc gtgtccaact acctgggtgg tcactcggtt 600

cctgacggca tgactgctgc tgaggccacg gaggatatca tcacgaagca gattcctgct 660

cttgtgcagg ccaagaagga cggcaccatc tcgcccgaat tcatcgacgt gttctgcgag 720

aagggtgtgt tcgagcacaa cgactctcac cgtattctag aggcaggcaa gaaggcggga 780

ttggacatta acttccacgg tgatgagatg tgtccgatgc agtccggtac cttggcggca 840

actctgggcg cccgtgcaat ctcgcactgt gagatgctga ctgctgagga tcttcaggct 900

atggcagcgc acaagccaga gcctgtattc gccgtgcttc tgcctactac caagtatatc 960

ctgaagctgc ccaatccgcc agcacgcgac atgatcgctg ccggtgttcc tgttgctctg 1020

ggcagtgact acaacccgaa cgctcattgc ctttccatgg cactgaccat gaacatggca 1080

tgtgtgctgt tcggtatgac catgaaggag gctcttgtag gcgccaccat caacgccgct 1140

gcctccatca accggtcggc tactcacggc agtctcgagg tcggcaagca gggcgacttg 1200

gtcctgatgc gtgctacgca gtgggagcag atcatctacg aaatgggcga ccctcctatt 1260

gaacacgtag tgaagaaggg agttgtctac acgaagtag 1299

<210> 17
<211> 1846
<212> DNA
<213> Phytophthora infestans

<400> 17
gcaacgaaca agtctcttcg ttccacttcc gtttcacctc gtcgtcatgg ccatcgccaa 60

ttcgaacacg aagtctgagc tcgttctgga cggcgagtct ctctgtgctg aagacctggt 120

gcagctgtcc aagggagaca cgagaatctc gctaagtcaa gaagcctgga agcgcgtggc 180

ttgtggccgt gaggtcgtgg acaatatcct caaggacaag actcgtgtgg cgtacggtat 240

taacacgggc ttcggactct tctccaacgt cattatcggc cccgagaagc tcacagagtt 300

gcaggaaaac ctcatccgct cgcactcgtc tggcacaggc gagccgttga cattcgctca 360

gacgcgtatg ctgctcgcgc tgcgtatcaa tgtattggct aagggacact cggggatccg 420

tgtgcacact ctggagcagc taatagacgc ctttaacgcc gactgtctgt ccgtggtgcc 480

agccagaggc actgtgggcg cctcgggcga cttggctccg ctagcacacc ttgcactcgg 540

tatgatgggt gaaggaccca tgtgggataa ggtggatggt cagttcgtca tcagcgaggc 600

ctccaaggtc ctggccaagc acggactgaa gcctgttcag ctcggagcaa aggaaggtct 660

ggctatgatc aacggcacac aactcatcac gtcggtgggt gctgaggcgg ttgtccgtgc 720

tcagaacgtc gctaactgcg ctgatatcgc cgtcgcattg acactcgagg tgctctgcgg 780

tactgtcaac gccttccacc cgcgtattca tgcagcccgt ccgcacactg gccagatgct 840

cgttgcctcg cgtattcgca cactactacg tgctgacaac ccgtccgagc tgttccgtag 900

ccacaactac gaaggaaagg tgcaggacgc ctacactcta cgttgtgcgc ctcaggtgca 960

cggcattgtg cacgacacga tcaacttcgt acgtggtgtg ctggacgtcg agatgaacag 1020

tgccacagac aaccccatgg tcttcacggg tagtgccgag gtcacgacgg atctgtctcc 1080

ttcaattgac acgaatcaag tcaagcccaa catcgaacag gtagaacacg agatcacgga 1140

tctgaatgac gccaaggagg agatcaagcg tcttaaggct cttgttgcac agaagcagaa 1200

gcctgtggaa cacccggcgg cgggtatgaa gcgcacatcg gacacgttct accgtggcgg 1260

cggtggcttt gtcatctctg gcggcaactt ccacggcgag tatccggcta aggtgctgga 1320

ctacctggca attggtatcc acgagattgc tagcgttagt gagcgtcgta ttgagcgtct 1380

agtgaaccca acgctcagca acctgccggc tttcctcgtg ccagagggcg gtctgaactc 1440

gggttttatg attgctcact gtacggcagc cgctctggtt tcggagaaca aggtgctcac 1500

acacccgtcg tcggtggact cgatctctac gagtggagcc aaggaggacc acgtgtcgat 1560

gggaggcttt gctgctcgta aggcgcttac ggttgtggag catgtcgaga ctgtagttgc 1620

tattgagatt ctggcagcgt gtcaagccct tgacttgctg cgtccgctgc gtacgacgga 1680

ggctcttgaa gctgtccacg gactggtacg cacccgcgtg gctaggtttg acaaggatcg 1740

cttcatgaag cccgacattg acgccgtgct ggacctcgtt cgtagtggag ctatctgcga 1800

cgttgtagct ccgttcttgt caaaactgca cgtgtcagga ctttaa 1846

<210> 18
<211> 1218
<212> DNA
<213> Phytophthora infestans

<400> 18
ccgcacagag caactgccac taggcatcat gctgcaccga cttggccgcc gctcgactgc 60

cacttccgcc cagcggtcac tgcgccgatc agccatgacg ccaccgcagc gccaatcctt 120

tcatttcagc tcgtcagcac gcaagccggc atctttggat gctcagaacc gcgtagctgg 180

tgctgcctcc tttttcctgg acgccaaggc cgtttgcaag ggcaagcgct gtgcattggg 240

caagaacaac agcggtattg gagctaaccc ggccacatgc ggggaggatt cgttcttcct 300

cacacctgac gtggtgggcg tggctgacgg cgtcggtggt tggaacgaga acggcgtgga 360

tccaggcaag atatcgcgct cgctaatgcg caacgcggca ttatttgtgc agcagcagac 420

ggccaatagc gagagtgcca ccacgcaaca ggttctggct cacggctaca agcaggcgct 480

gctggatgac gaggtggagg cgggtagcac gacagcctgt atcgtgagac tgaagcagtc 540

gtctgagggc aaacccgtgc ttgagtactc gaacctcgga gattctggct tcgtggtcat 600

ccgcaacggc gagataatct tccgcagtaa atttcaggtt cgaattgact gttgggagtg 660

acagtagaag agttactgac ggcgttgctt tagtattatg gacgtgctcc gtaccaactg 720

gcaaagatcc ctctgcggtt taagcagtat ggtgctattg agaatcaccc tgatgatgta 780

agtctgcttc gcatttggat actatttatg ctggagacag tacaatgcta acggttattg 840

ctccaggcgg actcgggtga gattgacgtg caagacggag acctcgttgt actagccact 900

gatggtgttt gggacaactt cgcccccgac ctccagaaag ctccttcgca gttcccggta 960

agattcttta ccgccgtgtt cgagctacct gtaattactg actttgtttc tcgttattgt 1020

ctgctatttc taccgtagcc gatgctctcc tggcgaagat attggtcagg tgaactcgca 1080

tcttttgtgg atgttattgc agagaacccc gacaaggctg ccgaaaatat cgtcctggcc 1140

tcgctccgcc acaaccttaa gcccgacgac atcactgtcg tcgtggcaaa ggtagtcgtg 1200

atggcatcaa agctgtag 1218

<210> 19
<211> 735
<212> DNA
<213> Phytophthora infestans

<400> 19
atgaagcagt tactgcagcc gatctcgttc tctggtgctg cgagtgtgtt cgtcgtggtg 60

gcaaagcaac gcaagaaacg ctgccgtgcc aatgacaagg aagatgacgc tgttaaggac 120

gacgaagaga ctgagacagc agaggacgaa ctggacgagg aagaccaacc cacgcgactg 180

tacgtggcca atgtgggcga ctgtcgagct gttttgtgca ctgcagacgc cttagcagtc 240

gacatgacga cggatcacaa ggcgtcgcta ccggctgaac aggagcgtat cgaggcgtca 300

ggtggcttcg tgcacaacgg ccgactggac ggcattctgc agatctcccg tggcttcggt 360

gacttggcgc acaaacagga tggccacttg gtggtcacgc cagatgtggt ggagcatctc 420

gtgaacccgt cggaccagtt cttgctgctt gcaagtgacg ggctcttcga cgtgttgacg 480

tcgcagcaag ctgtcaactt tgtgctgcgt aaacttcaga cccatggaga cgtccaactc 540

gcagctcagg agctggtgct caaggcgcaa gcttacttcg cacacgataa catcagtgtg 600

gtcattgtgg cgttgaacca gaaaggtgac gcgtgaaatg aacagcggct tctattgact 660

acggaagatc ggagaccgac gaagaagcag aaaacccgga gatcaagctc gagatccaag 720

aaattatgaa caaga 735

<210>  20
<211>  1845
<212>  DNA
<213>  Phytophthora infestans

<400>  20
atgcgacgca aggagaccaa ggtgttgttg acgctcgaag aggtgcgcga ggccttcgag      60

cagcagacgc cgctggccgt caaggacgcg ctgggtgtca ttcatgaggc gcagttcatc     120

atgaacctgg agccgaacct tgtggccgtg cggcaaagag cttcaacata tgtttttgga     180

gatatccatg gccagttcta cgacctgatg cagctgatgg acgccgttgg cgttgccgac     240

ttggccgagc gtgatgtcca gctggtgttt ctgggagact acgtggaccg cggggccttc     300

tcgtgcgaag tgatgctcta tctgctactg ctcaagatcc gattttccga caaagttgtt     360

ctcctccgtg gcaatcacga gtgcgaatcg atttcatcct tctacggatt tcgcaacgag     420

tgtaagggga agtacggcat ctcagcctac taccacttct tgtcctgttt ccaatcgatg     480

ccagtggctg cgctgctgtc cacgtcgcgt gggcaagttc tgtgtgtgca cggtggcctc     540

tcacccgagc tgaaaacaat cgaagacatt caaactatgg accgacggcg ggaaatccct     600

accacggggc tgttatgcga ccttttatgg tcggacccga agacttcgca cactcgcgat     660

gtggacgcag aggtggacac tcagccagga tgggagccca atcaggcccg tggatgctcc     720

tactacttca attcggcggc attgttcgag tttttaggca ccaacaagct gctgtcgatg     780

cttcgtgcac atgagttcga ggatgaaggc ttcacgtatc acttcaactc gcaggagtat     840

caagagctgg atactcgagt ggacaagtcc atgcctccac tcattaccgt tttttcggcg     900

ccgaactact gcgacagcta cggcaacacg gccgcctatt tgctcttccg gaatgaacct     960

ttctcgtggg aaatccagca aattaactcc gcgggacatc cagccccacc gattgcgagc    1020

gcagaacgag gatccgacat gtggcgacta ttcaatcaaa cgttgccatt cctcccagca    1080

agcaaggaat tctttgagga agttttgtgg ttagcagaag gacgacgacg tcttacaagt    1140

gaaatgcacc cacaagctat ccgaaagatg ttggatgacg aagtgaacaa atgggaattg    1200

gtagaagaaa cgccaacgcg cagctcgggg ccagcgtctc ctcctcgaat ccaccgcaga    1260

ccttctttga gtaatctgga cgagacccaa acggacgagc gtccaagtct gcttactccg    1320

caagagatgg acacaattaa gctgatgttt tcattaatgg acacggatgg cagcttggaa    1380

ctcagctcga ctaaagtgtc tcagttcatt ctcaatattc tgggtgaaaa aatcagcact    1440

gcggacgcgg aggcttattt ggatgctttg gactacgacc gcaacggagt ggtggatttc    1500

gcggatattc tgtcgtgggt tgccgtgatg aaggcgaatc gcaacaagca cgagtcgagc    1560

cgactcctga gttggccaac tgtacagagc ggtgtgcgca tgttgactcg tgggattttt    1620

tccagtaaag tcttgctatg gctcgcattc ggctgcttac tacgcgatat tatcgtccca    1680

aagcagcgaa aagtgatcaa gtcgtcgtcg atccgagtgc tgggctcagc gtcgctagtc 1740

ctgtacatgg tgggcgtatt gtcagggcga gagcgtggct ggctgcggct tttctctctg 1800

caacgcgctg tcggaattat gacgcagcga ttcgtatcaa agtag 1845

<210> 21
<211> 1489
<212> DNA
<213> Phytophthora infestans

<400> 21
atgatgccgc ggggcttacc accacgaggc ccacctggca gtttcccacc tactagtgtt 60

gcggctgtac cccctgcagg cggtgccgct gcagctcgtt tcggcaagct ctctgtgaag 120

gtgttgcgtg cgtttgacct aaaaaagctg ggaatgctgg acactgcaga cccgtatgtg 180

aagctcacga tcggcacgca gaacgtacag actaaggtcc aagcaggcgg tggcaagacg 240

cctgagttta atgagacctt cgacttcaat attgcaaccg agaaggagct cgtggtcgaa 300

gtttgggacc aagaaaaagg aggacaagac cggttcatgg cgcaggcgaa ggtggagatc 360

gtgtcgtggc tatcaaaggg tggctttgaa ggtgacgtcg agctacggga tcgcgagaac 420

agccctgccg ggaagctggc gatcgttgcc aagttcacga agcccgaaat tggcgcgaca 480

ggacccgtca aggctccacc catggcccct ccgatactct caggtcctgc agtggctccg 540

ttgcccccag gagcgaatgc cgtatctgtt cctggtgcgc tgccattggc accgtccgaa 600

ccgcctcgag acccgaacgg caagttcacc gacaaggaga ttctcgaggc atttaaagcc 660

tttgatctgg atcacaataa ctatgtgggt gctgcggaaa tccgccatgt gctaataaat 720

attggcgagg cgcctacaga cgaagaagta gatgaaatga tcaagatggt tgacaaggat 780

ggcgatggcc aggtgagctt tgccgagttt tacgcgatgg ttacgaaagg gaagcagccc 840

ccacccggat tgggtgtcac tacagctctt ccggagaaag ctgcagctcc tggaggtgca 900

gtttcgggcg ctcaggccat tcagcttcgt aatcagcgca aaatggcgct ggaagagttt 960

gcacgcgata acggtatcaa acccgagagt gtgaagaagg cgtacaagcg attccaagcc 1020

acagataaag atggatcggg ccagatcgac tactcggagt tctgcgaggt gctgcaggtg 1080

gatccatcgc cgcagtgcga gaaggtgttc cagttgttcg acaatgacaa gacgggtcgg 1140

atcgacgtcc gggagttcat gattgcgttg tctaatttca cgggtgctga gaaggaggag 1200

aaactaaagt tcgcgttcct cgtgtttgat gaagatggta acggtgtgat cacgcgacaa 1260

gaactgatga agatcctgaa ggcgaaccac atggcctcta gcgaatcgga agttgcgcgc 1320

aaggcggaca ctattatgtc ccaaggggat aaggacggcg acggagttat ctcgttcgac 1380

gagttctcag ttgtgagcaa gaagtttcct aatattttgt tcccagccta cacgctaggt 1440

acggccaaag attagaatac ccgatgaata caatatcctt atctctcgt 1489

<210>  22
<211>  2862
<212>  DNA
<213>  Phytophthora infestans

<400>  22
atggctagca agctcaacgc gttcctggcc gcggtgcctg cgaacgccaa cgtgcccagc      60

ccgcccaaga cagcgccggc cgtgatggcg ttcccgcagc ttgagttcaa ggaagcgccc     120

gagaacgtgg gcgcattcgc ccccaccacg ccgcagccgc ccgtggtgcg tcgtcgccag     180

cgcaaaaata accccagcct caagctggag ctggactcga accaggaaca tgacagacct     240

gaggatgatc gagatatgtc gcgtccttcc cctctaaaga gcttcatggc cgcccaagca     300

gccgccgtta agctgaaggc acagacggag aagcacgata tcccaacacc agacttggcg     360

cgcacaagca gcgggtccga gctggacgcc aagtcggagg cgggacccat gcgcatggcc     420

agcgtacaag gctcgcacca ccgtcgcgtg tccaccgaca caacgctatt taaccggtat     480

ctgcgtcaac aagcagacct ggatattagt cacgacacgg tgcagtattt cgcaaatgtc     540

ccggtcatta ccgccactgc agctgccgtt gatgctctgg aatgcgaaat gagtgtggat     600

aataacgccg acgaggagca cgcgatcatc gtacaggaac ccgaggagat ccttgctgtc     660

ttccgactcg gaggcaccat ccctgtgtcc agtgcgctgg aaattgtacg acgagctacg     720

aacctcatgg cactagagca gaacgtcatc tcgattcgtg cgccgtacac tctagtgggg     780

gatctccacg gccagttcca ggacttgctc gaactcttcc gagtacacgg ctctcctgcc     840

gtggacaacc cgttcttgtt cctaggtgac tatgtggacc gtggcgtgtc ttcgtgcgag     900

ataatcttat tgctcttggc cttcaaagtg gctttcccgg acagtgtcca tttattgcga     960

ggcaatcacg agtgtcggag tttgagtaca ttttacggtt tccgtgccga atgcctcaag    1020

aagtacggac ctgtgctcta taaccgtatg atcaagtgtt tcgagagtat gccactggct    1080

gcacgactcg agacagcgca cggcacgttc ctggctgtac atggcggact gtcgcccgat    1140

attcagttcg tgggggacat taacggacaa gtgaaccgct ttatggagcc tgaaccaaac    1200

ggagctctgt gcgacttgtt gtggtcagat cctgcgaagg gcgaagctca ggagcaggag    1260

tgggcaccca atgggatgcg tggctgctcg ttcacattta atgaacgcgc ctgtcgcgaa    1320

tttctgaagc gcaacaacct cctggccatt gtacgtgctc atgaactgga agaaaatggt    1380

tataaggagc actttcgaca cgaaggagac cgcacagaag aagacgagga cgggaaactg    1440

gctctacctg cggttgtgac ggtgttttca gctccggagt actgcaacac gaaccacaat    1500

gtcggcgcga cgctgaaaat tccctgggaa agacaaaatg gtcgtctgct gcagtatcag    1560

cagcataaac gcagtcaatg ttccgagttt gagttcacgc gagccagcga ggagacggcg    1620

gccaaggcgt ttctggagga gaatttgccg ttcttgccga ttgactttta cgacttggtg    1680

aatgtgtgtc ggcagctgcg gctcactctt gagagagcag cgagtatcac cccagcacct 1740

gcttcagaac ctattcgcaa gctgtcgctt gtgtcgtcgc tgtgtgcgcc agcaactagt 1800

ctggaaacga ggatcgagga ggaagaccca gagcttcccg tgtcgccacc tgcttctgca 1860

ccagcaaatg aagtggagaa ggaagctaca gcaactctga aggcagtggg cgagtcgttg 1920

caggattggg aacttgttga gacgaagagt gtcgaggttg acactaggaa ggtgaagaaa 1980

gacaagaaga aggagaaaaa ggagaaaaaa ctgaaggaga agatcgaaaa gaagaagcaa 2040

aaggacaaga agaagtggga tacgagtcga attgctagcg gttggaagct ctgccctggg 2100

tttgtacgct tctacgaccg ctacttctca agagacagta tgaagaagag cgagccggag 2160

atcacctcag ctcctggcca tctaggcaag cggccgtcgt ggatctcaaa ctacaaaaca 2220

ccttttagac gctcgacttc gaccggcgag gcccccaccg acactgaaac tgtcgacacc 2280

gtcttggact ctaccggtcc tgctcgacgg aagagtatga cggattggat gcccatgccg 2340

tcgttcgagc aggtggcgca gctgacgaac acgcttcagg gtcacattcc cgtgcagtcg 2400

aacggcgtca atgtattcac gaaggcacag tggcaagctc tgaagttgta cttctcgatc 2460

ctggatctgg atggcaatgg cgttttgatg gaagagagct tcgttgttct cctcgcagag 2520

caagacagcg gtgggttgag ttaaagtgaa gtgaggatgt gaatttgttg ctaatttaaa 2580

ttgttgttgg tgttgtgtgc agatgcatac gcgactgaag acgaactgag tctgttgatg 2640

gaagtcatgg actgcaacgc cgacaatatg atcactgagc aagatttctt gctattcgca 2700

taccgagcct tactgcgatg gaaaaaagct ctacatggct ccgactagag tgtgcaggag 2760

cggaatcgaa ctgacgtagc gaagtatgaa caatgccaga ttagaatcca cagtagcgcg 2820

gcatatgccc ataatgaaga acgagaaatc tattagttga aa 2862

<210> 23
<211> 984
<212> DNA
<213> Phytophthora infestans

<400> 23
atgtccgcgc cgaccttcca tcgatcattc cgcaatttgc agcttccaag tcaacagtcc 60

aagctccaaa ccgaaacaag agtgatgatc gcaatttcga cagctgagga tttccactct 120

gccgtggccg ctgcgactca gaagccgttg gttgcctgtt tctccgcgcc gtggtgcggc 180

ggttgcaagt tggtggcgcc caaggtggac aagctagccg aggagcttgc cgagaccgtc 240

tcgtttgcca gggtgagtgc tgaggagcac gagacgttat gcgaagaagt ggaagtggac 300

agcttcccgc acttccgcgt gtacagggac ggcaagattg tgggtgacta cacgagctcc 360

aagttcgaca aggtggagtc ctttatccgc ggccatgtgg ctcctaatac tgtgcaggag 420

accgaggaaa cacctgaagg cgaggttacg gaggaaacgg cggagaagga tgctgcgaaa 480

gaggaggaag tgtcagctga ggctgcgacg gaaggctcaa agaagcgcca ggagcgtgaa 540

gaggaggaga cgaatgacga gcaggtggct aagaaggcca agacggagga ggaggcgtcg 600

acggaggctg agaaggctgc aactacggag ccagctgaga aggttgagga agcggcaacg 660

gaggagaccg agacggctga aaaggattca tcagaagaga agatcgaagc tgctcccgtt 720

gagaagtctg aaacggccgg gaaagacact gctccagtcg atgctgctgc tgcttaaacg 780

acactgctct gctttgcatg atgtctaaat cgacggacat cttctgtggg ctagctgggg 840

gctgattgat ccagctgaaa tcaagatgaa acaataaaaa aagaagtatt ttatggcgat 900

tcttttgttt gtgcccgata tcctcgacat gttctgaata catgtacatg tgctctgcct 960

tcttgtcaga acaatgtgga atgc 984

<210> 24
<211> 501
<212> DNA
<213> Phytophthora infestans

<400> 24
caagcagtga agctaaggaa caacaagatc aaacaaacca tacacctatc tacgatgcct 60

gtcgagatca acacccccga agagttcaac gccgctatcg gcgagaagaa gctgacggtc 120

gtgcagttct ctgcgccgtg gtgcggcggc tgcaagatgg tggcccccaa ggtgaccaaa 180

ctgatggagt cggacttcgc tgacgtcaag ttcctcaagg tgagcgcgga ggagctagag 240

gatttctgcg aggagatcga cgtcgacagc ttcccaacgt tccgcgtgta caaggacggc 300

gaagtggcgg cgtcttacgt gagctccaag tttgagaagg tagagcagtt catccgtgag 360

aatgcgaagt aagcgtgacg gcgatttgcg accgtctatc aaagctcgcg catggtgatc 420

tacaggcttg atgacctacg ctaagatggt ggagagcgag tgagtttggt ccccggcatt 480

gcgtatcggc agtggactga a 501

<210> 25
<211> 734
<212> DNA
<213> Phytophthora infestans

<400> 25
gacaagcagt gaagctaagg aacaacaaga tcaaacaaac catacaccca tctaagatgc 60

ctgtcgagat caacaccccc gaagagttca acgccgctat cggcgagaag aagctgacgg 120

tcgtgcagtt ctctgcgccg tggtgcggcg gctgcaagat ggtggccccc aaggtgacca 180

aactgatgga gtcggacttc gctgacgtca agttcctcaa ggtgagcgcg gaggagctag 240

aggatttctg cgaggagatc gacgtcgaca gcttcccaac gttccgcgtg tacaaggacg 300

gcgaagtggc ggcgtcttac gtgagctcca agtttgagaa ggtagagcag ttcatccgtg 360

agaatgcgaa gtaagcgtga cggcgatttg cgaccgtcta tcaaagctcg cgcatggtga 420

tctacaagct tgatgaccta cgctaagatg gtggagagcg agtgagtttg gtccccggca 480

ttgcgtatcg gcagtggact gaaggatgct actaatatat tgaaatagga aaccatttta 540

acgaagaccc tgagcctcag gctcattctc aaccggaaat atagaccgca tgcggtccca 600

ggaggacaaa tagagtcttc taattggctt acggggtagg tggtcgcatg cgacgaagtt 660

cataaaaaga cgctcatgcg gcaatttgcc cgcatgcgct aaacttgccg cacagtagag 720

tgtaggggtc gagt 734

<210> 26
<211> 1448
<212> DNA
<213> Phytophthora infestans

<400> 26
ctcgagccca agcgacaatg gcggcggtga ctggcagtgt ggtaagcgtt cagagtgtgg 60

cgcagttcga cgaggcaacg gcgcgcgagt cgacgctatc cgtgagtttc ttctgggccg 120

aatttcacga ggcctgccgt cccaatggac agctggacgt ggtggtgcgc cagctcgcga 180

ctctgcaccc tcgcatccgc ttcctcaagg tggcggccga ggagattccg gagctctcgg 240

agcgttttca gatcgccgta gtgcccactt tcgtcgtcgc acagggtcgc gccgtgctgg 300

acaaactcga gggcgccaat gtggccgaac tggccaagcg cgtggatgtt ctgagcaaga 360

gcgtggccaa gagcagctcc acatccgcgt ccacagccga ggacgccccg aaaccgctgg 420

atgacgcgct cgaataccgt ctcaagaagc tcatcagcgc gtcacccgtc atgctcttca 480

tgaagggcaa tccgacggag cccaagtgcg gctttagtcg acagatggtg gcgctgttga 540

acgaagaaaa gatccaattc ggcacctttg acatcctcaa tgacgacgaa gtgcgtcaag 600

gactcaagca gttttccaac tggcccactt accctcagct ctatgtgaat ggctcgctca 660

ttggcggact ggacatcgtc aaggagatga agtccgaggg atccatcgtc gagcaactag 720

gactcacgaa gaacgtcgag gaggctgaag ctgccttcca agagagtctc cgtgcactcg 780

tcaactccgc tccagtgctg ctcttcatga aaggccatcc gtccgagccc aaatgcggct 840

ttagcaagaa gacagtcaag ctcctacgtg accaccagat tggcttcagt tcgttcgaca 900

tcttgagtga cgagcaggtg cgtcaaggtc ttaagaagtt ctccaactgg cctacgtacc 960

cgcagctgta cgtcaagggc aaactcgttg gtgggctgga tatcctgaac gagatggcgg 1020

aggacggcga cctcagtgaa cagcttggcg tggagaagaa ggccaagaag gagaacaaat 1080

acgaacagct aatcaaccgc gctcgcgtca tgatcttcat caagggaacg ccccagcagc 1140

cgcagtgtgg cttcagtcgc aagctcgtgg acatcctgga cgccgagggc ttcaagtacg 1200

actactttga catcctcacg gacgacagcg tgcgtcaggg actgaaggag cactcgaact 1260

ggcccacgtt cccgcagctt tacgtcaatg gcgagttgat cggcggactg gacatcgtgc 1320

agcagctgca ggaggacgga gaactcgccg agctcaagga gtagacgacc aggtcatcct     1380

tcgcgtaaaa cgaatacaga aaatcatttc tcttatcctg catgtgtatg ctgggtttga     1440

tggtttaa                                                              1448

<210>  27
<211>  1106
<212>  DNA
<213>  Phytophthora infestans

<400>  27
gtcggaagag gtcgtcggtc gtccatgcag ctgcagacgt tggcgtacgc catctccggc       60

gccttcacgc tgctttccat tatcctttcg ggatggctca tctggacaca cttgctgtac      120

aatccgtcag ccggcatccg caagcacgtg atccgcatcc tcatgatggt ccccatttac      180

gcgctaacgt cctacatggc gctggtattc aacgagtcca aactgttgtt cgagactgtg      240

cgcgatctgt acgaagcctt cgcgctctat tcgtttcact gcttcctggt cgagtatctg      300

ggtggccaat ccgttctagc aagcaccatg cgctccaagc cgcagatgac acacgtcttc      360

cctttctgct gtgtacagcc gtggtccatg ggcggcaagt tcctgcgtca aaccaccatc      420

ggcatcttgc agtacattcc cattaagctg cttatgagca tcgtcatgct catcacgagc      480

ttggcaggtg tatacggaga aggagagctc atgaaccccc tagtgagcta cggctacgtg      540

tgctttatcc tcagtgcgtc gcagacgtgg gcactttact gtctgctgat attcttccac      600

ggagctcacg aggagttgca gcccatgcgg ccatggccca agttcctggc cattaaggca      660

attattttct ttacgtactg gcagtcgatc atgattagtg gacttgtaag tgtcggggtc      720

atctcggaga agtggcatat cggctgtccg gactgctggg acgctcagaa aatcgcctcc      780

gcactgaatg actttgtcat ctgcgtcgag atgctgggct ttgccattgc ccaccactac      840

gccttcgcga ttgaagactt tttgtcgccg tcaggtacgg caggtgttag tgtgccgtct      900

tcgaacgtca aggcaccact gctggccaat tttatggacg ctatcaacgt gacggacgtg      960

tcgacagacc tcaagaactc ccggaacgag attctcacca agaaacaggc gctggctgcc     1020

aagttcgagc gcatgaactc gacttcacct ggtggtggca tgttttaata gcgtcagtta     1080

aaagggagcg atacagtaag cacacg                                          1106

<210>  28
<211>  2007
<212>  DNA
<213>  Phytophthora infestans

<400>  28
atgagctcac aggacggcgc tggtcgcgca tctgaggaca agaatggaga cggcgtgtac       60

gtgacgaaga accgctcgct cttctccatg tggctgcacg gcaaggcgat tccaagccgt      120

tctggtccgg ccgtggtgtt ccgctcggcc gacgtgatac aggaagggta tttgctcaag 180

cagggcctgc gacttaaaat gtggtcccgc cgctacttta tactgcgact cgaggagcga 240

cacatgactc tagggtatta taccagcaag gactctctga ctttatgctc cgagacgccc 300

atcggaccag gacacttgtt gggacacgtc aacacgacca aatacccgcg tcgtctcgag 360

ttgcgctgcg gtacaaaggt catggtactc gaggcggagg accaaaagtc gtatgaagcc 420

tggaagaacg ccctccaaga agcgatacgc tggaaccacg ccatggtgcc ttcaaaagac 480

ggcagttttg tcacgtacgg gaagcaagcg accgaggata taaaacaaga ggaacgcagt 540

cgcgccgagg cggccaagaa gctgcgagag aagcagcgag cggacgaggc cgccgccgcc 600

gccaacgccg ccaataaacc caaatatctg cccgcgacac gccccgggac gcaatgcttt 660

atgacatcga atacaagatt tgagattccc tctcatttcg aatatgtcaa aaccatcggc 720

tcgggcgcct acggagtcgt catctccgct acgagctcgc aaacaggcac cacggtggca 780

attaagaaca tccagcgcgc gttcgacgac ctgacggacg ccaagcgcat tgtacgcgag 840

attaagctca tgcgccactt gaaccacaag tgcgtgcttg gggtggagga cattttcgag 900

cccgtggcgc tgtccaagtt cgaagacgtg tacattgtgt cccaattgat ggctacagat 960

ctccaccgcg tcatctactc gagacacgcg ctgtcggacg aacacatcgc cttcttcatg 1020

taccagatgc tgtgtgccat gaagtacgtg cactcggcca acgtgatcca ccgagacctg 1080

aagccgtcca acgtcctggt gaacgccaac tgcgagctca agatctgcga cttcggactg 1140

gcgagaggcg ttttccccga agaagagctg gaattgacag aatacgtagt cacaagatgg 1200

taccgagcgc cggagatcat gctggggtgt atgaagtaca ctcgggaggt ggacgtctgg 1260

tccatgggct gtatcttcgc cgagatgatg tcgcgcaagc ctcttttccc gggacaggac 1320

tacattgatc agctgcatct catcatgaac gcgctggggg ctccaaacga ccaggatctc 1380

tactttttga gcaacgcgcg tgctaggaag ttcatgaacg ccgagttcca gaagcgcgga 1440

cccaacccga cgaagcctct ggcgcacatg ttcgcagatt cgcctccaga cgctctggat 1500

ttactgcaga agatgctggt gattgacccg aataagcgga ttagtgtgga cgaggcgctg 1560

gcccatccgt acttggctgc gatccggaat gtggaggacg agacgaccgc cacttcgagc 1620

ttcgattttg actttgagaa cgagaaattg acgaaacccg tgctgcagag actgatctgg 1680

gacgagatga ggcatttcca ccctgaagtg ggcgacgaga cagcgacgga gggagatgac 1740

agcagtgtcg ctaccacgca ggcttctatc acacccgtga cacctgtgac ccccgctacg 1800

gtagagcaag acacaacaga gacgacaagt gacagctcgg acgcccctgt caaggtatcg 1860

acgccaacag cgtcagagga agccaagcca gaagacgaag acggggaaca acacagcacc 1920

aatagcgaca agatccatag gacagacaaa ctgacagacg cacagacgcg acaagaggcc 1980

ggcgaaccag ctcgggaagt cgcataa 2007

<210>  29
<211>  2053
<212>  DNA
<213>  Phytophthora infestans

<400>  29
gctcaagcca gagtccaaac gctcgttcaa cgcccattca acgctcgagc aacggctgat      60

accgacatga accgtaagct ggagctcatg gagctggaca gcggcggcgc cagccctaac     120

agcagcaaca gtggaagccc cagtccgact ctcgacctgc tgcatcgctc tgattcgagt     180

agcagtaacc aggggggtga gatgcctctt gtcggggttg cccacaaccc agacgtacgt     240

cgtctgcctt tctctatttg tagtgatagt attttcacgt agacgtttgt tacagctgag     300

ttatttgagg gcaactgcaa cacatggagg cagcagcacg accagcagca agtatggcag     360

ccccgaggag tcatttggtg aagaggacga ggacaacaac aagaagacgg cgcgtcgcaa     420

gaccgactcc aacagcaagc gtccgtggac acgcgaggag aatgacaagc tcatgcaact     480

cgtcaagcag tacggcgcca agcgctggtc tctcatcgcc atgcacctgc caggccgtgt     540

cggtaagcaa tgccgtgaga gatggcacaa ccacttgaat ccgtcggtcc gcaaggacgc     600

atggacggct gaagaggact atgtcatctt cgagtgccac aagaatgtgg gcaaccaatg     660

ggccgagatt tccaagatgt tgcctggcag gacggacaat gccatcaaaa accgctacta     720

ctcgaccatg cgtcgcatgc agaggcagtc aatccgcaaa aaaggtccca tgcgtgatgg     780

caagagcatc cgcgtggcgt cagtcacgtc gtctcccgtg caaaacaaca accaaatggg     840

tcctgctccc agccagcgat ctttgactgg catgcaacac cagttgccac cgcaacaaca     900

gcttccgcac cgaggggtga gtttccaatc cacatatcaa cggctcttct cggaggcctc     960

tggggacgcc gcacgcgtga gtcccgctgc ttcgaattaa ttggagagga tcgatactaa    1020

ctattatggt gttgatatat aatgcaggat ggcaactcga tagtggactt tgaacgttca    1080

aacatgatgt ccgcatcact acgacaaccc acgatggtct acccgctaaa cggaggtagc    1140

ttttcaaacg ggatgaaccc ggactatagt cgtcccatgt ccatgacgtc gtctccctgc    1200

tcggtgtccc ccgccgatga tctgaatcat aacagccaga acgagacctt tgactacgtt    1260

ccgatgcaat cttcgatcca gcgtgtacgg tcatctagtc cggttgtgat gggcactcct    1320

ccttcgacgt caataggcat gatgaactca ccttacggct cgcctgcaag tcacatccaa    1380

cagcaacagc ctggcaacta catgatggca ggaaacccat attctaacaa caacgtgcgt    1440

gggatgtact ttgggacgtc tgggcttgct cagcaatacc gtcctgatgc ccctgtaaac    1500

gtcccacaca agcgcctcct cgacgtccag ggttcacaac gtgatatgtg gaagaatgat    1560

agccctgtat cggtagcagc accgatattc cgcggtatgc cggcgacgca catgcagcaa    1620

ggtcaagtca gcgaaccgat ggcattgcag cagtccaagc cagcttccat gccactgtat    1680

aggcaagcgc cgaatatggg tcacttcaac agcatggagc aggtatggac ggatgatgca 1740

tatctgtgat tggaccgcca ttgtggcatg ctcctgatag actgcatgaa acaaatttaa 1800

agttattact taccaaagac agtggcttct cttactcttt acttggttaa cttttttcgc 1860

ttgatctaca gcggtttcac gttaggtagc tgtagctcct gatcgagttg cttattaaat 1920

agcaccactt gttgacaggg attgatatta aaatgaattt ttttcagact aatatattag 1980

ctgatattac atgtaccaaa atgtcaaact aatagctgac tattagtttg aaaaaggcgc 2040

caaaacaagc taa 2053

<210> 30
<211> 1173
<212> DNA
<213> Phytophthora infestans

<400> 30
atgcttggcg acgtagacag tttcggcggt ctgggtccca ttccgtccat gaacgcggtg 60

ttggagaagc gcacgtcaag tcagttttcc attagctcgc tgctgccccc gtccgtgagt 120

gtctcgagta ctggtgtgtt aaacacaact gttacacgga gcgagagcgc agtgacagat 180

agtgttgaag caactaccga taatgaaggc caaggatctg caccaagggt gtctaccaca 240

actgcagagg ggtctggtgg caacaccaag tacatggacc ccactacggg cgactacatg 300

tacccggaca acgtcaagcc catgagcttc tacgacaaac aacgtcttgt gcagaagatt 360

acgatgcttc ccagtcaata tctgcgtggc ctcatggacg tcatcagcaa gtaccagcca 420

gatactgtac gtcagatcga cgacgatggc tacgcgttcg atctgggtca gatgaacgag 480

aatactgtgt gggctatcag tgactacgta aaggactcga tgattgagct ggatggatac 540

attaaatcac tcaaccaaac tgctattgac cttgctgccg gtgaggatcg acatggagaa 600

actgttctga cggccgcaag tgcggctaca cccaccagca gtgcggttcg ccacttgacc 660

gacactctgg cgatgaacac gtccaagatg tctgtcatca ctaataacga gaatcagaat 720

aaattcctgg aacaggtgga gatgtattcc aaacccaaag tgaccaagtc gcgtgtaaag 780

cccagcaaga agcaccagtg tccgacgtgt aacaagcaat tccgcggacg ctccgagctg 840

cagaaccata tcagaactca cacaggcgag aagccactca aatgctcgta cgcgggatgc 900

acgaagcgat atgcacacag ctcgaatctg cgtgcacacg agcgaacaca cgccggaata 960

aagccgtata catgtcacta cgacggctgc gggaagagct tcgcccactc tgtatcactt 1020

aaggaacata tttggatgca tgcaggattc cagccctacg tgtgtccgta cgagggatgc 1080

cagaagaagt ttacgcaggt ctcaaatttt gcccgacaca agaagacgca cgagaaggaa 1140

gacaacgaac actcgattga gagcgataat tag 1173

<210>  31
<211>  754
<212>  DNA
<213>  Phytophthora infestans

<400>  31
atgattaagc tcgcggccgg tgtgtccaag aaatccgtcg tcgatgtgta tgtcactctc      60

agtgtgcccg acagccccgt acttagcacg gcacagaaga acgtggagct gaacgtcgag     120

aagttcttcg tggtcagcaa gactctgcct gaattgcctt ccaggtagaa gacgctgccc     180

gccccgacgc cttcgtcaag gcaacggatc ctcctacgtg aacgtcggtc tggaggatcg     240

tctcaactcc cgtccgcttg acctgcatac acctgccaac cagtgtatca agcgcatcca     300

ggccgctgtg ggccagctgt tccgtgcatt cctcatccag cgcgacttcg tggagaccca     360

cacacccaag ctggtcgctg gcgcctcggg gagcggagcc aactgcttca cactcaagta     420

cttcgattag gacgtcatct tggctcagag tccgcagaag tacaagcaga tggcgtgtgc     480

tgctgctggt ctcgagcgcg tgttcgagat cggcccggtc ttctgtgctg agaactcgag     540

cacgcaccgt cacatgtgcg agttcgtggg tctggatctg gagatgacta ttaaggagca     600

ctaccacgag gtcctggagg tgttctcgga cctgtacatc tacattttcg atggcttgaa     660

ggaacgttac gccaacgaat tggctactat caacaatcag tacccgttcg agccgctcaa     720

gtacattaag ccgtcgctta tcatcaactt ctag                                 754


<210>  32
<211>  659
<212>  DNA
<213>  Phytophthora infestans

<400>  32
gggctccatg tcatctgcga ctttggacgg agctctctat tgtagaattc aactgactct      60

agacaagtaa ccatgcagaa cgaggccggc cagtcgatcg acatctacat cccgcgcaag     120

tggtgcgtat tgcgctggtg actttaatac gctcagagct actggagcta actggtgctt     180

gctattgtct gtcatcgtta tatagctcgt ggaccaaccg catcctggcg gccaaggacc     240

acgcctcggt gcagatcaac gtcggccgcg tgaacgccaa cggcgtcttc acgggtgagt     300

cggacacctt cgctcttgct ggctacattc gccaccacgg tgagggcgac atggccatca     360

ccgagctggc tcgccaggcg gacgccaaga actaagcaga ccaagttgtc gtgatcgcgc     420

gctgctgctg cgaagtggag tgtgcgttca tctacgtcta gctggcagtt caggaggctg     480

gtgattacga tatgtatcaa cagatacgtc tgtgatcgct ggcaggaatt gcggtaccgt     540

cgcttatgga gtcggttgtt tgcgggttcg acctcgcctt ttgatttgat cgggtgtagg     600

cttagtattt aactctggcc aacggacgag caagaataaa gtagttcaaa cccgtcaga      659


<210>  33

<211>  1049
<212>  DNA
<213>  Phytophthora infestans

<400>  33
ctcgagcgca agacgatctc cgctatcgcc ccaccatgtt cgccagcttc gccactgccc      60

tcatcgcttc cactctccgg ccctcgagcg cagccgccac caactatgcc ggtggctggc     120

ttccgtcgga ccccaccaac cagtgtgtgg atatttgctc cgctccgtcc aagacgtgcc     180

tcacttcgga cgctgcctgc ctcgccaagt cgatggtccc gggcgacttc gactatatgg     240

tcctggagca gctcttcgtg ccgcagttct gccgtgacct gctaaagggt gtcgactcca     300

ctatttcgca ccaaaacatc gatatgtacc cgaacggcac agcctgcgtg gagagtgtgg     360

tcaagagtga gctcacgatt cacggtctgt ggcccaatta caacgatggc tacgtgagct     420

gctgcaatcc gagctcagcg gtggccaacg acccttataa cgccgctgac ttcgctgccg     480

cccaaagcag cctactcacc accatgggtg agaagtgggt ggacgctacg caagccacta     540

cgtacgaatc gctatgcgag atctacaacc acgagttcca gaagcacggt ctctgctatg     600

ccgctgacga cgctgattac atctcggctg ctgtcacgta cttcactgct acgctgagca     660

cggcggaccg catcagttca gccaccgagc agatcaacaa gtgggcagct cagtcgacgc     720

ctcagacgac tctggccgag attgaggctc tctacggcca cagtgtcatg gtgttgtgct     780

cggctgtgga tggcaacaac caactgtcgg ctatccgtac gtgctacgag aagccgacaa     840

acatcacgag cgagggagcg tccgcgcaga ttgactgcgc agccgcgacg gccacctcct     900

cgttctccgt atgctccggt gactcgccga tcactctgac tgcatacaca gcccccacct     960

cggcttcgat gcaataagct ccgacgatag agcgacggtg cagtctgcta gtataaagtt    1020

caatgcacca gttacttaaa aatgtacca                                      1049

<210>  34
<211>  1203
<212>  DNA
<213>  Phytophthora infestans

<400>  34
atgaccaagt ggggggttgt agtggcacta acactgctgt tagcgacgcc tagtttagtg      60

ctaggcgcct gccccaacaa gtgttccgga cacggcaaat gcggtctcaa tgacgtctgc     120

caatgcatgc agaactgggt cggcggtgat tgctcgggtc ggcaatgtgc gttcactcgc     180

gcgtggcaag atacagcaca gcgcgacgac gacgcacact accacgcaga atgtggcagc     240

cgcggaacgt gtgatcgagc tactggagaa tgcacatgtg acgcaggatt catcggcagc     300

ggctgtcgac gaatgcagtg tcccaatgac tgcagtggcc acggcacgtg tgagtttatt     360

gaggaattgg cgacggatac ggcccacaag cggattgggg gagtggcagg tcgtaaatac     420

acgctttggg accaagagaa gatcatgggc tgcgtatgtg acgccaacta cgaaggtcac     480

gattgctcga tgcgctcgtg tcctagaggc gacgatcctc tgaccccgaa tcaatacgac 540

atggtgcaag ctattattct tgataaggct ggcggtgagg ggtacttgac gtactatgat 600

ccgtacggca atgcgtatac tacggagaaa atcacgcttg gtggaacgct gagtgtcgct 660

tttcaaccaa cagacgatga taccacatgt gcaaacattc agaaggcgct ccgccgttta 720

ccgaacaacg tgctcaacac agtcactgtg gtagctgttg acaggtttta tgccttcaag 780

cgctctgacc caacggactc gttggggtat ggaacgttaa acaaaattgt aaatgacgac 840

gctgcagcgt acgcaggaac cggaactcaa attaaagcga tgtgcgaggt gatttttacg 900

tcggagccgg gcactacggg gtatcagaat ttgctggatt gcaatgttgc cgcgcatggc 960

gatactaagg gccagcaccc gcttactact ggtgtaacgt ctggcacttg cgtcgttaaa 1020

gaagtctatc cggtgacgct tggcacgagc aacatgcttg ccgaagatac tccagcgtat 1080

cgtccattga ccgagctcac cgagtgtgct ggtcgcggca cctgcgacta cgacacagga 1140

acatgcgagt gcttcgcggg ccacatgggc ttggcatgcc agaagcagga agcgctcgtc 1200

tag 1203

<210> 35
<211> 7947
<212> DNA
<213> Phytophthora infestans

<400> 35
atggcgcgcg ctccggcaca attggcgcag cttttactag cggcgctgct gctgtcagcg 60

atgtgtgagg cgttattgcc tatcacgaac gcgctgcagc gtgcccgcca aaacctcacg 120

gccttgcagc ggcaatggat cgatccggac acggacccca agttctacaa catatcagtc 180

cccaagggtg ccttcaactt cggtggcaac tcgagctaca acaacgagta caagctcatc 240

ttctcggacg agtttaatag ctccaagcgc acgttcgagt cgggtttcga ctccaaatgg 300

acggcagtca acctccgcga taccaccaac atggggcagc actacttttt gccccaggct 360

gtgcagatcg acaagggcaa cctcatcatc acgacctcca agcccaaaca gcgctaccgt 420

ggcactaagt acgtcagcgg tgctgtgcag acatggaaca agttctgtta caccggcggc 480

tacgtcgagg taagggccat tctgcccggc aagtggggta tccccggcac gtggcctgct 540

atctggatca tgggcaatat cggccgtgcg ccattcctcg gatcccagga cggtacgtgg 600

ccgtggagct tcgactattg cgccccctac gtggagaagg ccgagaaggt caagcagaag 660

atcaatgcct gtggcaatct gaccaacaaa cacgacaagg aatcgtatcc agagatgtac 720

ggcttgaacc cctttcaggg tcgtggagcc acggaaatcg atgtgattga agcgcagatt 780

cgtgctcgtg atgagcctgc attcatctcc acgtccctgc agatccgtcc cagtctgtac 840

gatgatatgc gcccggcttc agagtcgctc cctgctcctg gacaatggta ccagggtctc 900

aagtttggtg agttcacgaa aatcaatagc gactactacg gtgagatggg cctcgactcc 960

atttcggctc tgacacagct cgagtccaat gcgttcaagt cgttccactt gttccgcctg 1020

gactggtctc ctggtcccga aggttacatt cgatggtgga tggacaacag cttcgtgttc 1080

gaaatccctg gatccgcgct gaataagtgg gtaggtgctg tcccccctcg ccagatcccc 1140

gtggagccta gttatctcat tctgagtacg gctgtttcgg agaagttctc tccaccgtgc 1200

gatgggcaga tttgtaactc gctatggccc tccaacttta cgatcgacta cgtacgcgtc 1260

tatcaaggca acccgaaccg gtatacttcg gtgggatgca atccggaagc ctacccgacg 1320

aaagattgga tctacgccca cccagtggaa tacggtctcc cgtggtatgt ttcactacgt 1380

gtggacatcg gtctgctgca tcttttcgct gtggttaatg cgctgctggg tctcttcatg 1440

gcgttccgtg gcacgtccca accgaagatg ttgtcagcgt acgccagctc gttgtggctg 1500

actgcagcct tttatggagc tcttagttca agcgcccccg tagaccttgc ttggattcag 1560

actgcgcttg cgtgtctgtg cgggctggta cttggcggtc tttgttgctt ggtgtacccg 1620

gtttcgcttg gtgcaatgct tggactgtat ggcggggtga tagctagcca attcgtgcct 1680

ttgctgtcta cgcgcgtgat cactgcatta ctggttggtg tgggaatcgc gctaggatct 1740

gctccacaga tcgacacgaa gcacgttgtg attctgtcga cctctttgct tggtagtttg 1800

gcgtttctgc tctctgtatc gctgtgggtg agtgaaggcg atattgctga gaatgcgtgg 1860

aatctcgctg gcttcatttt caatggtaac aacgatgacc acgtgggttt ctgcacgaaa 1920

tattgcctgg cgatgtatat tctgcttgtc gtactcagtg cggtgagcac tacgtatgga 1980

tacctccgta tgcgtggcgt gacgctgcgc accaacccgc gtgaggccaa gttccccgcg 2040

gtgtcagcaa gttctgacgc gcgggcttgg agacctgatg atgatgcggg cgtggagaag 2100

actacggtga attttttctc gccatccaag ctgccgtaca acatgcagca gttcagtacc 2160

atcttccgta tcgctgtgaa cgtgcagcgc tcgtttggct tccaacttga caacttccgc 2220

aaccagacgg aacacgttgt ggtgcttctc accaacaact cgcgaaagag cggaaatcct 2280

taccgcaagc tgcacgattt ggtgttttcc aactacaaca actggtgctg caagcttaag 2340

atccagcctc tgaactgggg cgagcagcga ccaccgcagg gtggtctcac aatggtggac 2400

gagatgtcgg tggacttgtg tctgttcttc tttatctggg gtgaggccag caacctgcgt 2460

cactcgcctg agttcctgtg tttcctgttt cacaagatga aagaagagtt cccgtccgtc 2520

cgtcactcag agcgcgaggc tggatacttt ttggatacgg tggtgacacc tgtctacggc 2580

ttgctgaagg ctgagatgac ttcaaagtac gaccatgagg accgtcacaa ctacgacgac 2640

ttcaacgagt tcttctggac gaagagatgt ctgaagtatg actacaaaca cgaagaggtc 2700

attgatttgg cgtcacccaa tccggctatg atctacaaac agaagcagca gcaacgtcaa 2760

ggtctgactg gccttggagc ccaaaaggct cgaggtggac tcaacggtgg ctcgaacggc 2820

tccaacttgt tcaacaagcg tcaaagtatt gccgagggat tcaccgagtc tgccaagacg 2880

ttcgttgaga agcgtacgtg gctgctaccg ctgcgcgctt tcaaccgtat cttcaacttc 2940

cacgtcatcg cgttccactt cttggcaatg ctcgcgttcg cgaatgagca agagatggac 3000

ttccaggacg cctgcaagat tatctcgagc actttgatat ctcacttctt gctggacatt 3060

ttacgtgatg gactcgacat tttcgctgtt tacgacgagc accggaaagt attctcaatg 3120

gcgcgttccg tgatgcgtgt gtttctgcat ctggctcttg tggtggtcac gtcgatgtta 3180

tactggtatg cgtgggcgta cggtggtgcc tggtggcagt cgtactacgt gaccgcggtg 3240

ctattccacg tgccaggcct gattaactgc gtcatgcaag tgatgcctgg tcttaccaac 3300

tggacacggc gcacggcgtt tgctcctgtt gcatttatcc gtgacattgt gagtccgatg 3360

aaccgcttat acgtgggtga caacgtgctg gatccggagt cgatgagcgt aggctaccag 3420

ttcttctgga tgtcgctatt ggcttggaag ttatacttcg gctacgagtt tgagatctac 3480

ccgcttgtgg tgccgagctt tctgctgtat gctgaccacg tggagaacaa cgtgagcatg 3540

attacgacag tgttcctcat cttcctaaac tggatgccgt tcttcttggt gttctgcgtt 3600

gacattacga tttggaactc gatctggatg gcattcacgg gtacgttcgt tggcttttcg 3660

tcgcgcattg gtgagattcg caacttcacc cgcgttcgat ctgcgttcag tcgtgctgtg 3720

gatgcattta acgcaaaggt gattgcgcga agctccaaga cgggacttca actctcggac 3780

agcaatggca cgtcgtatgg atcgacatca gtaggtcacg aggtgcttga tcgtgttgcc 3840

ggtggtgcgg atccgacgtc ccgcctcctg ttgcagcgcc ggacatcagc ccatgacgac 3900

gagactccgt tactgtcttt ctcgcgtcgc aaacagacgc ctacggagcg ccaagctgct 3960

cgtcgccgca agtggttctc gttctctgtg gcctgggaca ctatcatcga tagcatgcgc 4020

gcggatgatt tgatctcaaa caaggagaaa tctctgcttc atttccaccg tcttgacggc 4080

taccagcgcg aaatttacct gccgcagttc caacttgctg gttgcttcga gaactttacg 4140

tcgcacattc ttgatattta ctcgtcgaac aacggcaagg tctcggagcg tgtgctgcaa 4200

gacaaactac tggaaattct cagtgataac ccaatggtgg aggagtcact tgaagagata 4260

tgggagcttg cgaactgggt gctggttaat gtgcttggtc cctgtcacgc gaatgatgtg 4320

aagtacatca catgtgtgct caactcgtgg gccgctcgtg gtgtgttccg tgcgctgaac 4380

ttgcaaaagg tggctccatg tggccgcgct ttggcgggtt tgatctcgct cttgaaggcc 4440

aacgtccgag gatggaagag caacgccaag gttatccctg ttcgcaaaga cccatccgac 4500

tacgcttcgt acgagtttcc gcaacagtct agctcctacc gtccttcgtc ggggcttact 4560

aagtctgcaa gtacgacagg tctgtcgtcg ttgggtctcg aaccacctcg tcgtagtcgt 4620

ggctctggtg ttgcgcgtat tgcacgtatg cagcagcaga cgcacaaacc agctgtcaac 4680

aatggcaagc ttactcattc tatctccagc tctcacatca tgcagattcg cgagcgcgtg 4740

cgtacgttcc tgaatcttgg aaaggagatt ctggcccacg tccacgagca agaccccgtg 4800

ttcgctgaaa gcaagggaat ttcggatcgg ttgacgtgga ttcttacaca ggagcgtggt 4860

tttatgtggg acgataatta tacgggtgaa caaatcactc tcacagcgtt tgagagccac 4920

accgatgtgg tgttatcgca tctgcacgga cttttgacct tgcagaagat tgatgcggag 4980

ccccagtcgt acgatgctcg tcgccgcttg ctgttcttcg tgaattcgct gttcatggac 5040

atgccgcttg ctccgctgct cgaggaaatg aagtcgtgga gcgtcatcac tccgttctat 5100

gccgaagacg ttctgtactc cagaaaggat ttggaaagca aacaggacgg tctggacgtg 5160

cacacgctgt tgttcttgca gacgctgtac aagcgagact gggagaactt cttggagcgt 5220

gtgaagccta agaagaacat ctggaaagac ccggagactg cgatcgagtt gcgtatgtgg 5280

gcttctctgc gtggccagac actgtcacgt acggtgcagg gtatgatgta cggtgaagct 5340

gccattcgtt tgctggctga gatcgaacaa gttccccaac agaaacttga ggagttgatc 5400

aacacaaagt tcacgtacgt ggtggcctgc caaatttatg gacgtcagaa gaagaacaac 5460

gacccgaagg cgagtgacat tgagtttttg ctgcaccgat tccctaactt gcgcgtggca 5520

tacatcgatg aggtccgtgt gaactaccaa aaggaacagt catacttctc ggtgctcatc 5580

aagggcggcg aggaactcgg ctcagttcac gagatctacc gcgtgcgtct gcctggcaat 5640

cctatcttgg gcgagggcaa acctgagaac cagaacgcag ccattgtttt cactcgcggt 5700

gaaaatctgc aggctatcga tatgaaccag gatggatatc ttgaagagaa cttgaagatg 5760

cgaaacctac tcgaagagtt tgacaagggt acggcagacc ggccgtacac gatcgtgggt 5820

atcccggagc acatattcac gggtagtgtg agctcgctgg caaactacat ggcgctgcag 5880

gagacgtcgt ttgtgacgct aagtcaacgt acgttggcgc gtccgctgcg tagccgtctg 5940

cactacggtc atcccgatgt gttcaacaaa cttttcttca taacgcgtgg cggtattagc 6000

aaggccagta agggtatcaa cctcagtgaa gatatctttg ctggctacaa caattgtatg 6060

cgtggcggtt ccgtgacttt cccggagtac accaagtgcg gcaaggacgt tgatgtggga 6120

atgcagcaga tctacaagtt cgaggcaaag ttagcgcagg gtgcagctga gcaatcgcta 6180

tcgcgtgacg tgtaccgtat tagccagcgt ctcgactttt tcaagttgtt gtcgttctac 6240

tacaaccatg tgggcttcta cctggcgatg tcaatcatca tctggactgt gtactttctg 6300

ctgtactgca acttattgcg tgcactgctg tcggttgagg gtgttggcgg tcgtgaaccg 6360

gtattgctaa gtaagctgca gttgatgctt ggatcggtgg cattcttcac tactgcgcca 6420

ctgctggcga cgatttcagt cgagcgtggc tttaaggcgg cgttgaacga gatcattgtc 6480

ctgttcgtga ctggaggccc gctgtacttc cttttccaca ttggcacgaa atggttctac 6540

ttcggacaga cgattcttgc tggtggcgcc aagtatcgtg cgaccggccg tggattcgtg 6600

acaaagcact cttcttttga tgagctttat cgtttctacg ctagcagcca cctctatgct 6660

gcagtggaga ttgccattgg gctttccgtt tactacaagt tcacggtcgg caatcagtac 6720

ttcgcgctga catggtcgct atggcttgtg ttcgtgtcat ggtactggtc gccgttctgg 6780

ttcaacccac tggcgttcga atggtctgac gttatggagg acttccgtct atggttcaaa 6840

tggatgcgtg gtgacggtgg taaccctgat caatcgtggg aggcgtggtt caaagaggag 6900

aacgcgtact tttcgacgct tcgaccgtgg tccaaggcgt gcattacgat caagggcgtg 6960

ctgttcgcgt tgatcgccgt ctctatctct tcgacgagtg acaaatatca ctcgatcttg 7020

acggaaacca cgtggcttcc gctgcttatc tgcttatcga tggccgcggt gtatcttagt 7080

gcagaggctg tcttcttcac ctcgtcgcgt tcgggcgaga ccgggcttgt tcgcttcctg 7140

aagctccttc tggtgattgt gctgggcgct ggtctgattc tcgctttcat ctacgcggac 7200

ggtatgtggc agatgctgct gagtatggga tatctcgctg cagctatggg ctgttgggcg 7260

cttgtgatcc ttggtagcaa ctcgcgcttt gttggaacgc tttacttcgt tcatgacgcc 7320

gtgctgggtt tggtttcgct gagtctcatt ctgttgctct cggcgctcta cgttccgggc 7380

aagatccaga catggctact gtacaataat gctttgagtc gtggcgtggt gattgaagat 7440

attctgcgag ccaactcgag caatgatgaa cgcgatgatg atctgtcagt gcagcagatg 7500

cgctccatca tccttgagca gcagcgtttc atcaacgctc tgactgctag cggcagcgag 7560

actgacatcc gcggtgctgg tcctgggaag aaagaagatc taatgcacgc tatgagcgac 7620

aacacgctga acgcggtact gaggaatatg tcggagtcgg aactcagtgc gctgcaggac 7680

tcgtcgattc gtctgcaggc catcatgtcg gaggaggagc gcaaggccgc acaacgcaag 7740

cagcaacaag aagagcagag actcaccgag gcaggaatga actcgtcgtt gtcgcgcacg 7800

cgtcgtgcct tctccacgag cgatttctcc gccatcccgc tgaatgctag cccctacagt 7860

cttgctccta ctcaggatgg ttccgctgca cccgtcaacc gccccactaa cggttctgct 7920

gctcacggag cttatcctga tgtgtag 7947

<210> 36
<211> 6744
<212> DNA
<213> Phytophthora infestans

<400> 36
atggccacga cgcccggcgg caagatggac cggcgccgcg ccggctccaa ctacttccag 60

ctcgatgcgg accgcaagcg caacacgtcc aacaaccgca gcaactacgg cgacggccgt 120

ggcaccttcg gagaccgctt gagcttgatg gaggtgcttc ctcccaacac acagggcggc 180

aaccagatgc gcggcggtgg cttacagggc gactttgcgg acgaagtgtc gatcgatttt 240

tgctgcgagg tactgtacaa caagttcggc ttccagtcag gcagtgtgga caaccagcgc 300

gagcacgtgc tgctgctatt ggccaattcc aaggcgcgtg ccaagcccca ggaccctccg 360

ggacaccacg tcgtcacgct gcacaaaaag ctcatgagca actacacgga atggtgccag 420

ttcatcggcg ttcccagcat ctcgtactcg ggacagccac agggagacct caagaaccct 480

ctgcacatgg acattatgct cttcctgttg ctatggggag aggctggtaa cttgaggcat 540

atgcctgagt gcctctgcta cttgtaccac cagtcgctaa acttgctgaa ccaggacttc 600

ctcggtcagc agaaagtacc tgaaggttgg tacttaaggc aggtggtgcg ccccatctgg 660

aaggaggcgt ctaacatgca gaggaagaac agcttgggca agaacttgga gcacacccaa 720

gtgcgcaact acgacgatat caacgagtat ttctggaaaa aatactgtct taacgtggat 780

gtcacgcaga tcggcgagga gctgaccaag aagcacacca agacatacta cgagcaccgc 840

agtatcttca cgctcgtact gaactactac cgtatttttc agttcaacat gatgttcatg 900

atggtcctga tggcgatcgg ctttatctcg gccatctcgc ctagcggagg acagcagtgg 960

ttcgctcagt tcgggtctat gggagaagtg gttgagcctt accagaaaca ggacgttaag 1020

ctgacctacg tggggattgt gttcgccctc tcctcgatgg gattctgcaa gaccgtcctc 1080

gaagcgtgtc acggatggca cttactcact gccagtgagt cgtcacagac gtcgtctcgt 1140

tcgttcaact acggtggtgc ccttgtggtt cgaatgctct ggaatggcgc gttcgctggc 1200

attttcggct tgatgatcta caccccgttg atcacgagca agaacacaga actgctcgat 1260

aaggctgcac cggcgtctgt tgcctacatc ctgcctggcg cacttattat cgtcgtgcag 1320

gcctttgctc catcggttgt aaccaaatcg tttgcggcca agtttatccg tgagggagaa 1380

acgtgctacg ttggccgcaa catggcgcct ccactgagct accagctcaa gtacatcact 1440

ttctggatca ttctgtgggc gctgaaggcg ttcgtctcat acttcattct agttcgccca 1500

ttggttctcc cgtcgctggc catttacgag atggagctcg agtacggtag caatgttgtt 1560

tcgttccaca acttcggagt catcgctgcg ctgtggctgc ctgtgatctt catcttcaac 1620

tacgataccc agatttactt taccgtgttc caggctacac ttggtggcgt tcagggtctc 1680

atcatgaaga ctggcgagat tcacggcatc aaggagatta ccaaggcttt ccgtgtggct 1740

ccacaactct ttgaccagaa ggttgtgacc aatctggctc gctcgaacga cgctgcggct 1800

gacggatctg ctgctgcata ccagtcgcaa atgatgctgc gcttcgtggt cgtctggaac 1860

gagattgtca actcgttccg cgaaggtgac ctggtggacg acaaggaggc tgccatcctg 1920

cagtatgaca tccagagctc gggcgacgtg ttcgaacctg tgttcctttc ggcgggtaaa 1980

ctgatggaag ctctggacta cacggttaag attgccaagg aaggtaaggg cgactcgcag 2040

cttcaagtgt acatggtgca gaaggattgt ctatcggctg tgcgcagctt cttcacagcc 2100

agcatgtacg tgatggaggc tctgctgggc agtgacgatg cagacattct tgatgcgctg 2160

cgtcaaatgg aggcgattgc tgcgaacagc agtttcatga gcacgtttga cgccaagagt 2220

ctagtgcagc tgcgcacagt ctcgatggag ttcctggaag ctgtgatgga tctgcccgac 2280

ccggatgcgc agtcctcgca catgacatcg tctcgagtgc acaccatggg agttgtgcgt 2340

aacttcgtga ccaagatgga aaacctgctc aacgctattc gcattttcgc caaccgcccg 2400

gagctcgctg ccaagttcag caactctaag ttctgctcga gcgccaacgg ctacgtgttt 2460

gctgctcgtg gcctcgtgaa cctgttccac aacgacactg cgatgggtgc tgctacccgt 2520

gcgtacctct tgatgtcgct cgagaaggcc gatgctatgc cccgtgtgcc tgaggctcaa 2580

cgtcgtcttg gttttttcat gaagtctctt ttgatggaca tcccgcagtt gacgtctgtg 2640

aaggagatgc actcgttctc cgtcgtgacg ccgttctaca gtgaaagtgt gttgatctca 2700

ttgtcggagc tgaacgatcc gctggccaac cacccggtct tccagaaggt ggaggagaag 2760

ggcaagaaca ttactattct gaagtatctg attaccatcc accccgagga gtgggagaac 2820

tttttggaac gtattgatgt gagcactgca gaggaagcac aagccaacta cccgctggaa 2880

atccgtctgt gggcgtcgta ccgtggtcaa accctggcac gtactgttca aggtatgatg 2940

ctgtacgagg atgctatcaa gattcttcac tggcttgaga ttggttcaag tcccggcaag 3000

tcggcggagc agaagcaggc tcaacttgag gacatggtgc gtctgaagtt ctcgtacatt 3060

tgtgcgtgtc aggtgtacgg taagcatcgc gcagagggca aggcccaggc cgatgatatc 3120

gactatctgc tcaagacgta cccgaacctg cgtgttgcct acgtcgacac catcgtgatg 3180

gacggtggca agcagttcga cacggtgttg atcaagagtg aaggcaatga aattgccgaa 3240

gtttaccgct acgagctgcc tggagacccg attcttggtg aaggtaagcc cgagaaccag 3300

aacaacgcgc ttccattcac gcgtggcgaa tacctccaga cgattgatat gaaccagcag 3360

cactacttcg aggagtgtct gaagatgccg cagcttctgg tgactgctga cctgcaccct 3420

tccaagaaac ctgtgtccat tattggtatg cgtgagcaca ttttcacggg taacgcttca 3480

tcgctgtcaa agtttaagtc gtggcaggag ctggtgttcg tgacgctgtc tcagcgtgtg 3540

ctggccgacc cgctgtatgt tcgtatgcac tacggtcacc ccgatatttt cgacaagatc 3600

attgctatgc ctcgtggtgg agtgtccaag gcttccaagg gcattaactt gtctgaggat 3660

gtgttcgctg gttttaactc gacgcttcgt ggtggtgtgg tgacgcacgt ggagttcatg 3720

cagtgtggta agggtcgtga tgtggctctg tcgcagattt ccatgttcga gggtaagctg 3780

gctaacggtg ctggcgagac gtctctcgct cgtgaggctc atcgtatggg ccagttcatg 3840

gacttcttcc gtctgaactc catgtactac tcgcacacgg gtttctactt cgccacgtgg 3900

atgacaattg tcacgacctt cgtgtacatg tactgcaagg tgtacttggc tctagcgggt 3960

gtgcagcagc agattgtgta cgatatgaac acgaccgctg tgatcaccga gaacatcgca 4020

aacaacttcg acgggcgtgt gttcaccgat ctgaaggctg tgctgaacac gcagttctac 4080

atccaagccg gtactttcct catgcttccg ctcatgtgtg tgtacttcgg tgaaggaggc 4140

ttcgtgcgcg gtatgactcg attcatcgac atgatcatca cgctgggccc tgccttcttc 4200

gtgttccagg tcggtacgac gatgcactac ttcgacaaca acatcgtgca cggtggcgcc 4260

aagtatcagg ctacaggtcg tggcttcaag atttcccgtg agacgctggt gctgctgtac 4320

aaggcgtatg caagctccca ctatcgaaag gcctgggagc tcatcgggtt gtgtcttgtg 4380

tacatggcgt tcggtaattt ttacatctgt cggaccgatg cggctgccaa cgacaacact 4440

tttgcgtccg actactgtga gactgctcag gcttacgggg tgcagacgtt ctcggtgtgg 4500

ttcatctcca tcttgtgggt ggtgggtccg ttcctcttca acagtgacgg tctggactac 4560

aggaaaacca aggtggatat ccagcagtgg tgcatgtgga tgtttgcccc cgaagactac 4620

aaggacgacg acccggccaa caagggaggc tgggtaggct ggtggaaggg cgatctggag 4680

cagctgcacg gctccaacat gatctcgcgc gtcacggtca tcctccgcga gtgtcgccac 4740

ttcctgctca tgttctacgt ggctacactc gagacgtccg acgtcatgta cgtggcgtac 4800

tcgttcggtg ctgcggtcgc gacgattgtt ctgcttggcg tgttccatgg ctttggtatg 4860

ggcatgcgct ccatgagccc ggtgacgcgc gctgtgatct acatggggac cgtcgcagcc 4920

atcgtgacgg cctacttctt ggccacttgg atcgtgctgg actggaaatt caagtacgcc 4980

atgtcgctct ggttcgccta cgtggctgcg ctctacggta tcaacgagtg cttccgcatg 5040

tggagtttcc caagctcgtc gatcgctggc attgctgtgt tccagcagct acagttcctc 5100

ttcgacttta tcttctgcat tggtatgatc atcccgcttg tggtcatgtc gtgcatcccg 5160

ttcctgaaca tcatccagac gcgtatgatg tacaacgaag gcttctccaa ggtcatgtct 5220

gcttcttcgc agtatgcctt ctctctggca gccttcatgg gaatcctggg cggtatcggt 5280

gtcggctggc tgttcaactt gctgtcaacg ctggagcagt ctgcgagttt cgcaagttac 5340

gtcgtcacgt acgatggcgt cctaagtgga aacgtgggcg atggcaccac gacgtacatg 5400

ctctacggtg cctgtgtggt gggtacgatc atcgctggct tcctcaactt cttcctgggt 5460

cgtcgtctgg ctattgttgc gggtggtctc ttcagtacgc tcggtatggt ggctgtcagt 5520

gccaacgacg atctccgttc gacgctgctg atgcctggta tcggtctgct tggagcctcg 5580

tgcggtattc tgctaccgtc gttggccatc tacatcttcg agatctcgac caaggagatg 5640

cgaggcaaag ccatgttgtt gctgggaatc ggcttcatca tcggcagttt gctgggcggc 5700

attttcgcga ccgtgaacca gctgggatgg atctggcaga cgtttgctgc ctgcatcgtg 5760

atcgccctgg tcacgccagt cgtcaatgtg ttcccagaga gtccgtactg ggtgctggat 5820

cgcaagggct gggacgcctg tgaagcctgt ctcgttatcc tgcgtcgtaa acctgatgtc 5880

caagaggagc tcaaggtcat gcgagaagag gaaacggcag atgaaggcgg cgccggcgcc 5940

tacaagttcc tcatcggtct cttcctcatg ctcgtgtcct ctctgactac tggtttcctc 6000

aacgccttca tcagctacaa gggagcgagc gagtacacgg accaagacca gctgttcgtg 6060

aacgcgatgg cgctgcagat ctccggtgct gcgatcgcca tcttctacat cgacaagctg 6120

gaccacaagt cgattctctt cggaacgttg atccctatcg ctatctgtgc tggcattctg 6180

ggcttcaacg agaactcaga gatgctggga gacaaagagg gctcgggtct gtacctcagt 6240

cttgtagtca tgctcatgta cttcttcatg gggctgggca cgagctctgc tctgtggtcc 6300

gcatgcgtcg gcatgttcaa tactcgcggc cgcgcctcgt ccaccaccat gctgttcgct 6360

atcttcttcc tggcggatat gggctacgtc tacttgcaca cggacgactc gatgatgcag 6420

aacgaatatt cctatctcta cgccatcgca ggcttcagtg tcgtcgccct ggtgctgtta 6480

atgggcgcag gcaccaagaa gaacggcgtc atctgtacca agtcagaagc tcagcgcgat 6540

cgcgaccgta tcgctcgtat gcgtgccgaa cgtgcaagtc gcaacacgcg gacgcctgga 6600

actgcgcgcc agcggaatct cagtcgcgtg cggtccaagt cgaacgctgg cggccagcgg 6660

ggcaacgcgg cgcatggcgg tggctaccaa atgtacgaaa cacccgccaa cgctgcgccg 6720

ccgctcatgc acgcgcgccc gtag 6744

<210> 37
<211> 2462
<212> DNA
<213> Phytophthora infestans

<400> 37
attcgagaga tttttcgaac tctgacatcg ttgcaaccat aacttgcctg cattaaggtg 60

ctcctcaact gtacccatgc cgagcaccca tcgactctgc cagtacgatg cttcaccaaa 120

gttttagtcc acaagaaaag ccgaaacgta catgtcgtaa cggaagccgt gcgacctttt 180

accgttcgtg cgctggtctc acgtagcttc attctccttc ttccctagcc agcaaaggcg 240

tctcccaaga tatcccagca attgtctatc ggttaagatc aaggagtaaa atgcactcgg 300

cgctgttctc gtgctcaact ttggctttga tcacgagttt ggtctcctca gagcgtgccg 360

actcgatgat caaatctcgg tctggattga gcccctgggt ggacgttgac acccccgaga 420

gtgcactcaa cgtcacgtcg tcgcgtggag atacgtggac gttggtcatg agcgacgaat 480

ttaacgttcc tgggcgtaat tttacgcccg gttcggatca catgtggacg gcgttagaaa 540

tgcccgacgg tgtcaatgca gctctcgaat actacagctt caatatgacc gataccgtaa 600

cggaatcgga cggtcgtggt gtcttccgga taaagattat ggaagaggac aacatcacct 660

acacggtgtg gaacacgtac gctaagcctg cgggtttcga gacccatcac atgtactacc 720

gagccggtat ggtgcaatcc tggaacaaat tctgtttcca aggaggaagg atggaagtgg 780

tggctcaatt accagcaaca accagctcta gtaaccccga tatgggtgac atcaaaggtc 840

gcgtcaagac gaacagcttc tatcccacgt ggcctggtat ttggcttcta ggaaatctag 900

gacgagctct attctcccaa tcgaccagtc ggatgtggcc ttggagttac gacgaatgtg 960

acgaaaaatt cgaatccagt caaagaatca gtgcgtgtga cggtaatcca ggaagtggat 1020

tgaatgctca tcaaggacga ggagcgccgg aaatagatct actggaagga ggaggtgtag 1080

ccatctccac aagtatccag gtcgctcctg gaatgcccga taaattccgt atcattgctc 1140

ccacggatga taaaagtccc ttctgcgtct tcacggccga gtgtactact attggtgcta 1200

actttcctgg gatcccagcc aaagcgtacg aagctcgaga ttatgaaagc tggtatcaag 1260

gactacggta cgctccaaat actctttgta gcccagttgg cagcttgatg caagatccca 1320

aaacggtgct tgcaaatgcc gagaaaggct tcacttctaa tacgtgtaaa ggagtcaatg 1380

cgtgtccagc ttcgggtgac ggatactccg atttaggctt gatcgacggg aaagggcctg 1440

attactgggg ggttaacaaa gaaggcggct gtatgccggt tattaacgga tacacgggag 1500

ctttcctatg tgaccctgac agttccaaca aaaaatgttc ctctccgtta ggtgcggaag 1560

aacccaagag taaagttatg gaaccattcg agtatcagat ggacgccctg tccgctaatt 1620

ggccagttca gctcgcagca tatacaagtt atgtgaaata ccaagtcgaa tgggtcatgg 1680

gctcacaagg ttacattcgc tggatggtcg aggatattgt gatttttgaa attccagccg 1740

agtcggtcga gaatgttcca caagatgctg ccaagtccaa ccctaagaaa ctcatgttgg 1800

aagaacccat gtacgtgatt ttcaacgtcg ctctgtcaac tagttggggt accacgccac 1860

cgaatccagg gtcgccatgt cgtggagatg ggagcaatgc gcaacataac gctatttgtg 1920

acggtttccc catgttcatg aagatcgact acattcgtat ctatcaggat ctctcttcca 1980

actccacgat ggctatcggg tgtgacccgt ctactcatcc gactaaacaa tggatcgagg 2040

atcacattga tgagtatgag acgacggaga ataaatggat tgaggttcac ggtggagcca 2100

attgcaagac tgataacgac tgtacggtca gtacttctca cattttgacg gggaaatgta 2160

gcaagaaaca tcgctgtaga tgcggatctt ccggtgcttg ggtggtccca cgttgcacaa 2220

ctccactagc tgatacagcg aatggagaag gcttcggacc accaacagtt attacgagct 2280

tggttggtgc attcgtcatt gtgcttttgg tctttgtggt gtacaagata atggaccaac 2340

gcagcaagaa ggcgatggtg ggagctggga ttactcaacc agcaattctc tccaagatcg 2400

agatggacga catcccacgt tcaaataaaa gccagagcgg ctcggaggat aaggtggtgt 2460

ag 2462

<210> 38
<211> 942
<212> DNA
<213> Phytophthora infestans

<400>  38
atggctccac tgccatccgc aaccttgacc acacccgacg tggctcctgt cacagctacc      60

accgcctcgc agcccttcga cgacctagtt gacaaggtcg agagtggcta tgcggctatg     120

cctaccaccc caaaggcggt ggctcccttc ccaacctcca aaccgcaagg tctccagcgt     180

ttcgccgttc agagcgtgca cctgcgcgag tgcttcgccg agttcctcgg caccttcgtc     240

atgatcgtct tcggtatggg cgtcaacaac caagtgacca actcacacga cgccaacggc     300

acgtggctca gcatcaatat gtgctggggg attggtgtac tcatcggcgt ctactgctcc     360

gagggcatca gtggtgctaa cctcaacaca gccgtgacgt tggcacattg cgtgtacggc     420

cgtctaccat ggtggaaagc acccggttac atgatctcac agctattggg cgccttctgt     480

ggagctttca ttatctacgt catgcagtac cagaacctca acgtcattga cccgtatcgc     540

gagaccacac agagcagctt ctcgacgtat ccgagcgaca acatctccaa ctacacagcc     600

ttctacaccg agttcatcgg tactgctatg cttgtgctca gtatctacgc catcacggac     660

aagcgcaaca gatctgccgg tctcgtgggt tctcccttcg ccttctgtct aatgatcatg     720

gcgttgggca tggcctttgg catgaacacg ggctacgctg tgaaccctgc acgtgacttc     780

ggcccccgca tcttcacggc catcgcgggc tggggctcca aggtctttac cactcggaac     840

tactacttct ggatcccgat cgtggccgat tctatggcgg gaattgctgg tgctggactc     900

tatcggctgc ttgtggagat ccaccaccca cccctctcgt ag                        942

<210>  39
<211>  1636
<212>  DNA
<213>  Phytophthora infestans

<400>  39
atgtccgacg accaagtgcc accgttttcg ctcggtacga aacaccttac acgcatgacg      60

tgcagcgcac agttcagaga tgactaactg gccatgctgc cgctacgtgt atttaccaga     120

taagccgcgc cacgatactt cgacgtacgt cggtcggtgg cgtaagtttg cggagctcgt     180

gtcgccaaag tacgtcaaac aaggtagacg atggaaaaag tgtggctcta aacatcatgc     240

tggggcttta ggtggctctt tctaagctcg gagcagattc agcacgcgac gcagactctg     300

gaggacttcc gcaacggtaa aatcgcccct ggtcagttca aggatgcgga actttggaat     360

cttcgccagg gtacgaaaca atgcgacagg atggtaccaa cttgtggttt ttgctgacgt     420

tttggtgata attgaatcga tatgtagcgt acgaggctgc cgtgcaccct cagactggcg     480

agacagttcc ggctgtgttt cggctgtcgg ctttcgtgcc tgtgaacatt cccatttgcg     540

ttggcatgct gctcgctcct gccacggtat gtaaagtctt gctgcacttg taacaatagc     600

tttattttaa tttaaatgct cacttaaata tgcttacagt tgggaaacac gatcttctgg     660

caatgggtta accagagcta cagcgccggt ttcaactacg ctaaccgcaa cgccagcagt     720

gaacaggaca actccaccat cttcagtacg aagttctcga tgctaatggc cttctttcag 780

cgttctaaca cggctctttg catcgtatca cagagtcgta cgctaccgcg acgctcgtgt 840

cttgctcgac tgccgttggt ctgggtaaga tggtggagaa agccaagaga ctttctccca 900

gcacgcgttc cttcctcgga aaggttaaaa ctgctaaatg ccgaatgctg tgctccttat 960

gtgactgaat ttttgggctg caacttgatc agatggttcc tttcgttgct gtcgcgagcg 1020

caggtgcttt caacgccgtg tcgatgcgct tcaacgagtt ccagtacgtg tagagtgtct 1080

tgcactggaa atatgatgct gttgtgctca ttggagtgtg gtgatgcgtt acagagaggg 1140

aattgatatc atggacgagc acggagatgt tcacgggcgt tcagttgccg ctggacgtca 1200

gtcgcttggc caagtggcac tcacgcgtat tgcgctaccc atgcccagta cgtcctcgca 1260

ttgacaagct cttcttgggt gaattatgtc taatattggt ggacttggac agttttgctg 1320

ctcccgccgt acctgtatga gatcatgaag aaaaccaaca tcatgcccaa ggccaagtac 1380

ccgaagcttg ctgcggagct cggtacgtgc attatttctg ccatagtccg cggtaatgag 1440

ctaacttatt gcgtctctgc ctgcatctat gatagtcgtg ttaacgatgt gtctgtgggg 1500

tgccatgccc agcgctgtgg cgctcttccc gcagttggga acgatttcgg ctgacagcgt 1560

tgaggaggaa ttccgatctc gggtggatcg caacggccag cccattcgtc acttcatcta 1620

caacaaggga atctga 1636

<210> 40
<211> 960
<212> DNA
<213> Phytophthora infestans

<400> 40
atgagcaccg ccaataaaga agcatctccc tttgtggagc ttcgccgtct taactttgcg 60

ttcgccaaag ttactatcgg tggccaactc atcaatcgct tgcagcatga ccccgctact 120

gctgacgcca tggaagacga cgatcgagtc ctgcgtgacg tgaatctaac gctgcagtct 180

ggtcagcgcc tgttggtcgt gggtggtaat ggagctggca aaagcacgct actcagtatc 240

ttggctggca agcacctgac ggctgacgac acggcgctga tcttcggtcg agacagcttc 300

cgtgacacga cactcaacgc tttaaggact ttcgttagtg ccgactgggg gcagcgctcg 360

gtggcgttcg caacgcacgc aatggcttac tcggctgaca tggcggtgga agaaatgatg 420

acgaaactgc agagtgaaca cccggagcga cgccaaaagt tactgaaggt gttgcgtatt 480

gacccaaagt ggcgtttaca tcgcctgtcg gacggtcaac gtcgccgcgt tcagctcttt 540

ctggcgctac tgagaccttc gcagctcatc gtactggacg aagtgctggg aatgctcgac 600

atcatctcgc gcgaaaacgt actggcattc ctaaaggagg aaacagagac tcgacaagcc 660

acggtgttgc tagccacgca catcttcgat gggacggacg tctgggcatc tcatgtgctg 720

tatattcgtc gcggtactgt tgggttctat ggtccgattc agcagtgtac tgatggaggg 780

aagatcccga tgtataaggt ggtggaacac tggctacgtg aggaattggc cgacgacgac 840

cgtgtggaat gcgaggcggt gggcccaagt ggtgagttcg acctgggcaa cgcgcagaac 900

cgcgcaggag ggtatgccga cggtcgactg ggtggtgtcg atgtcaccac atcgttttaa 960

<210> 41
<211> 510
<212> DNA
<213> Phytophthora infestans

<400> 41
atgagcgacg gtactaagct cattgccgtg atcggagacg aagacaccgt cacgggcttt 60

atcctcgcgg gtgtcggcca tcgaacggcc gagggaacca actttctcgt cgtcaaaccc 120

tgtgcgtcgg cattcccgaa aggatttttt taaagcttag attaactact aacacgcgtc 180

acatacgacg gtgctgcagc tactccaatc tccgctattg aggccagttt ccggacgctc 240

tcgagccgtg acgatatcgc aattatcctc atcaaccagc acgtacggca tcctagttag 300

aacccactct gaggtataag cctttatgtt tttctcactc gcttgatatt attgtttggg 360

caggttgctg aggagatccg tcaccttctc aacacgtacg acaagaccat tcccacggtg 420

ctagagatcc cgagcaaaga ctcgccttac gacccagcta aggactatat catgaagcgc 480

gtgaatctca tgcttggagg agagtcgtga 510

<210> 42
<211> 1491
<212> DNA
<213> Phytophthora infestans

<400> 42
atggtcgctg aagacgcggt gttcacggaa gcgaagacgc ccaaggccgg cgatgtccag 60

gccggcagat ccgtcccgct ctcgccgacc ttctggttca gcctcgcggt gctgctcctg 120

ctccccttcc agttcggctg gtcggtcggc cagctcaacc tcacgacctt caacgacgaa 180

gacgagtgca acgcgcgacc tctagtcgac ggaacgtgcc tcatgtttcc cggtcatagc 240

agcacggaat ggaccttgat cgtgaacgcg tggattgtag gcggtatgat cgggagtctc 300

ggttgtggcg ttatctccga acgtttcggt cgcaagaagg tattgctggc gaacgccgtc 360

gtcatgttgg ccggcgctgt cattcaagcg agtacgtcga gtatctcggt ttttatggtc 420

gggcgtatcg tcgccggtat cgcgtcgggt tgtgcgacgg gtatggtggg tggctacatc 480

agtgagatta cacccccgag tctccgtaac tcgtacggca cgttcatgca ggtatctctc 540

tctgccggta tcttagtagt gaccatcagt tttttcttcg cggataccag tagcggctgg 600

aggtacatcg ccgcatttcc cgttcttaac gccggtttct tcttggcgtt cgcaccgttc 660

gttcttgtag agagtcccgc gtggcttttg gaaaagggcg accgggagca tgcggagcgg 720

gagatcgcca gactttacgg cttcgatttc gtcccagtag cactgacgtg gatggaacca 780

ggtatcaata ctgacctgga gtcagaggaa ctgtgcggcg aacagcacga aggcggcaca 840

ttgtcgctgc tgttttcgcc gctctttatc aagcaactgc ttgtggctct tggtgtatca 900

gctgcgcaac aacttacggg tatcaacgcg gtgtgctatt actcgtccga tatcttctcg 960

gatgcaggga tgtcggatgg tcgtgtgggt ggcgtcatcg tgtacgtgct gatgttacta 1020

ccgacgatgg ctgttgcgcg attgtcggag cgattcggca accgacggct tctgcttact 1080

ggactggccg ggatgtttat tagtgctacg ggtataactt tggcgcttgc gttgtcggtc 1140

gaggtactct ctatcgtctt catgggtaca ttcgttgcgt tcttctctgc gagcgtgggg 1200

ccgcttatct agcctatcac ggccgcgctg ttcacggact cggtacgcgc cactgccgtc 1260

tctatgtgca tctttatcaa ttgggtatgc aacttgatca ttggcgtctg cttcccgtac 1320

gtctcggatg cgctagacga gtacaagttt gtccccttta tggtgaccac cgctgcgttt 1380

ttcttcttca ctcagttttg gatcccggag actgcaggta agagcaccga ggagatccaa 1440

gccacgttcc gatctaggaa agctcagaag ccggtggtgg tgttaagctg a 1491

<210> 43
<211> 2462
<212> DNA
<213> Phytophthora infestans

<400> 43
ttgtgctact cgagtgcctt cttctccatg acgcagcaag acgttcgcca ggcgctggac 60

gcccccgcat tagcagccta catccactcg cgactgggtg catcgcacgg cgccatcgcg 120

tccatccgtc agttccagca tggccagtcc aacccgacgt acctcttgac aatggccggc 180

tcgaaagcca agtgggtgtt gcgtaagaag ccgcacggtc agatcctgcc gtcagctcac 240

gccgtagagc gcgaatatcg tgttctagag gctctggagc actcggaggt gcccgttccc 300

cgcgctgtgt tgctatgtga agaccccaaa gtcattggca cacctttcta cctcatggaa 360

tacatacatg gtcgtatctt ccaagaccca tcgctgcctg gcattaaacc tatgtatcgc 420

tacgccatgt acagctcggc agtagacgcg ttggttaagt tgcacgagct ggactacaag 480

aagatcggac tggccgactt cggtcgcccc gagaagtact gccaccgtgt cgtgacgcgc 540

tggagtcgac aagtccaaag tggccagaag gtctttagtg aagctggagt gaaggagaac 600

ccgaagatga cgcagctgca acgttggctg gagcagaacg ccgacgacgc cgagaaggcc 660

acgaccagtg ccgagggagc gagtatcgtc cacggagact tccgcattga caacatgatc 720

ttccacccga cggagcctcg tgtgttggcc attctggact gggagctgtg tactatcggc 780

aatccgttct cggatgtagc cacattggcg tcggcctaca gactgccact ggacaactcg 840

aacacaatca tgacgcctgg tctctcggat gctccgttga agacactcgg catcccatcg 900

gagagtgaat tgttgctagg ctactgtcgt cgtgccagac ggttccctct acccactcag 960

acgtggcatt ttttcatggg gatgatcgta taccgcttcg cagccatctg ccacggtgta 1020

tacgcccgcg cactgctcgg caacgcgtcg tcggccaacg cagcatgtgc caagacgacg 1080

atggaccgac tcttggccat gagcgacgac atcatggacg catcggctga tatttacccg 1140

gagccggagc tgacgcacat tctgccgttc ccgatccgtc ctcatgctct gcagatgtac 1200

aagaagctgc taaagttttg ccagaaccga gtgtaccccg ctgagtccgt ccacattgct 1260

cagatcgcca aggcgagaga agaaggacgg gagtggcaga gtgtgcctcc tgttattgaa 1320

gagctcaaga cggaagccaa ggcgctggga ctctggaacc tcttcctgcc tcagtgtgtg 1380

gtgccagctc tggatggcaa tggaccagac gtcaactacg gtggagacct gacgaatctc 1440

gagtacggac tcatgtgtga ggtcatggga cgctccattg tgttggctcc ggaggttttc 1500

aactgctcag cacccgatac cggcaacatg gagatcttga cgcggttctg cactgtggag 1560

cagaagcatc agtggcttgt tcctcttctt caaggtgaga tccgatcgtg tttcgcgatg 1620

acggagaagc gtgtggcttc gtcggatgcg acgaacatcg agacgcgcat tgtacgcgac 1680

gaacagcgtc aagaatacgt catcaatggc cacaagttct acatctctgg agctggagac 1740

ccacgatgca agatcattgt gctcatgggc aagcacacgg aacgagccaa ggagagtcca 1800

ttcaagcagc agtcgatgat cctcgtgcct atggacacac ccggcgtgca ggtcgtgaag 1860

cccatgcatg tctttggcta cgacgacgca ccgcacggac acatggagat gctgttcaag 1920

gacgtgcgtg tgccgttcag caacgtgttg ctcggtgaag gtcgcggctt tgagatcgct 1980

caggctcgtc ttggacctgg ccgtatccac cactgtatgc gagccatcgg agctgctgag 2040

cgttgtcttg agctgatggt gcagcgagcc aagacacgca cagcgttcaa gcagctcctg 2100

gccgagaatc cgctcgtgtg ttcgcagatt gccaagtcgc gttgtgaatt ggacagcgcg 2160

cgcttgttga cgctccaggc tgcacatcag atggacaagc acggcaacaa ggtggcgcaa 2220

caggccatcg ctatgatcaa gatcgtggcg cctaacatgg cactggacgt ctgcgaccga 2280

gccatccaga tccacggcgc ttctggcgtg agtcaggact ttgtcctgtc ttacttgtac 2340

gccgccctgc gcacgctacg tatcgccgac ggaccggatg aagtgcacat gcggaccatc 2400

gcaaaactcg agctgagcca ctcgaagttg taagagaaat gtaaggcaaa agcaagcgtg 2460

aa 2462

<210> 44
<211> 20
<212> DNA
<213> Artificial Sequence

<220>

<223>  Primer S334

<400>  44
atcccactat ccttcgcaag                                                    20


<210>  45
<211>  28
<212>  DNA
<213>  Artificial Sequence

<220>
<223>  Primer S1259

<400>  45
ttgatatcgc ggaaggcgag agacatcg                                           28


<210>  46
<211>  24
<212>  DNA
<213>  Artificial Sequence

<220>
<223>  Primer S 329

<400>  46
ctaagggttt cttatatgct caac                                               24


<210>  47
<211>  28
<212>  DNA
<213>  Artificial Sequence

<220>
<223>  Primer 1

<400>  47
cgctcgaggc tggatctcgc gctgaggt                                           28


<210>  48
<211>  28
<212>  DNA
<213>  Artificial Sequence

<220>
<223>  Primer 2

<400>  48
ttgatatcgc ggaaggcgag agacatcg                                           28


<210>  49
<211>  19
<212>  DNA
<213>  Artificial Sequence

<220>
<223>  Primer Bo2299

<400>  49

gtggagaggc tattcggta                                                    19

<210>  50
<211>  20
<212>  DNA
<213>  Artificial Sequence

<220>
<223>  Primer Bo2300

<400>  50
ccaccatgat attcggcaag                                                   20

## Beschreibung

Die vorliegende Erfindung betrifft eine transgene Pflanze der Art *Solanum tuberosum* mit einer Resistenz gegenüber einem Oomyzet der Gattung *Phytophthora,* transgene Teile einer solchen Pflanze, ein Verfahren zu deren Herstellung sowie ein Mittel zur äußeren Applikation bei Pflanzen.

Die durch *Phytophthora infestans* hervorgerufene Kraut- und Knollenfäule der Kartoffel ist auch heute noch die wichtigste und wirtschaftlich bedeutendste Kartoffelkrankheit.

Weltweit führt das Pathogen zu Ertragsausfällen mit Ernteeinbußen von über 20 Prozent. Dies macht einen aufwendigen Einsatz chemischer Pflanzenschutzmittel notwendig, weil die natürlichen Abwehrmaßnahmen der Kartoffel, mit deren Hilfe *P. infestans* abgewehrt oder die Ausbreitung verzögert und einschränkt werden kann, nicht ausreichend oder nicht von Dauer sind.

Es ist bekannt, dass natürliche pflanzliche Abwehrmechanismen wie die hypersensitive Reaktion an der Infektionsstelle, die Lignifizierung der Zellwand, die Produktion von PR(pathogenesis-related)-Proteinen und die Synthese von Phytoalexinen zwar zur Resistenzsteigerung beitragen, aber stets einen Energie- und somit einen Ertragsverlust der befallenen Pflanze mit sich bringen.
Zu den in Pflanzen vorkommenden natürlichen Abwehrmechanismen zählt auch die Expression von sogenannten Resistenzgenen (R-Gene), deren Genprodukte mit mikrobiellen Avirulenz-Genen (Avr-Gene) interagieren (Gen-für-Gen-Hypothese) und damit eine spezifische Abwehrreaktion induzieren. Allerdings kann diese Resistenz gebrochen werden, wenn ein Erreger wie *P. infestans* auf die Synthese des Avr-Gen verzichten kann und die Erkennung des Pathogens und folglich die spezifische Abwehrreaktion im pflanzlichen Wirt ausbleibt.

Bereits Fire et al. (1998) konnten zeigen, dass doppelsträngige RNA (dsRNA) zum Sequenz-spezifischen Abbau homologer RNA führen kann. Ausgehend von diesen Ergebnissen wurden mittlerweile transgene Pflanzen entwickelt, die mit Hilfe von RNA-Interferenz (RNAi) über Wirtspflanzen-vermitteltes Silencing konservierter und essentieller Gene beispielsweise aus Nematoden oder *Lepidoptera-* und *Coleroptera*-Arten sowohl *in vitro* als auch *in vivo* Resistenz gegen diese Schädlinge zeigen.
Auch auf die Interaktion Wirtspflanze - phytopathogener Pilz läßt sich das Konzept des Wirt-vermittelten Gensilencing (host induced gene silencing: HIGS) zur Resistenzvermittlung anwenden (EP 1 716 238).

Van West et al. (1999) setzten die Methode des Gen-Silencing zunächst in *Phytophthora* ein, um funktionale Analysen zu diesen Oomyzeten-spezifischen Genen durchzuführen.

In WO 2006/070227 wird erstmals die Verwendung von RNA-Interferenz zur Kontrolle von pilzlichen Pathogenen basierend auf dem Kontakt von dsRNA mit Pilzzellen außerhalb der Pilzzelle beschrieben. Es wird dort ein Verfahren zur Herstellung einer pathogenresistenten Pflanze vorgestellt. Dabei kann die RNA-Interferenz gegen ein oder mehrere Gene eines Pathogens sowie gegen mehrere Pathogene gerichtet sein. *P. infestans* wird als ein mögliches pilzliches Pathogen und Kartoffel als mögliche Wirtspflanze aufgeführt.

Bisherige Arbeiten geben Anlass zur Annahme, dass Wirtspflanzen-vermitteltes Gensilencing nicht für jedes Gen funktioniert und die Auswahl der Zielgene essentiell für ein funktionales Silencing ist. So konnte beispielsweise die Plasmamembran-H+-ATPase PnMA1 in *Phytophthora parasitica* nicht ausreichend über Wirtspflanzen-vermitteltes Gensilencing reduziert werden, um einen effizienten Schutz gegenüber dem Pathogen zu vermitteln (Zhang et al. 2011). Demnach ist die Selektion der Zielgene auch für eine effektive Pathogenabwehr entscheidend (Yin et al. 2011).

Kürzlich wurde ein Screening-System vorgestellt, das die Auswahl geeigneter parasitärer Gene für Silencing-Konstrukte zur Herstellung von pathogenresistenten Pflanzen erleichtern soll (US 2010/0257634). Auch die Identifikation von geeigneten Testkonstrukten zur Vermittlung von Phytophthoraresistenz in Kartoffel wird von den Autoren ausgeführt. Dabei werden Zielgene basierend auf bioinformatorischen Analysen von Genomsequenzen oder basierend auf Sequenzhomologien zu essentiellen Genen oder Virulenzfaktoren aus bekannten Modellorganismen definiert. Ein Hinweis auf die mit der vorliegenden Erfindung offenbarten Gene zur Ausbildung einer Resistenz gegenüber einem Oomyzeten der Gattung *Phytophthora* enthält diese Druckschrift nicht.

Ein Verfahren zur Erzeugung einer Breitband-Resistenz in transgenen Pflanzen gegenüber multiplen Pilzen wird in WO 2009/112270 beschrieben. In einer Ausführungsform des erfindungsgemäßen Verfahrens ist die Breitband-Resistenz gegen *Uncinula necator, Plasmopora viticola, Uromyces spec., Phakopsora pachyrhizi, Erysiphe sp.* als auch *P*. *infestans* gerichtet.

Darüber hinaus wird die Entwicklung von *P. infestans* resistenten Kartoffelpflanzen durch RNAivermitteltes Silencing in WO 2006/047495 dargelegt. Zum einen wurden Pflanzen generiert, die für die RNA Interferenz Gensequenzen der rRNA-Gene aus *P. infestans* tragen. Das in WO 2006/047495 beschriebene gegen die rRNA-Gene von *P. infestans* gerichtete Silencing-Konstrukt umfasst die Basenpaare 1-600 der Accession Nummer AJ854293 und damit 32 bp des kodierenden Bereichs der 18S rRNA sowie den vollständigen kodierenden Bereich der 5.8 S rRNA-Gens des Krautfäuleerregers. Bei der Auswahl von Zielgenen für HIGS-Ansätze ist mit Blick auf die Anwendbarkeit entscheidend, dass es möglichst kurze vorzugsweise keine über 17 aufeinanderfolgende Basenpaare hinausgehenden Homologien zu Gensequenzen von Nichtzielorganismen gibt, da andernfalls die Genexpression der Nichtzielorganismen im Fall des Konsums der transgenen Pflanzen oder ihrer Ernteprodukte gestört werden kann ("off-target" Effekt). Die in WO 2006/047495 beschriebene Sequenz umfasst jedoch 32 bp der *P. infestans* 18S rRNA, die zu 100% identisch mit dem homologen Sequenzabschnitt der 18S rRNA-Gen aus Mensch (*Homo sapiens*), dem Schwein (*Sus scrofa*) und dem Rind (*Bos taurus*) ist. Der menschliche Kartoffelverzehr betrug 2005 in Asien 26 kg, in Nordamerika 58 kg und in Europa 96 kg pro Person (FAOSTAT). Vor dem Hintergrund hoher von Mensch und Tier konsumierten Kartoffelmengen sind die in WO 2006/047495 beschriebenen rRNA Sequenzen von *P*. *infestans* als HIGS-Zielgene aus Sicherheitsgründen für den Konsumenten ungeeignet.
Zum anderen wurden in WO 2006/047495 Pflanzen erstellt, die für die RNA-Interferenz in einem Doppelkonstrukt Gensequenzen des Kathepsin B-Gen aus *Myzus persicae* und des Elicitin-Gen INF1 aus *P. infestans* tragen und somit Resistenzen gegenüber zwei pflanzlichen Pathogenen aufweisen. Das dort verwendete Zielgen INF1 kodiert für einen Elicitor. Eine auf einem Elicitor als Pathogenitätsfaktor basierende Resistenz ist insofern nachteilig, als dass das Elicitin-Gen INF1 nicht immer für eine Infektion von Kartoffeln durch *P. infestans* notwendig ist (Kamoun et al. 1998).

Aufgabe der vorliegenden Erfindung ist es daher, eine transgene Pflanze der Art *Solanum tuberosum* bereitzustellen, die pathogenresistent gegenüber einem Oomyzeten der Gattung *Phytophthora* und die insbesondere hinsichtlich eines Konsums geeignet ist.

Erfindungsgemäß erfolgt die Lösung der gestellten Aufgabe dadurch, dass in die transgene Pflanze eine doppelsträngige erste und zweite DNA stabil integriert sind, wobei die erste DNA (a) eine Nukleotidsequenz gemäß den SEQ ID NOS: 1 - 43, oder (b) ein Fragment von mindestens 15 aufeinanderfolgenden Nukleotiden einer Nukleotidsequenz gemäß den SEQ ID NOS: 1 - 43, oder (c) eine Nukleotidsequenz, die komplementär zu einer der Nukleotidsequenzen nach (a) oder (b) ist, oder (d) eine Nukleotidsequenz, die mit einer der Nukleotidsequenzen nach (a), (b) oder (c) unter stringenten Bedingungen hybridisiert, umfasst.

Überraschend wurde gefunden, dass eine solche erste DNA in besonderer Weise geeignet ist, um über einen Wirt-vermittelten Gensilencing-Ansatz eine Pathogenresistenz in Kartoffelpflanzen auszubilden.

Dabei sind die erste und die zweite DNA stabil in das Genom einer transgene Pflanze der Art *Solanum tuberosum* integriert. Vorzugsweise sind die DNAs stabil in ein Chromosom der Pflanze integriert. Sie können aber auch in ein extra-chromosomales Element integriert vorliegen. Der Vorteil einer stabilen Integration besteht darin, dass die DNA an nachfolgende Generationen der transgenen Pflanze weitergegeben werden können.

Die doppelsträngige DNA setzt sich aus einem kodierenden und einem nicht-kodierenden Strang zusammen.

Darüber hinaus ist die Nukleotidsequenz des kodierenden Strangs der zweiten DNA zu der Nukleotidsequenz des kodierenden Strangs der ersten DNA ganz oder teilweise invers komplementär. Unter invers komplementär wird bezogen auf eine Nukleotidsequenz in 5'-3'-Richtung eine Nukleotidsequenz in 3'-5'-Richtung verstanden, dass die Basen entsprechend den Basenpaarungsregeln mit den Basen der ersten DNA korrespondieren und in umgekehrter/gespiegelter Reihenfolge aufgeführt sind. Ist die Nukleotidsequenz des kodierenden Strangs der ersten DNA beispielsweise *atggttc,* so lautet die dazu invers komplementäre Nukleotidsequenz des kodierenden Stranges der zweiten DNA *gaaccat.* Es wird auch von sense und entsprechender antisense (invers komplementärer) Orientierung der Nukleotidsequenzen gesprochen.
Insbesondere kann die Nukleotidsequenz des kodierenden Stranges der zweiten DNA zu der Nukleotidsequenz des kodierenden Stranges der ersten DNA über die gesamte Sequenzlänge invers komplementär sein. Sie kann aber auch nur teilweise invers komplementär sein, d. h., invers komplementär über eine begrenzte Länge Die Nukleotidsequenz des kodierenden Stranges der zweiten DNA kann auch in mehr als einem Bereich, beispielsweise in zwei oder drei Bereichen ihrer Nukleotidsequenz zu der Nukleotidsequenz des kodierenden Stranges der ersten DNA invers komplementär sein.

Ausgehend von den zueinander ganz oder teilweise invers komplementären Nukleotidsequenzen des kodierenden Stranges der ersten und zweiten DNA wird eine doppelsträngige RNA gebildet. Die doppelsträngige Struktur der RNA entsteht durch die Ausbildung von Wasserstoffbrückenbindungen zwischen komplementären Nukleotiden. Es können doppelsträngige RNA-Bereiche über einen einzelnen Nukleinsäurestrang, der zu sich selbst teilweise komplementär ist, oder über zwei verschiedene, diskontinuierlich komplementäre Nukleinsäurestränge gebildet werden. Die Wasserstoffbrückenbildung kann folglich intramolekular als auch intermolekular erfolgen.

Gemäß der Erfindung umfasst die erste DNA eine Nukleotidsequenz gemäß den SEQ ID NOS: 1 - 43, wobei es sich bei diesen Sequenzen um Nukleotidsequenzen von ausgewählten Zielgenen aus *P. infestans* handelt. Die Gruppe dieser Zielgene umfasst essentielle Gene des Primärmetabolismus sowie der Aminosäurebiosynthese, insbesondere der Biosynthese der aliphatischen Aminosäuren (Valin, Leucin, Isoleucin) sowie der Glutamatbiosynthese, Gene der zellulären Regulation und der Signaltransduktion wie der Redoxregulation, des Calciumsignallings, des G-Proteinsignallings, des MAP-Kinasesignallings und Transkriptionsfaktoren, aber auch Gene der Translationskomponenten, Gene mit Funktion in der RNA-Prozessierung, Gene, die für Entwicklungs- und Differenzierungsproteine kodieren wie zum Beispiel mit Funktionen im Zellwandaufbau, sowie Gene, die für Transporter, Kanäle und Membranproteine kodieren. Eine Zusammenstellung dieser Zielgene aus *P. infestans,* die für das Konzept des Wirt-vermittelten Gensilencing genutzt werden, ist in Tabelle 1 aufgeführt.

**Tabelle 1.**

| **ID** | **Zielgen_ID** | **Funktion** | **Kategorie** | **Identifizierung** |
|---|---|---|---|---|
| 1 | PITG_03410 | Acetolactatesynthase | Aminosäurebiosynthese | A |
| 2 | PITG_00375 | Haustorium-spezifisches Membranprotein (Pihmp1) | Entwicklung/Differenzierung | D |
| 3 | PITG_13490 | Urokanase | Glutamatbiosynthese | C |
| 4 | PITG_00146 | Glucose-6-P-Dehydrogenase | Primärmetabolismus | C |
| 5 | PITG_00561 | Ubiquinon-Biosynthese Protein COQ9 | Primärmetabolismus | B |
| 6 | PITG_06732 | Acyl-CoA-Dehydrogenase | Primärmetabolismus | B |
| 7 | PITG_07405 | Pyruvatkinase | Primärmetabolismus | B |
| 8 | PITG_12228 | NADH-Cytochrom B5 Reduktase | Primärmetabolismus | B |
| 9 | PITG_15476 | Malatdehydrogenase | Primärmetabolismus | B |
| 10 | PITG_18076 | Phosphoglyceratmutase | Primärmetabolismus | B |
| 11 | PITG_19736 | Alkoholdehydrogenase | Primärmetabolismus | B |
| 12 | PITG_20129/ | Acyl-CoA-Dehydrogenase | Primärmetabolismus | B |
| 13 | PITG_00221 | Tryptophansynthase | Aminosäurebiosynthese | A |
| 14 | PITG_05318 | N-(5'-phosphoribosyl)anthranilat-isomerase | Aminosäurebiosynthese | C |
| 15 | PITG_13139 | Threoninsynthase | Aminosäurebiosynthese | C |
| 16 | PITG_00578 | Imidazolonpropionase | Glutamatbiosynthese | C |
| 17 | PITG_15100 | Histidinammoniumlyase | Glutamatbiosynthese | A |
| 18 | PITG_11044 | Proteinphosphatase | Signaltransduktion | B |
| 19 | PITG_21987 | Proteinphosphatase 2C | Signaltransduktion | B |
| 20 | PITG_01957 | Calcineurin-like Katalytische Untereinheit A | Calciumsignalling | C |
| 21 | PITG_02011 | Calcineurin-Untereinheit B | Calciumsignalling | C |
| 22 | PITG_16326 | Calcineurin-like Katalytische Untereinheit A | Calciumsignalling | C |
| 23 | PITG_00708 | Thioredoxin | Redoxregulation | C |
| 24 | PITG_00715 | Thioredoxin | Redoxregulation | C |
| 25 | PITG_00716 | Thioredoxin | Redoxregulation | C |
| 26 | PITG_09348 | Glutaredoxin | Redoxregulation | C |
| 27 | PITG_08393 | PsGPR11 G-Protein gekoppelter Rezeptor | G-Proteinsignalling | D |
| 28 | PITG_10447 | SAPK-Homolog | MAP Kinasesignalling | D |
| 29 | PITG_06748 | Myb-like DNA-Bindeprotein | Transkriptionsfaktor | A |
| 30 | PITG_19177 | C2H2-Transkriptionsfaktor (PsCZF1-Homolog) | Transkriptionsfaktor | D |
| 31 | PITG_06873 | Aspartyl-tRNA-Synthetase | Translation | B |
| 32 | PITG_09442 | 40S Ribosomales Protein S21 | Translation | B |
| 33 | PITG_16015 | Ribonuklease | RNA-Prozessierung | B |
| 34 | PITG_09306 | PnMas2-Homolog | Entwicklung/Differenzierung | D |
| 35 | PITG_03335 | Kallosesynthase (Fks1/2-Homolog) | Zellwandaufbau | D |
| 36 | PITG_05079 | Glycosyltransferase (Fks1/2-Homolog) | Zellwandaufbau | D |
| 37 | PITG_18356 | Beta-Glucansynthese-assoziiertes Protein (KRE6-Homolog) | Zellwandaufbau | D |
| 38 | PITG_09193 | Aquaporin | Kanal | B |
| 39 | PITG_00562 | Mitochondrialer Tricarboxylatcarrier | Transporter | B |
| 40 | PITG_08314 | ABC Superfamilie-Protein | Transporter | B |
| 41 | PITG_12289 | ATPase H- oder Na-translokalisierender F-Tvp | Transporter | B |
| 42 | PITG_12999 | MFS Superfamilie-Transporter | Transporter | B |
| 43 | PITG_16478 | Acyl-CoA-Dehydrogenase | Primärmetabolismus | B |

Der Begriff "Gensilencing" oder Silencing beschreibt Prozesse zur Stilllegung von Genen. Silencing kann z. B. auf transkriptioneller Ebene oder post-transkriptioneller Ebene ansetzen. Das Gensilencing umfasst aber auch die antisense-Technologie, RNAi, oder dsRNA.

Die Expression einer Nukleotidsequenz eines Zielgens in *P. infestans* wird durch Gensilencing selektiv inhibiert. Eine Zielnukleotidsequenz kann dabei auch ein unprozessiertes RNA-Molekül, eine mRNA oder eine ribosomale RNA-Sequenz sein.

Die Zielgene wurden über (i) öffentlich verfügbare Expressionsstudien wie beispielsweise Microarray-Daten zu Oomyzeten-Differenzierungs- oder Infektionsprozessen und öffentlich verfügbare Daten zur Untersuchung metabolischer Prozesse während der Oomyzeten-Differenzierung oder Infektion (Grenville-Briggs et al. 2005, Judelson et al. 2009a, Judelson et al. 2009b) (A), (ii) bioinformatorische Vergleichsstudien gekoppelt mit Pedantanalysen (BioMax Bioinformatic Framework) (B), (iii) Analysen von Stoffwechselwegen gekoppelt mit Pedantanalysen (C) sowie (iv) Auswertung öffentlich verfügbarer Daten zur Charakterisierung von homologen Genen in eukaryotischen Organismen (Roemer et al. 1994, Inoue et al. 1995, Mazur et al. 1995, Lesage et al. 2004, Avrova et al. 2008, Wang et al. 2009, Li et al. 2010, Wang et al. 2010) (D) identifiziert.

Bei der Auswahl der Zielgene wurde berücksichtigt, dass die Nukleotidsequenz dieser Gene spezifisch für *P. infestans* ist, um ein unerwünschtes Silencing pflanzlicher und menschlicher Gene auszuschließen. Dazu wurden die ausgewählten Zielgene auf Proteinebene (BlastX) mit dem Proteom von *Solanum tuberosum* und *Solanum lycopersicum* verglichen. Ergänzend wurden die Zielgensequenzen auf Nukleotidebene (BlastN) mit dem Genom von *Solanum tuberosum, Solanum lycopersicum* und einem allgemeinen BlastN (Kriterien: BlastN; Database: Human genomic + transcript; Optimize for: Somewhat similar sequences (blastn)) verglichen. Zielgene gelten als sehr geeignet, wenn sie keine Homologien auf Nukleotidebene zu *Solanum tuberosum* und *Solanum lycopersicum* und keine oder nur partielle Homologien im allgemeinen BlastN in nur kurzen Sequenzbereichen (<17 nts) aufweisen, so dass eine Interaktion mit endogenen Pflanzennukleotidsequenzen verhindert wird bzw. nicht erfolgt.

Gemäß der Erfindung können die verwendeten Nukleotidsequenzen unterschiedlich lang sein. So können die Nukleotidsequenzen gemäß einer der SEQ ID NOS: 1 - 43 beispielsweise eine Länge zwischen 501 und 735 Nukleotiden aufweisen.

Bei den verwendeten Nukleotidsequenzen kann es sich aber auch um ein oder mehrere Fragmente einer oder mehrere Nukleotidsequenzen gemäß den SEQ ID NOS: 1 - 43 handeln. Die Fragmente sollten dabei mindestens 15, 16, 17, 18, 19, 20, 21, 22, 23, 24, 25, 30, 40, 50, 60, 70, 80, 90, 100, 150, 200, 250, 300, 350, 400, 450, 500, 550, 600, 650, 700, 750, 800, 850, 900, 950, 1000, 1100, 1200 oder 1300, 1400, 1500, 1600, 1700, 1800, 1900, 2000, 2100, 2200, 2300, 2400 oder 2500 aufeinanderfolgende Nukleotide einer oder mehrerer Nukleotidsequenzen gemäß den SEQ ID NOS: 1 - 43 umfassen. Bei einem besonders geeigneten Fragment handelt es sich um ein Fragment der Nukleotidsequenz gemäß der SEQ ID NO: 1 mit 290 Nukleotiden.

In einer bevorzugten Ausführung der Erfindung werden Kombinationen von zwei, drei, vier, fünf, sechs, sieben, acht, neun, zehn oder mehr Fragmenten derselben Nukleotidsequenz wie etwa der gemäß SEQ ID NO: 1 oder von verschiedenen Nukleotidsequenzen wie etwa denen gemäß der SEQ ID NOS: 1, 2, 3, 4, 5, 6, 7, 8, 9, 10, 1, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42 oder 43 verwendet. Eine bevorzugte Kombination umfasst Fragmente der Nukleotidsequenzen gemäß den SEQ ID NOS: 4, 23, 27 und 28, deren Gene die Signaltransduktion betreffen. Eine weitere bevorzugte Kombination umfasst Fragmente von Nukleotidsequenzen gemäß den SEQ ID NOS: 3, 16 und 17, wobei es sich um Gene der Glutamatbiosynthese aus *P. infestans* handelt. Weitere vorteilhafte Kombinationen umfassen Nukleotidsequenzen oder Fragmente von Nukleotidsequenzen von Genen des Zellwandaufbaus (SEQ ID NOS: 25, 36, 37), des Calciumsignalling (SEQ ID NOS: 20, 21, 22), von Genen des Primärmetabolismus (SEQ ID NOS: 5, 6, 7), von Genen der Redoxregulation (SEQ ID NOS: 24, 25, 26), oder weisen mehrere Nukleotidsequenzen oder Fragmente von Nukleotidsequenzen von Transporter-Gene gemäß SEQ ID NOS: 39, 40, 41, 42 auf. Weitere bevorzugte Kombinationen umfassen Nukleotidsequenzen oder Fragmente von Nukleotidsequenzen unterschiedlicher Zielgengruppen wie beispielsweise Gene des G-Proteinsignalling, des MAP-Kinasesignalling, des Primärmetabolismus und der Redoxregulation (SEQ ID NOS: 27, 28, 4, 23).
Durch die Kombination mehrerer Zielgene wird die Wahrscheinlichkeit vermindert, dass die Resistenz der transgenen Pflanze durch eine natürlich vorkommende Mutation in dem Oomyzet gebrochen wird.

Die in die erfindungsgemäße Kartoffelpflanze eingebrachte doppelsträngige erste DNA kann eine Nukleotidsequenz umfassen, die mit einer der folgenden Nukleotidsequenzen unter stringenten Bedingungen hybridisiert: (a) einer Nukleotidsequenz gemäß den SEQ ID NOS: 1 - 43, oder (b) einem Fragment von mindestens 15 aufeinanderfolgenden Nukleotiden einer Nukleotidsequenz gemäß den SEQ ID NOS: 1 - 43, oder (c) einer Nukleotidsequenz die komplementär zu einer der Nukleotidsequenzen nach (a), (b) oder (c) ist. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde. Der hier verwendete Begriff "stringente Hybridisierungsbedingungen" kann auch bedeuten: Hybridisieren bei 68 °C in 0,25 M Natriumphosphat, pH 7,2 7 % SDS, 1 mM EDTA und 1 % BSA für 16 Stunden und anschließendes zweimaliges Waschen mit 2 x SSC und 0,1 % SDS bei 68 °C.

Von der vorliegenden Erfindung erfasst sind insbesondere auch solche Fragmente von Nukleotidsequenzen, die über einige wenige, beispielsweise 1 oder 2 Nukleotide verfügen, die nicht komplementär zu der Zielgensequenz aus *P. infestans* sind. Im Oomyzeten auftretende Sequenzvariationen, die beispielsweise auf einer genetischen Mutation durch z.B. Addition, Deletion oder Substitution oder auf einem Polymorphismus in einem *Phytophthora infestans-*Stamm basieren, und welche in einer Falschpaarung über einen Bereich von 1, 2 oder mehr Nukleotiden führen, können somit toleriert werden, sofern die von der transgenen Kartoffelpflanze gebildete RNA immer noch mit der vom Oomyzet gebildeten Zielgen-RNA interferiert.

Gemäß der Erfindung bildet die transgene Pflanze der Art *Solanum tuberosum* eine Pathogenresistenz gegenüber einem Oomyzeten der Gattung *Phytophthora* aus. Zur Ermittlung der Resistenz wird dabei die transgene Kartoffelpflanze mit einer Kontrollpflanze verglichen, die idealer Weise den identischen Genotyp wie die transgene Pflanze hat und unter identischen Bedingungen angezogen worden ist, welche aber nicht die DNA enthält, die in die transgene Pflanze eingebracht wurde. Die Ermittlung der Resistenz kann über eine optische Bonitur, wobei Boniturnoten von 0 (nicht anfällig) bis 100 (sehr anfällig) vergeben werden, erfolgen. In bevorzugter Weise bildet die transgene Pflanze gemäß der Erfindung eine Resistenz aus, die im Vergleich zu einer Kontrollpflanze zu eine verringerter Ausbreitung der Infektion auf der Pflanzenoberfläche um mindestens 10, 15, 20, 25, 30, 35, 40, 45, 50, 55, 60, 65, 70, 75, 80, 85, 90, 95, 100 Prozent führt (siehe die Bedingungen unter Messung der Resistenz in transgenen Kartoffelpflanzen unter Freilandbedingungen).

Die Gattung *Phytophthora* umfasst verschiedene Arten, beispielsweise die Arten *alni, cactorum, capsici, cinnamomi, citrophthora, clandestina, fragariae, hedraiandra, idaei, infestans, ipomoeae, iranica, kernoviae, mirabilis, megakarya, nicotianae, palmivora, parasitica, phaseoli, ramorum, pseuodotsugae, quercina, sojae* oder *tentaculata.*

Nach einer bevorzugten Ausgestaltung der Erfindung weist die transgene Kartoffelpflanze eine Resistenz gegenüber *Phytophthora infestans* auf.

Bereits die Hemmung der Biosynthese der aliphatischen Aminosäuren Valin, Leucin und Isoleucin durch ein gegen die Acetolactatsynthase von *P. infestans* gerichtetes Konstrukt führte zu einer starken Reduktion des Krautfäulebefalls und zu einer drastischen Erhöhung der Blattresistenz unter Labor- und Feldbedingungen. Durch Kombination mehrerer Zielgene, wie beispielsweise Gene aus G-Proteinsignalling, MAP Kinasesignalling, Primärmetabolismus, Aminosäurebiosynthese und Redoxregulation, in einem Konstrukt, kann die Resistenzwirkung noch erhöht werden, da der Pathogen durch die multiple Wirkung der Kombinationskonstrukte an der Nutzung alternativer Signal- und Stoffwechselwege gehindert wird.

Überraschenderweise ist die Abwehrleistung der erfindungsgemäßen Kartoffelpflanzen gegenüber Isolaten unterschiedlicher Aggressivität von *P. infestans* so verändert, dass eine Resistenz erzielt wird, welche die Pflanze effizient und dauerhaft gegenüber diesem wichtigsten Pathogen schützt.

Auch wurde überraschend gefunden, dass die Resistenz keine hohen energetischen Einbußen oder negative Veränderungen der agronomischen Eigenschaften der Kartoffelpflanze bewirkt. Die Anbauversuche unter feldnahen Bedingungen zeigten keine Beeinträchtigungen der Qualität der Pflanze. Durch die sorgsame Auswahl der Zielgene von *P. infestans,* die eine über 17 aufeinanderfolgende Basenpaare hinausgehende Homologie zu Genen von Nichtzielorganismen (Kartoffel, Mensch, Schwein, Rind) ausschloss, gibt es keine Beschränkung der Pflanzen zur Verwendung als Futter- oder Nahrungsmittel sowie keine Einschränkungen bezüglich Aussaat, Anbau, Ernte oder Prozessierung des Ernteguts. Die Pflanzen können uneingeschränkt als Nutz-, Nahrungs- oder Futtermittelpflanze verwendet werden.

Nach einer bevorzugten Ausgestaltung handelt es sich bei der doppelsträngigen RNA um miRNA oder siRNA.
MiRNA beschreibt kurze interferierende RNA und umfasst natürlich produzierte miRNAs und synthetische miRNAs, welche beispielsweise über rekombinante oder chemische Synthese oder über die Prozessierung von Prä-miRNA hergestellt werden können.
SiRNA beschreibt kurze interferierende RNA und umfasst natürlich produzierte siRNAs und synthetische siRNAs, welche beispielsweise über rekombinante oder chemische Synthese oder über Prozessierung von dsRNAs hergestellt werden können.

Die transgene Pflanze produziert ausgehend von der eingebrachten doppelsträngigen DNA dsRNA, die durch endogene RNAi- bzw. Silencing-Mechanismen zu siRNAs und miRNAs prozessiert werden.

Um dsRNA zu erhalten, kann eine doppelsträngige erste DNA mit einer Nukleotidsequenz gemäß einer der SEQ ID NOS: 1 - 43 oder einem Fragment davon in sense und einer doppelsträngigen zweiten DNA in antisense Orientierung verwendet werden, die durch ein Intron getrennt sind, das keine Ähnlichkeit mit den betreffenden Zielgenen hat. Beispielsweise kann die DNA mit einer Nukleotidsequenz gemäß SEQ ID NO: 1 gegen das Acetolactatsynthasegen aus *P. infestans* gerichtet sein. Bei Expression in einer pflanzlichen Zelle wird ein RNA-Transkript gebildet, welches sich aufgrund der Homologie zwischen den sense und antisense Sequenzbereichen zu einer dsRNA zusammenlagern kann. Durch die fehlende Basenpaarung im Bereich des Introns bildet die dsRNA eine hairpin-Struktur aus. Eine dsRNA mit einer hairpin-Struktur kann auch durch eine doppelsträngige DNA mit einer Nukleotidsequenz gemäß einer der SEQ ID NOS: 1 - 43 in sense und einer zweiten in antisense Orientierung unterschiedlicher Länge bereitgestellt werden. Dabei kann die Nukleotidsequenz in sense Orientierung beispielsweise um 190 Nukleotide länger sein als die Nukleotidsequenz in antisense Orientierung oder *vice versa.*

Definierte Sequenzbereiche der ausgewählten Nukleotidsequenzen der Zielgene werden durch PCR amplifiziert und sowohl in sense als auch in antisense Richtung in einen Vektor kloniert, der zur Synthese von hairpin-Strukturen geeignet ist. Dabei können auch mehrere Fragmente mit Sequenzbereichen verschiedenerer Zielgene in einen Vektor kloniert werden, um ein Kombinations-hairpin-Konstrukt zu generieren. Die Vektoren können über in der Pflanzenbiotechnologie bekannte Transformationsverfahren in eine Pflanzenzelle eingebracht werden. Der Fachmann weiss, dass er beispielsweise auch eine ausgewählte Nukleotidsequenz eines Zielgenes in sense Orientierung in einen Vektor und die Nukleotidsequenz des Zielgens in antisense Orientierung in einen zweiten Vektoren klonieren kann, welche dann zum Beispiel über Co-Transformation in eine Pflanzenzelle eingebracht werden.

Der Silencing-Mechanismus geht von dsRNA wie beispielsweise hairpin-RNA-Strukturen oder Genduplexen aus. Die dsRNA wird mittels einer dsRNA-spezifischen Endonuklease (Dicer) zu kurzen dsRNAs führen, die bei Einsatz längerer Nukleotidsequenzen zu kurzen dsRNAs von vorzugsweise zu 21 - 25 Basenpaaren prozessiert werden, ein Prozess der sowohl für "stemloop" (Prä-miRNA) als auch für lange komplementäre dsRNA-Vorstufen ähnlich abläuft. Argonaut-Proteine als zentrale Komponenten des RNA-induzierten Silencing Complexes (RISC) binden und entwinden siRNA und miRNA, so dass der Leitstrang des Duplexes mittels Basenpaarung gezielt an die mRNA bindet und zu deren Abbau führt. RNAi mittels miRNA bezieht sich auf einen vergleichsweise ähnlichen Prozess, mit dem Unterschied, dass die produzierte miRNA auch partiell Regionen umfasst, die nicht identisch zu den Zielgenen sind.

Nach Befall einer Wirtspflanze mit *P. infestans* kann ein Austausch von in der Pflanze gebildeter RNA, welche gegen ein oder mehrere *Phytophthora-spezifische* Zielgensequenzen gerichtet ist, zwischen der Wirtspflanze und dem Oomyzeten stattfinden. In dem Oomyzeten können diese RNAs zu einem Sequenz-spezifischen Gensilencing von einem oder mehreren Zielgenen führen. An diesem Prozess können auch Proteine und Proteinkomplexe wie Dicer, RISC (RNAinduzierter Silencing-Komplex) sowie die RNA-abhängige RNA-Polymerase (RdRP) beteiligt sein.

Es ist bekannt, dass in Pflanzen der siRNA-Effekt fortgeführt werden kann, wenn die RdRP neue siRNAs aus den abgebauten mRNA-Fragmenten synthetisiert. Diese sekundäre oder transitive RNAi kann das Silencing verstärken und auch zum Silencing verschiedener Transkripte führen, wenn diese hochkonservierte Sequenzen teilen.

In einer bevorzugten Ausführung sind die erste DNA und die zweite DNA mit mindestens einem Promotor operativ verknüpft.

Ein "Promotor" ist eine nicht-translatierte DNA-Sequenz, typischerweise stromaufwärts einer kodierenden Region, welche die Bindestelle für die RNA-Polymerase beinhaltet und die Transkription der DNA initiiert. Ein Promotor enthält spezielle Elemente, die als Regulatoren der Genexpression fungieren (z.B. *cis*-regulatorische Elemente). Mit operativ verknüpft ist gemeint, dass die DNA, welche die integrierte Nukleotidsequenz umfasst, verbunden ist mit einem Promotor in einer Weise, die eine Expression dieser Nukleotidsequenz erlaubt. Als weitere Komponente kann die integrierte Nukleotidsequenz stromabwärts mit einem Terminator-Signal verknüpft sein.

Der Promotor kann pflanzlichen, tierischen oder mikrobiellen Ursprungs oder synthetisch hergestellt sein und kann beispielsweise aus einer der folgenden Gruppe von Promotoren ausgewählt werden: konstitutiv, induzierbar, entwicklungsspezifisch, zelltypspezifisch, gewebespezifisch oder organspezifisch. Während konstitutive Promotoren unter den meisten Bedingungen aktiv sind, zeigen induzierbare Promotoren Expression infolge eines induzierenden Signals, welches beispielsweise von biotischen Stressoren wie Pathogenen oder abiotischen Stressoren wie Kälte oder Trockenheit oder Chemikalien ausgehen kann.

Als Promotoren kommen beispielsweise der konstitutive CaMV 35S Promotor (Benfey et al., 1990) sowie der in grünen Geweben aktiven C1 Promotor (Stahl et al., 2004) in Betracht.

Die erste und zweite DNA können aber auch mit einem doppelten Promotor operativ verknüpft sein, wie z.B. dem bidirektional aktiven TR1' und TR2'Promotor (Saito et al., 1991).

Des Weiteren können die erste und die zweite DNA mit jeweils einem Promotor operativ verknüpft sein.

Die Verwendung von zwei Promotoren, die jeweils das 3'- und das 5'-Ende des Nukleinsäuremoleküls flankieren, ermöglicht die Expression des jeweiligen individuellen DNA-Strangs, wobei zwei komplementäre RNAs gebildet werden, die hybridisieren und eine dsRNA bilden. Zudem können die zwei Promotoren derart eingesetzt werden, dass der eine Promotor auf die Transkription einer ausgewählten Nukleotidsequenz und der zweite Promotor auf die Transkription einer zu der ersten Nukleotidsequenz komplementären Nukleotidsequenz gerichtet ist. Sofern beide Nukleotidsequenzen transkribiert werden, entsteht eine dsRNA.

Ferner kann ein bidirektionaler Promotor eingesetzt werden, der eine Expression von zwei Nukleotidsequenzen in zwei Richtungen ermöglicht, wobei eine Nukleotidsequenz in 3'-Richtung und eine zweite Nukleotidsequenz in 5'-Richtung abgelesen wird. Sofern die beiden Nukleotidsequenzen komplementär zueinander sind, kann eine dsRNA gebildet werden.

Die vorliegende Erfindung betrifft auch Teile einer transgenen Pflanze der Art *Solanum tuberosum.*

Im Sinne dieser Anmeldung sind mit "Teilen" der transgenen Pflanze insbesondere Samen, Wurzeln, Blätter, Blüten sowie Zellen der erfindungsgemäßen Pflanze gemeint. Dabei sind unter "Zellen" beispielsweise isolierte Zellen mit einer Zellwand oder Aggregate davon oder Protoplasten zu verstehen. Bei den transgenen Teilen der transgenen Pflanze handelt es sich auch um solche, die geerntet werden können, wie etwa die Kartoffelknollen.

Die vorliegende Erfindung betrifft weiterhin ein Verfahren zur Herstellung einer transgenen Pflanze der Art *Solanum tuberosum,* die eine Resistenz gegenüber einem Oomyzeten der Gattung *Phytophthora* aufweist.

Geeignete Verfahren zur Transformation von Pflanzenzellen sind in der Pflanzenbiotechnologie bekannt. Jedes dieser Verfahren kann genutzt werden, um eine ausgewählte Nukleinsäure vorzugsweise in einem Vektor in eine Pflanzenzelle einzubringen, um eine transgene Pflanze gemäß der vorliegenden Erfindung zu erhalten. Transformationsverfahren können direkte und indirekte Verfahren der Transformation beinhalten und sind für dikotyle und zumeist auch für monokotyle Pflanzen anwendbar. Geeignete direkte Transformationsverfahren schließen PEGinduzierte DNA-Aufnahme, Liposomen-vermittelte Transformation, biolistische Methoden mittels Particle Bombardement, Elektroporation oder Mikroinjektion ein. Zu den indirekten Verfahren zählen beispielsweise die Agrobakterium-vermittelte Transformationstechnik oder die virale Infektion mittels viraler Vektoren.

Ein bevorzugt eingesetztes Verfahren ist der Agrobakterium-vermittelte DNA-Transfer unter Verwendung binärer Vektoren. Nach der Transformation der Pflanzenzellen werden die Zellen auf einen oder mehrere Marker selektiert, die mit der DNA der Erfindung in die Pflanze transformiert wurden und Gene umfassen, die vorzugsweise Antibiotika-Resistenz vermitteln, wie z.B. das Neomycinphosphotransferase II-Gen *NPTII,* welches Kanamycinresistenz vermittelt oder das Hygromycinphosphotransferase II-Gen *HPTII,* welches Hygromycinresistenz vermittelt.

Im Anschluss werden die transformierten Zellen zu vollständigen Pflanzen regeneriert. Nach dem DNA-Transfer und der Regeneration können die erhaltenen Pflanzen beispielsweise über quantitative PCR auf das Vorhandensein der DNA der Erfindung überprüft werden. Resistenzprüfungen dieser Pflanzen gegenüber *P. infestans in vitro* und im Gewächshaus schließen sich an. Weitere phänotypische Untersuchungen können im Gewächshaus oder im Freiland routinemäßig von entsprechend geschulten Personen durchgeführt werden. Die auf diese Weise untersuchten transformierten Pflanzen können direkt angezogen werden.

Das erfindungsgemäße Verfahren zur Herstellung einer transgenen Pflanze der Art *Solanum tuberosum,* die eine Resistenz gegenüber einem Oomyzeten der Gattung *Phytophthora* aufweist, umfassent die Schritte:
(i) Herstellen einer transformierten ersten Elternpflanze enthaltend eine doppelsträngige erste DNA, die stabil in das Genom der Elternpflanze integriert ist und die (a) eine Nukleotidsequenz gemäß den SEQ ID NOS: 1 - 43, oder (b) ein Fragment von mindestens 15 aufeinanderfolgenden Nukleotiden einer Nukleotidsequenz gemäß den SEQ ID NOS: 1 - 43, oder (c) eine Nukleotidsequenz, die komplementär zu einer der Nukleotidsequenzen nach (a) oder (b) ist, oder (d) eine Nukleotidsequenz, die mit einer der Nukleotidsequenzen nach (a), (b) oder (c) unter stringenten Bedingungen hybridisiert, umfasst,
(ii) Herstellen einer transformierten zweiten Elternpflanze enthaltend eine doppelsträngige zweite DNA, die stabil in das Genom der Elternpflanze integriert ist, wobei die Nukleotidsequenzen des kodierenden Stranges der ersten und zweiten DNA zueinander ganz oder teilweise invers komplementär sind,
(iii) Kreuzen der ersten Elternpflanze mit der zweiten Elternpflanze,
(iv) Auswählen einer solchen Pflanze, in deren Genom die erste DNA und die zweite DNA zur Ausbildung einer Pathogenresistenz gegenüber einem Oomyzeten der Gattung *Phytophthora* integriert sind, so dass ausgehend hiervon eine doppelsträngige RNA gebildet werden kann.

Erfindungsgemäß handelt es sich um eine Nukleotidsequenz oder ein Fragment einer Nukelotidsequenz gemäß den SEQ ID NOS: 1 - 43 aus *P. infestans.*

In einer bevorzugten Ausgestaltung des Verfahrens kann es sich bei der doppelsträngigen RNA um miRNA oder siRNA handeln.

Die Erfindung betrifft auch ein Mittel zur äußeren Applikation bei Pflanzen.

Dieses Mittel wird zur äußeren Applikation bei Pflanzen bereitgestellt. Es enthält doppelsträngige RNA, wobei ein Strang dieser RNA dem Transkript einer doppelsträngigen DNA umfassend (a) eine Nukleotidsequenz gemäß den SEQ ID NOS: 1 - 43, oder (b) ein Fragment von mindestens 15 aufeinanderfolgenden Nukleotiden einer Nukleotidsequenz gemäß den SEQ ID NOS: 1 - 43, oder (c) eine Nukleotidsequenz, die komplementär zu einer der Nukleotidsequenzen nach (a) oder (b) ist, oder (d) eine Nukleotidsequenz, die mit einer der Nukleotidsequenzen nach (a), (b) oder (c) unter stringenten Bedingungen hybridisiert, entspricht.

Doppelsträngige RNA zur Herstellung des erfindungsgemäßen Mittels kann *in vitro* über dem Fachmann bekannte Methoden produziert werden. Beispielsweise kann die Synthese der doppelsträngigen RNA synthetisch erfolgen, wobei die RNA *in vitro* direkt gebildet wird. Die doppelsträngige RNA kann auch ausgehend von einer doppelsträngigen DNA über die Bildung eines mRNA-Transkriptes, welches dann z. B. eine haipin-Struktur ausbildet, synthetisiert wird. Das erfindungsgemäße Mittel kann als Fungizid für eine Pflanze oder deren Saatgut verwendet werden. Dabei ist das Mittel zur Kontrolle des Pathogenwachstums, zur Eindämmung der Pathogenausbreitung oder zur Behandlung von befallenen Pflanzen einzusetzen. Beispielsweise kann das Mittel als Fungizid durch Aufsprühen in Form eines Sprays oder weiteren dem Fachmann geläufigen Wegen der äußeren Applikation auf das Pflanzengewebe oder durch Aufsprühen oder Mischen mit dem Aufzuchtsubstrat vor oder nach Aufgang der Pflanzen eingesetzt werden.

In einer weiteren Anwendung kommt das erfindungsgemäße Mittel zur Vorbehandlung des Saatguts zum Einsatz. Hierzu wird das Mittel zunächst mit einer Trägersubstanz verbunden und in einer Kombination, welche die doppelsträngige RNA und die Trägersubstanz umfasst, auf das Saatgut aufgebracht, wobei die Trägersubstanz beispielsweise RNA-stabilisiernde Wirkung hat. So kann die RNA-Stabilität und damit ihre Wirkung gegenüber den ausgewählten Zielgenen von *P. infestans* zum Beispiel über chemische Modifikationen, wie den Austausch von Ribose gegen eine Hexose, erhöht werden. Als RNA-Stabilisatoren können auch Liposomen, welche die RNA-Moleküle verkapseln, eingesetzt werden.
Idealer Weise handelt es sich bei den mit dem Mittel behandelten Pflanzen um solche der Art *Solanum tuberosum.*

Die obigen Ausführungen zur erfindungsgemäßen Pflanze sowie zu dem erfindungsgemäßen Verfahren gelten entsprechend auch für dieses Mittel.

Die vorliegende Erfindung wird nachfolgend mit Bezug auf die Figuren und Sequenzen beschrieben:
Fig. 1: Plasmid pRNAi als beispielhafte Darstellung eines Vektors, der zur Bildung von hairpin-Konstrukten gegen ein Zielgen genutzt werden kann. Dieser Vektor enthält einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator.
Fig. 2: Plasmid pRNAi_PITG_03410 als beispielhafte Darstellung eines Vektors, der ein sense-Intron-antisense-Fragment zur Bildung von dsRNA gegen ein Zielgen (hier PITG_03410) enthält. Dieser Vektor enthält zusätzlich einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator.
Fig. 3: Plasmid pRNAi_HIGS_CoA als beispielhafte Darstellung eines Vektors, der verschiedene definierte Sequenzen im sense-Intron-antisense-Fragment enthält, das zur Bildung von dsRNA gegen diverse Zielgene führen soll. Dieser Vektor enthält zusätzlich einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator.
Fig. 4: Plasmid pGBTV/EcoRI_kan. Binäres Ti-Plasmid, das als Klonierungsvektor eingesetzt wurde.
Fig. 5: Plasmid pGBTV/EcoRI_kan_PITG_03410. Binäres Ti-Plasmid, das für die Agrobakterien-vermittelte Transformation eingesetzt wurde.
Fig. 6: Plasmid pAM, das als Klonierungsvektor eingesetzt wurde.
Fig. 7: Plasmid pAM_HIGS_CoA, als beispielhaftes Plasmid, das als Klonierungsvektor eingesetzt wurde.
Fig. 8: Plasmid p95P-Nos. Binäres Ti-Plasmid, das als Klonierungsvektor eingesetzt wurde.
Fig. 9: Plasmid p95N_HIGS_CoA. Binäres Ti-Plasmid, das für die Agrobakterien-vermittelte Transformation eingesetzt wurde.
Fig. 10: Plasmid p95N_HIGS_dPRNAi_PITG_03410 als beispielhafte Darstellung eines binären Vektors zur Bildung von dsRNA gegen ein Zielgen (hier PITG_03410) unter Verwendung von zwei CaMV 35S Promotoren, die jeweils das 3'- und das 5'-Ende des Nukleinsäuremoleküls flankieren.
Fig. 11: Transgene Kartoffel-Sprosse auf Selektionsmedium nach der Transformation im Stadium der Regeneration.
Fig. 12: Diagnostische PCR zur Überprüfung der Transgenität der Kartoffeln (PR-H4) nach Transformation mit dem binären Vektor pGBTV/EcoRI_kan_PITG_03410.
   Nachweis des sense Fragments (370 bp) (Primer S334 5'-ATCCCACTATCCTTCGCAAG-3' x S1259 5'-TTGATATCGCGGAAGGCGAGAGACATCG-3') und des antisense Fragments (450 bp) (S 329 5'-CTAAGGGTTTCTTATATGCTCAAC-3' x S1259 5'-TTGATATCGCGGAAGGCGAGAGACATCG-3'). Mix: PCR-MasterMix, PCR-Kontrolle. Marker: Tracklt™ 1 Kb DNA Ladder.

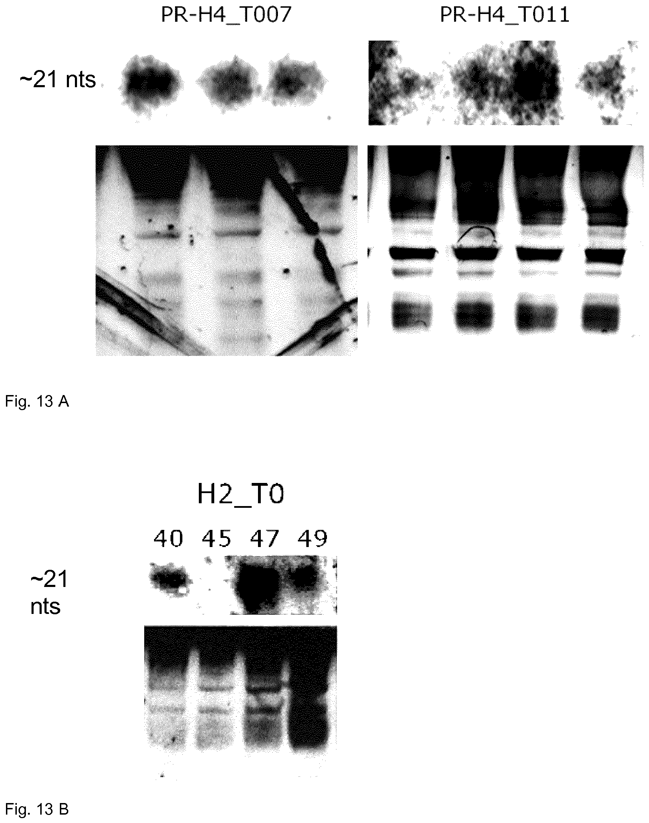

Fig. 13: Detektion siRNAs in transgenen Kartoffelpflanzen nach Transformation mit dem binären Vektor pGBTV/EcoRI_kan_PITG_03410 (A) und dem binären Vektor p95N_HIGS_PITG_00375 (B). Der Nachweis erfolgte mittels Hybridisierung der Northern Blots mit der radioaktiv markierten Sonde dsRNA_PITG03410 (A) bzw. mit der radioaktiv markierten Sonde dsRNA_PITG00375 (B). A: Mehrfache Auftragung verschiedener Proben der Linien PR-H4_T007 und T011. B: Einfache Auftragung der Proben der Linien PR-H2_T040, T045, T047 und T049.

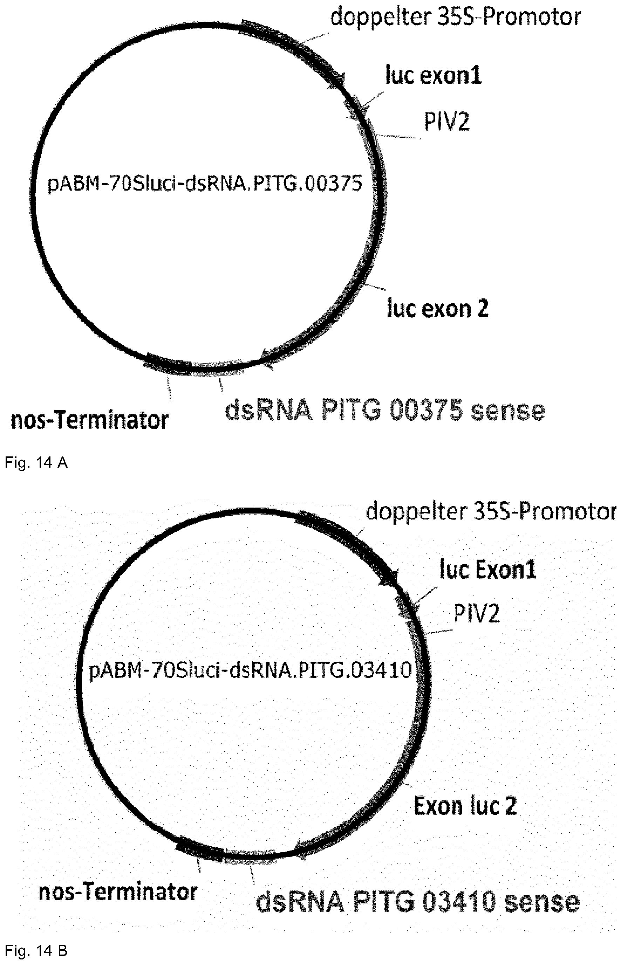

Fig. 14 A: Plasmid pABM-70Sluci_dsRNA.PITG_00375 als beispielhafte Darstellung eines Vektors, der ein Fusion-Konstrukt bestehend aus einem Luciferase-Reportergen und dem zu testenden HIGS-Zielgen-Fragment PITG_00375 enthält. Der Vektor enthält zusätzlich einen doppelten CaMV 35S-Promotor, eine multiple Klonierungsstelle, die kodierende Sequenz des Gens *luc* aus *Photinus pyralis,* das für eine Luciferase kodiert, getrennt von einem modifizierten Intron PIV2 aus dem Kartoffel-Gen *St-LS1* (Eckes et al. 1986, Vancanneyt et al. 1990), eine weitere multiple Klonierungsstelle sowie einen Nos-Terminator des Nopalin Synthase-Gens aus *Agrobacterium tumefaciens.*
Fig. 14 B: Plasmid pABM-70Sluci_dsRNA.PITG_03410 als beispielhafte Darstellung eines Vektors, der ein Fusion-Konstrukt bestehend aus einem Luciferase-Reportergen und dem zu testenden HIGS-Zielgen-Fragment PITG_03410 enthält.

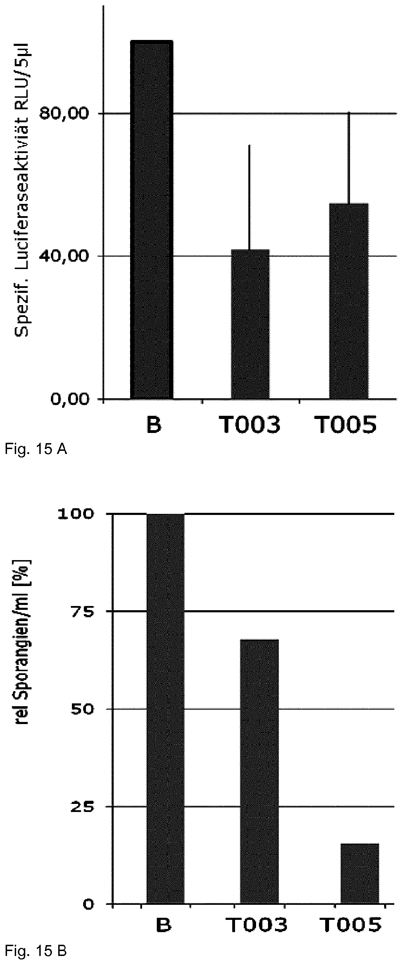

Fig. 15 A: Relative Luciferaseaktivität in transgener Kartoffellinien des Genotyps Baltica mit stabiler Integration des HIGS_RNAi-Konstrukts gegen das Gen PITG_03410 aus *P. infestans* nach Beschuss mit dem Vektor pABM-70Sluci_dsRNA.PITG_03410. B: Baltica (nicht transgene Kontrolle), T003, T005 transgene HIGS-Kartoffellinien.
Fig. 15 B: Relative Sporangienproduktion von *P. infestans* auf transgenen HIGS-Linien. Die Kartoffellinien der Sorte Baltica wurden mit einem RNAi-Konstrukt zur Bildung von dsRNA gegen das *P. infestans-Gen* PITG_03410 transformiert. Diese Linien zeigen nach Infektion mit *P. infestans* im *detached leaf assay* (Einzelblatt-Biotestsystem) eine verminderte Sporangienproduktion im Vergleich zur nicht-transgenen Sorte Baltica (Mittelwert aus 4 biologischen Replikaten). B: Baltica (nicht transgene Kontrolle), T003, T005: transgene HIGS-Kartoffellinien.

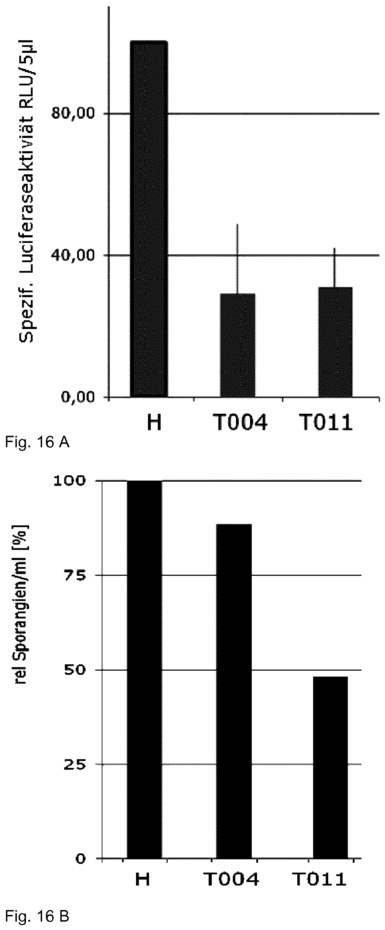

Fig. 16 A: Relative Luciferaseaktivität in transgener Kartoffellinien des Genotyps Hermes mit stabiler Integration des HIGS_RNAi-Konstrukts gegen das Gen PITG_03410 aus *P. infestans* nach Beschuss mit dem Vektor pABM-70Sluci_dsRNA.PITG_03410. H: Hermes (nicht transgene Kontrolle), T004, T011: transgene HIGS-Kartoffellinien.
Fig. 16 B: Relative Sporangienproduktion von *P. infestans* auf transgenen HIGS-Linien. Die Kartoffellinien der Sorte Hermes wurden mit einem RNAi-Konstrukt zur Bildung von dsRNA gegen das *P. infestans-Gen* PITG_03410 transformiert. Diese Linien zeigen nach Infektion mit *P. infestans* im *detached leaf assay* (Einzelblatt-Biotestsystem) eine verminderte Sporangienproduktion im Vergleich zur nicht-transgenen Sorte Baltica (Mittelwert aus 4 biologischen Replikaten). H: Hermes (nicht transgene Kontrolle), T004, T011: transgene HIGS-Kartoffellinien.

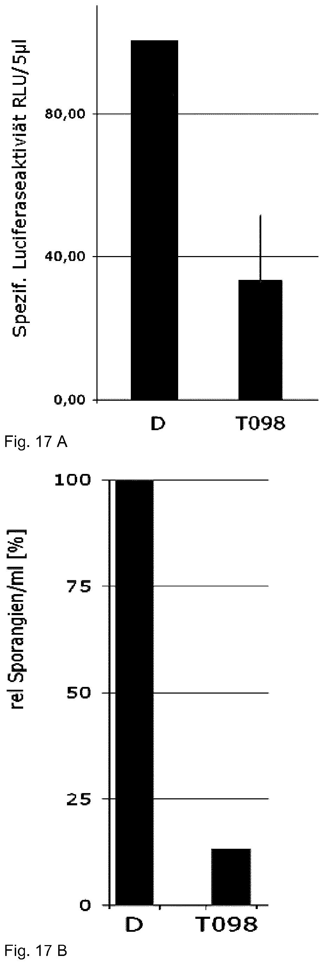

Fig. 17 A: Relative Luciferase-Aktivität in transgener Kartoffellinien des Genotyps Desiree mit stabiler Integration des HIGS_RNAi-Konstrukts gegen das Gen PITG_03410 aus *P. infestans* nach Beschuss mit dem Vektor pABM-70Sluci_dsRNA.PITG_03410. D: Desiree (nicht transgene Kontrolle), T098: transgene HIGS-Kartoffellinie.
Fig. 17 B: Relative Sporangienproduktion von *P. infestans* auf transgenen HIGS-Linien. Die Kartoffellinien der Sorte Desiree wurden mit einem RNAi-Konstrukt zur Bildung von dsRNA gegen das *P. infestans-Gen* PITG_03410 transformiert. Diese Linien zeigen nach Infektion mit *P. infestans* im *detached leaf assay* (Einzelblatt-Biotestsystem) eine verminderte Sporangienproduktion im Vergleich zur nicht-transgenen Sorte Baltica (Mittelwert aus 4 biologischen Replikaten). D: Desiree, (nicht transgene Kontrolle), T098: transgene HIGS-Kartoffellinie.

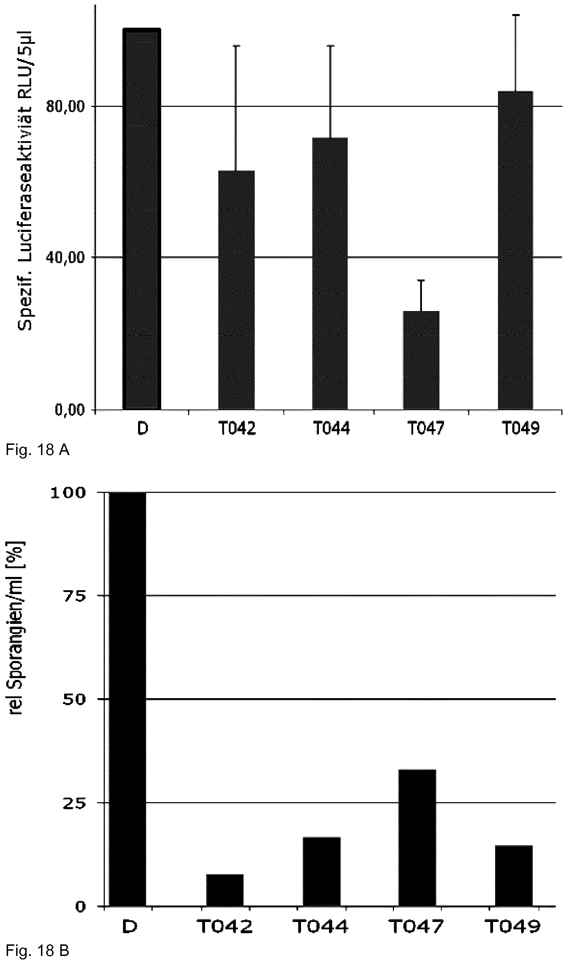

Fig. 18 A: Relative Luciferase-Aktivität in transgener Kartoffellinien des Genotyps Desiree mit stabiler Integration des HIGS_RNAi-Konstrukts gegen das Gen PITG_00375 aus *P. infestans* nach Beschuss mit dem Vektor pABM-70Sluci_dsRNA.PITG_00375. D: Desiree, (nicht transgene Kontrolle), T042, T044 T047, T049: transgene HIGS-Kartoffellinien.
Fig. 18 B: Relative Sporangienproduktion von *P. infestans* auf transgenen HIGS-Linien. Die Kartoffellinien der Sorte Desiree wurden mit einem RNAi-Konstrukt zur Bildung von dsRNA gegen das *P. infestans-Gen* PITG_00375 transformiert. Diese Linien zeigen nach Infektion mit *P. infestans* im *detached leaf assay* (Einzelblatt-Biotestsystem) eine verminderte Sporangienproduktion im Vergleich zur nicht-transgenen Sorte Baltica (Mittelwert aus 4 biologischen Replikaten). D: Desiree, (nicht transgene Kontrolle), T042, T044 T047, T049: transgene HIGS-Kartoffellinien.

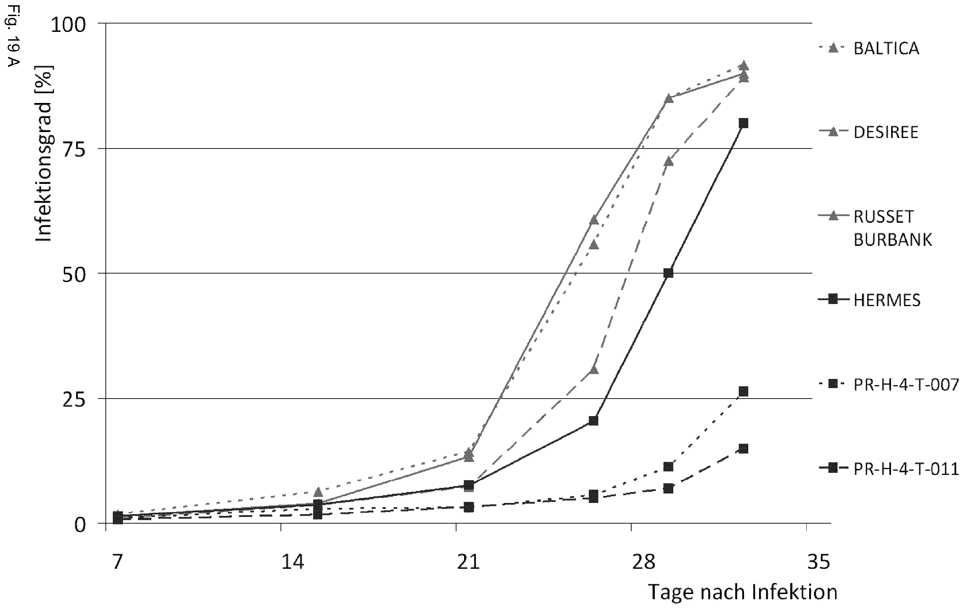

Fig. 19 A: Infektionsgrad in transgener Kartoffellinien des Genotyps Hermes mit stabiler Integration des HIGS_RNAi-Konstrukts gegen das Gen PITG_03410 aus *P. infestans* nach Infektion der Pflanzen unter Freiland-ähnlichen Bedingungen mit *P. infestans.* Graue Linien mit Dreieck: Baltica, Desiree, und Russet Burbank (nicht transgene Kontrollen), Schwarze Linien mit Quadrat: Pflanzen des Genotyps Hermes: durchgezogene Linie: Hermes (nicht transgene Kontrolle), gestrichelte Linie: PR-H-4-7 & gepunktete Linie: PR-H-4-11: transgene HIGS-Kartoffellinien.

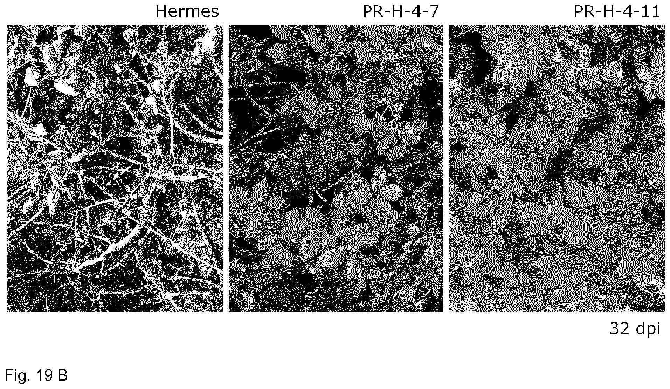

Fig. 19 B: Photographische Dokumentation des Infektionsgrads der transgenen Kartoffellinien PR-H-4-7 und PR-H-4-11 des Genotyps Hermes mit stabiler Integration des HIGS_RNAi-Konstrukts gegen das Gen PITG_03410 aus *P. infestans* nach Infektion der Pflanzen unter Freiland-ähnlichen Bedingungen mit *P. infestans* im Vergleich zur nicht transgenen Kontrolle Hermes. Photographie 32 Tage nach Infektion.

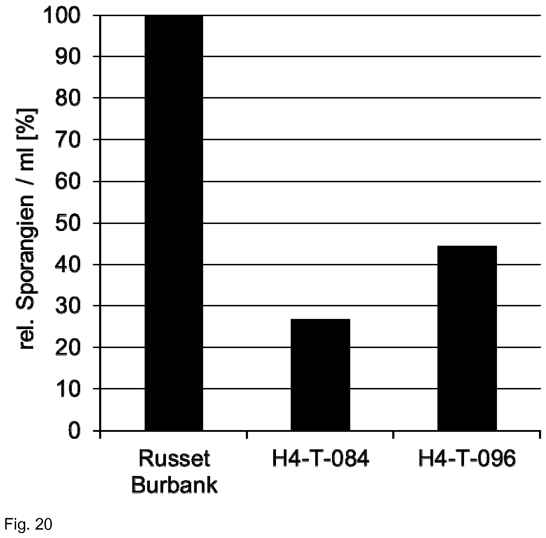

Fig. 20: Relative Sporangienproduktion von *P. infestans* auf transgenen HIGS-Linien. Die Kartoffellinie der Sorte Russet Burbank wurden mit einem RNAi-Konstrukt zur Bildung von dsRNA gegen das *P. infestans-Gen* PITG_03410 transformiert. Diese Linien zeigen nach Infektion mit *P. infestans* im detached leaf assay (Einzelblatt-Biotestsystem) eine verminderte Sporangienproduktion im Vergleich zur nicht-transgenen Sorte Russet Burbank (Mittelwert aus 3 biologischen Replikaten). Russet Burbank (nicht transgene Kontrolle); H-4-T084, H-4-T096: transgene HIGS-Kartoffellinien.

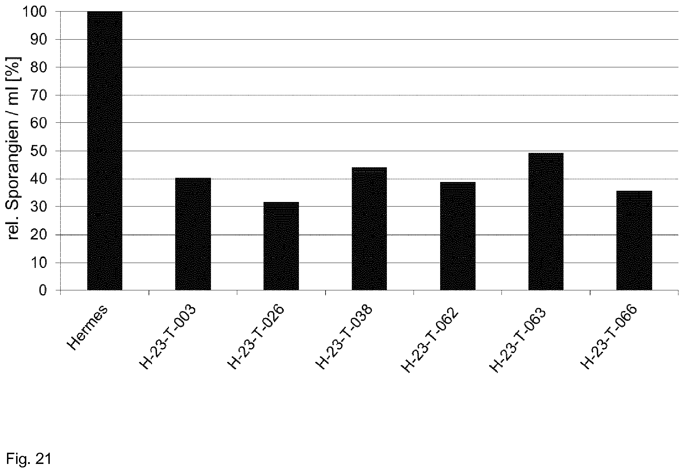

Fig. 21: Relative Sporangienproduktion von *P. infestans* auf transgener HIGS-Linien. Die Kartoffellinien der Sorte Hermes wurden mit einem RNAi-Konstrukt zur Bildung von dsRNA mittels eines Doppel-Promotor-Konstruktes HIGS_dPRNAi_PITG_03410 gegen das *P*. *infestans*-Gen PITG_03410 transformiert. Diese Linien zeigen nach Infektion mit *P. infestans* im detached leaf assay (Einzelblatt-Biotestsystem) eine verminderte Sporangienproduktion im Vergleich zur nicht-transgenen Sorte Hermes (Mittelwert aus 3 biologischen Replikaten). Hermes (nicht transgene Kontrolle); H-23-T0003, H-23-T026, H-23-T038, H-23-T062, H-23-T063, H-23-T066: transgene HIGS-Kartoffellinien.

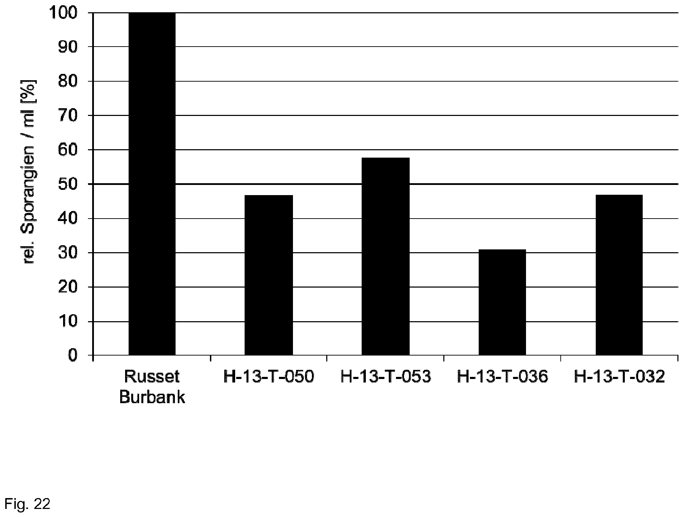

Fig. 22: Relative Sporangienproduktion von *P. infestans* auf transgener HIGS-Linien. Die Kartoffellinien der Sorte Russet Burbank wurden mit einem RNAi-Konstrukt HIGS_CoA zur Bildung von dsRNA gegen die Gene PITG_00146, PITG_08393, PITG_10447 und PITG_00708 aus *P. infestans* transformiert. Diese Linien zeigen nach Infektion mit *P. infestans* im detached leaf assay (Einzelblatt-Biotestsystem) eine verminderte Sporangienproduktion im Vergleich zur nicht-transgenen Sorte Russet Burbank (Mittelwert aus 3 biologischen Replikaten). Russet Burbank (nicht transgene Kontrolle); H-13-T050, H-13-T053, H-13-T036, H-13-T032: transgene HIGS-Kartoffellinien.

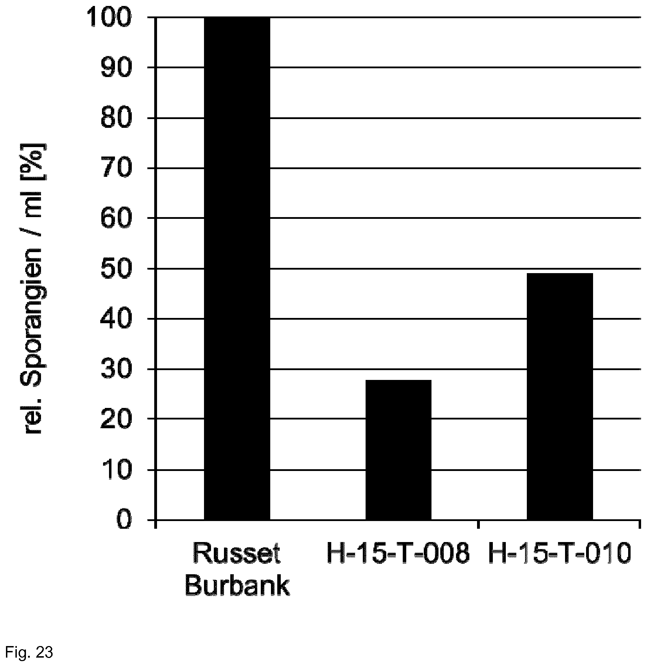

Fig. 23: Relative Sporangienproduktion von *P. infestans* auf transgener HIGS-Linien. Die Kartoffellinien der Sorte Russet Burbank wurden mit einem RNAi-Konstrukt zur Bildung von dsRNA gegen das *P. infestans-Gen* PITG_06748 transformiert. Diese Linien zeigen nach Infektion mit *P. infestans* im detached leaf assay (Einzelblatt-Biotestsystem) eine verminderte Sporangienproduktion im Vergleich zur nicht-transgenen Sorte Russet Burbank (Mittelwert aus 3 biologischen Replikaten). Russet Burbank (nicht transgene Kontrolle); H-15-T008, H-15-T010: transgene HIGS-Kartoffellinien.

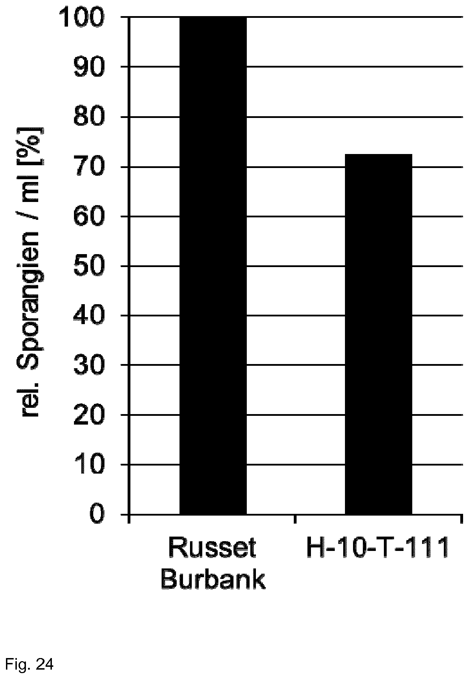

Fig. 24: Relative Sporangienproduktion von *P. infestans* auf transgener HIGS-Linie. Die Kartoffellinie der Sorte Russet Burbank wurden mit einem RNAi-Konstrukt zur Bildung von dsRNA gegen das *P. infestans-Gen* PITG_09306 transformiert. Diese Linie zeigt nach Infektion mit *P. infestans* im detached leaf assay (Einzelblatt-Biotestsystem) eine verminderte Sporangienproduktion im Vergleich zur nicht-transgenen Sorte Russet Burbank (Mittelwert aus 3 biologischen Replikaten). Russet Burbank (nicht transgene Kontrolle); H-10-T111: transgene HIGS-Kartoffellinie.

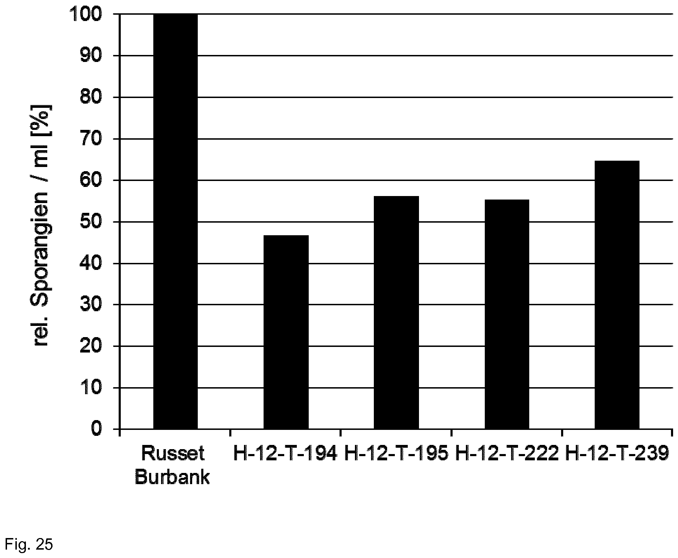

Fig. 25: Relative Sporangienproduktion von *P. infestans* auf transgener HIGS-Linien. Die Kartoffellinien der Sorte Russet Burbank wurden mit einem RNAi-Konstrukt zur Bildung von dsRNA gegen das *P. infestans-Gen* PITG_09193 transformiert. Diese Linien zeigen nach Infektion mit *P. infestans* im detached leaf assay (Einzelblatt-Biotestsystem) eine verminderte Sporangienproduktion im Vergleich zur nicht-transgenen Sorte Russet Burbank (Mittelwert aus 3 biologischen Replikaten). Russet Burbank (nicht transgene Kontrolle); H-12-T194, H-12-T195, H-12-T222, H-12-T239: transgene HIGS-Kartoffellinien.

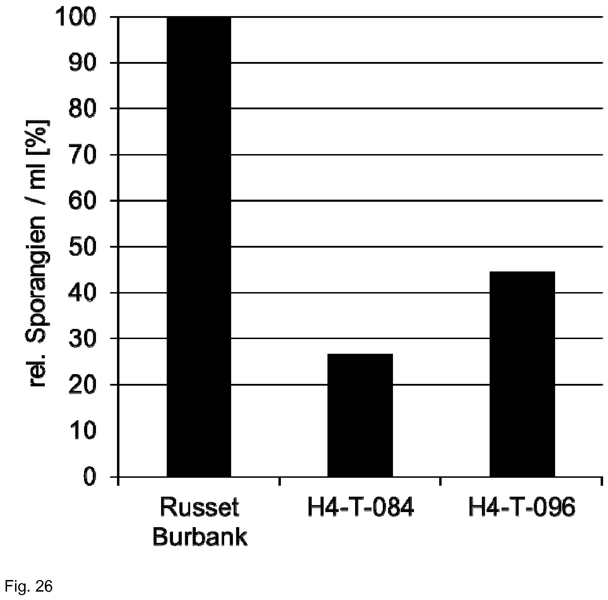

Fig. 26: Relative Sporangienproduktion von *P. infestans* auf transgener HIGS-Linien. Die Kartoffellinien der Sorte Desiree wurden mit einem RNAi-Konstrukt zur Bildung von dsRNA gegen das *P. infestans-Gen* PITG_09193 transformiert. Diese Linien zeigen nach Infektion mit *P. infestans* im detached leaf assay (Einzelblatt-Biotestsystem) eine verminderte Sporangienproduktion im Vergleich zur nicht-transgenen Sorte Desiree (Mittelwert aus 3 biologischen Replikaten). Desiree (nicht transgene Kontrolle); H-12-T187, H-12-T216, H-12-T237, H-12-T245: transgene HIGS-Kartoffellinien.

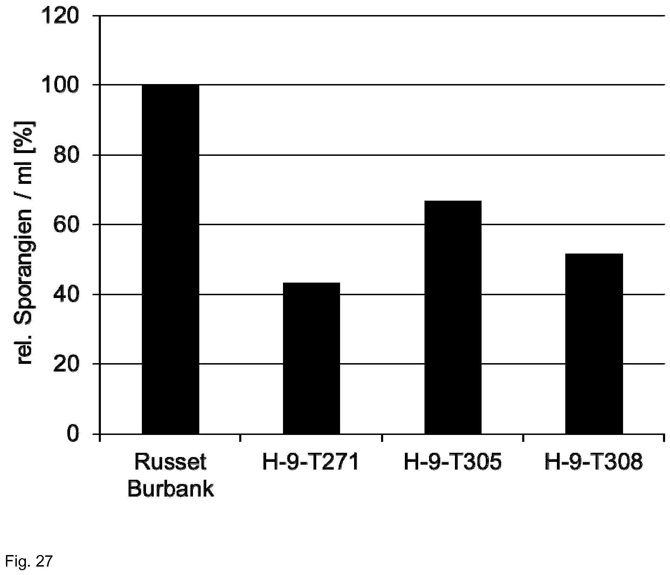

Fig. 27: Relative Sporangienproduktion von *P. infestans* auf transgener HIGS-Linien. Die Kartoffellinien der Sorte Russet Burbank wurden mit einem RNAi-Konstrukt zur Bildung von dsRNA gegen das *P. infestans-Gen* PITG_19177 transformiert. Diese Linien zeigen nach Infektion mit *P. infestans* im detached leaf assay (Einzelblatt-Biotestsystem) eine verminderte Sporangienproduktion im Vergleich zur nicht-transgenen Sorte Russet Burbank (Mittelwert aus 3 biologischen Replikaten). Russet Burbank (nicht transgene Kontrolle); H-9-T271, H-9-T305, H-9-T308: transgene HIGS-Kartoffellinien.

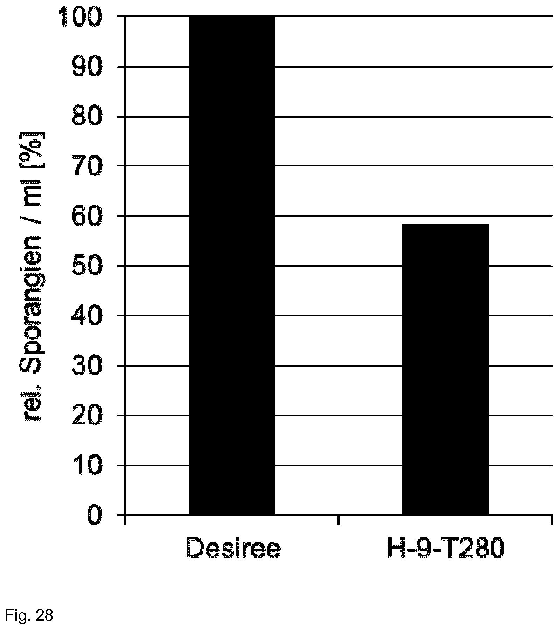

Fig. 28: Relative Sporangienproduktion von *P. infestans* auf transgener HIGS-Linie. Die Kartoffellinie der Sorte Desiree wurden mit einem RNAi-Konstrukt zur Bildung von dsRNA gegen das *P. infestans-Gen* PITG_19177 transformiert. Diese Linie zeigt nach Infektion mit *P*. *infestans* im detached leaf assay (Einzelblatt-Biotestsystem) eine verminderte Sporangienproduktion im Vergleich zur nicht-transgenen Sorte Desiree (Mittelwert aus 3 biologischen Replikaten). Desiree (nicht transgene Kontrolle); H-9-T280: transgene HIGS-Kartoffellinie.

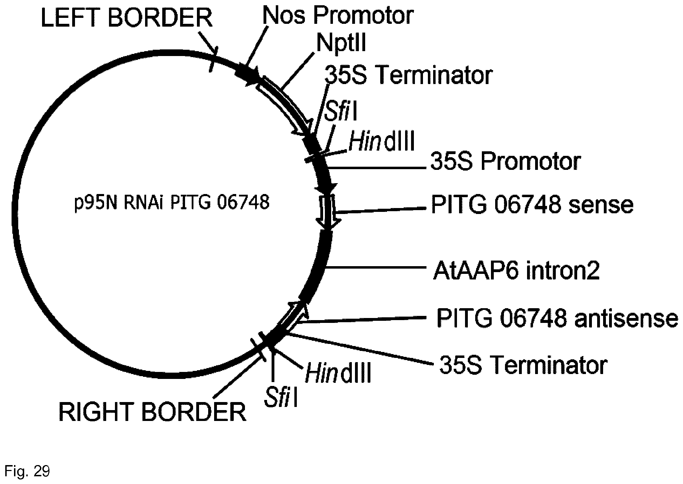

Fig. 29: Plasmid p95N_RNAi PITG_06748 als beispielhafte Darstellung eines Vektors, der ein sense-Intron-antisense-Fragment zur Bildung von dsRNA gegen ein Zielgen (hier PITG_06748) enthält. Dieser Vektor enthält zusätzlich einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator.

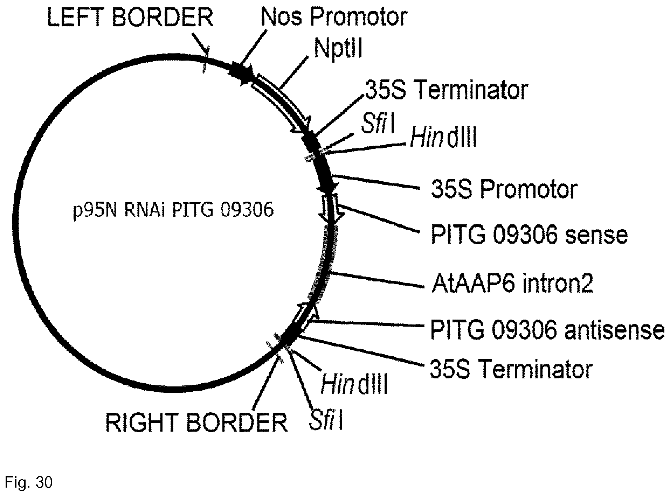

Fig. 30: Plasmid p95N_RNAi_PITG_09306 als beispielhafte Darstellung eines Vektors, der ein sense-Intron-antisense-Fragment zur Bildung von dsRNA gegen ein Zielgen (hier PITG_09306) enthält. Dieser Vektor enthält zusätzlich einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator.

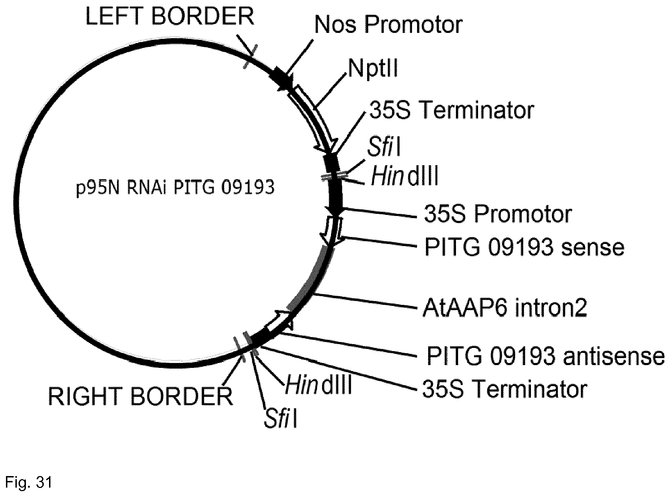

Fig. 31: Plasmid p95N_RNAi_PITG_09193 als beispielhafte Darstellung eines Vektors, der ein sense-Intron-antisense-Fragment zur Bildung von dsRNA gegen ein Zielgen (hier PITG_09193) enthält. Dieser Vektor enthält zusätzlich einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator.

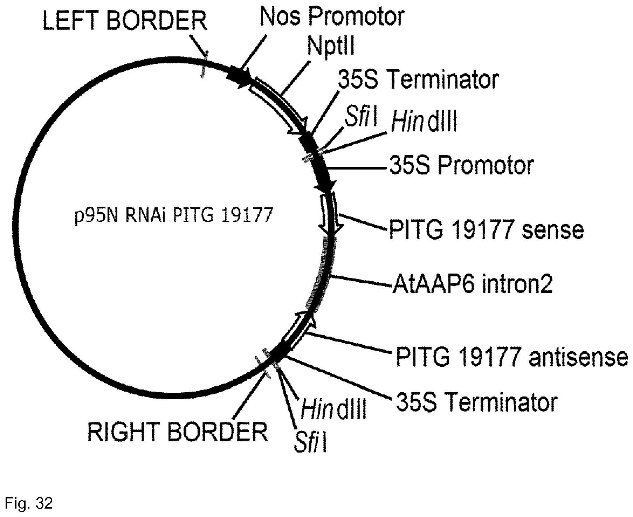

Fig. 32: Plasmid p95N_RNAi_PITG_19177 als beispielhafte Darstellung eines Vektors, der ein sense-Intron-antisense-Fragment zur Bildung von dsRNA gegen ein Zielgen (hier PITG_19177) enthält. Dieser Vektor enthält zusätzlich einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator.

### Ausführungsbeispiele

### Bereitstellung der Konstrukte

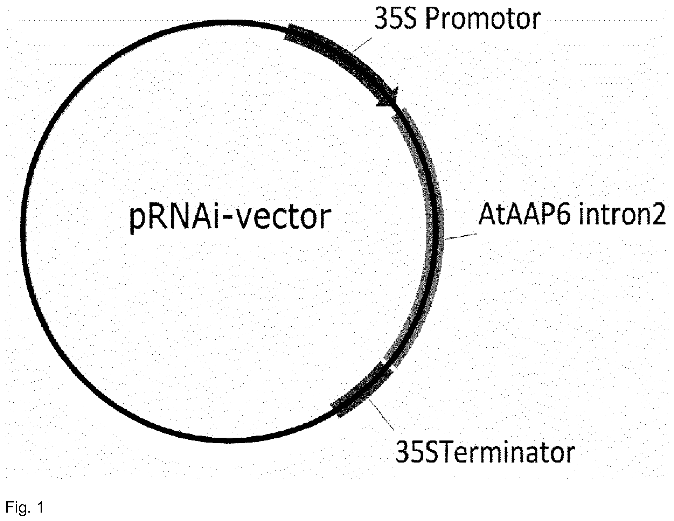

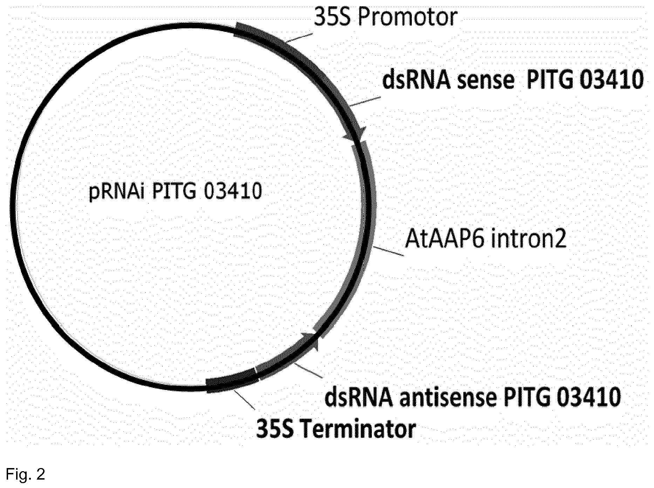

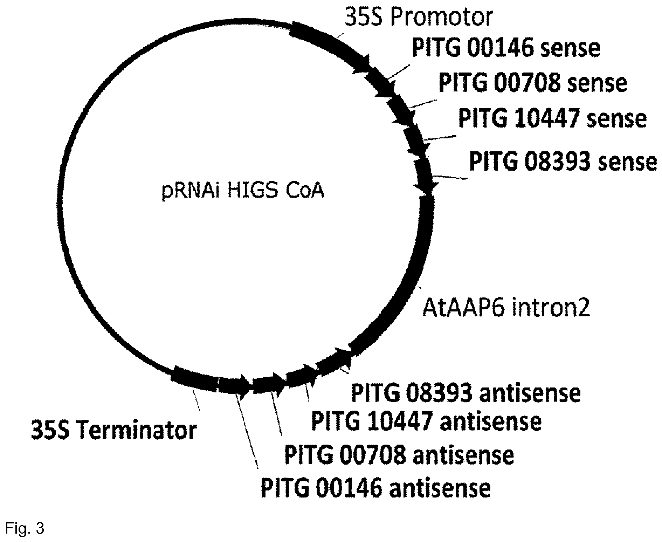

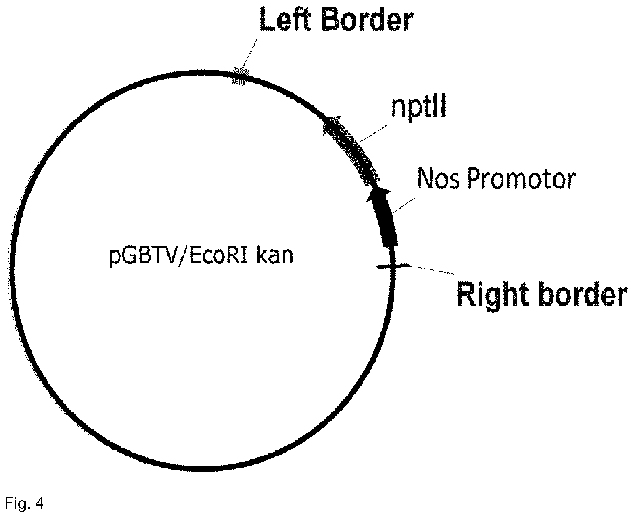

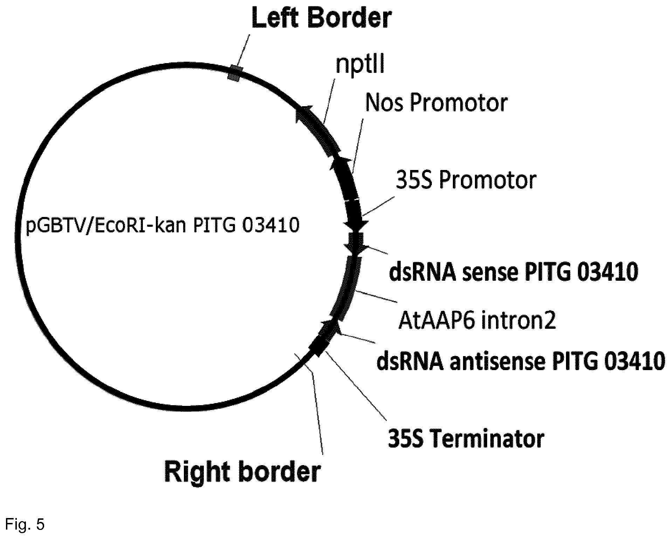

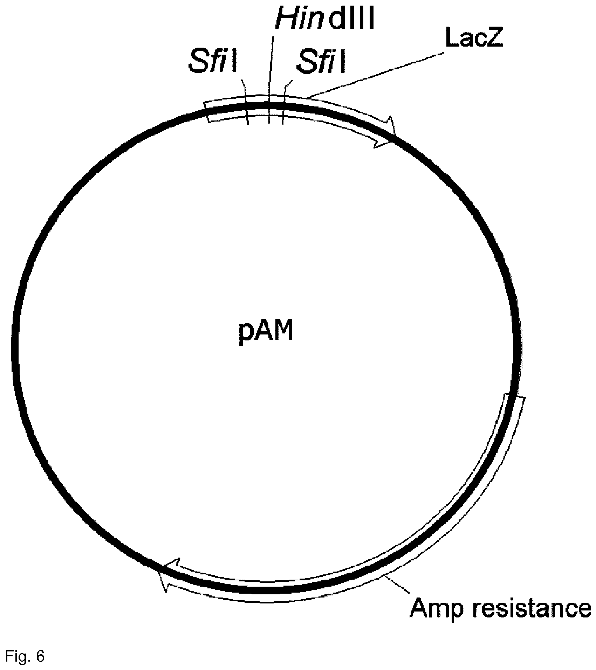

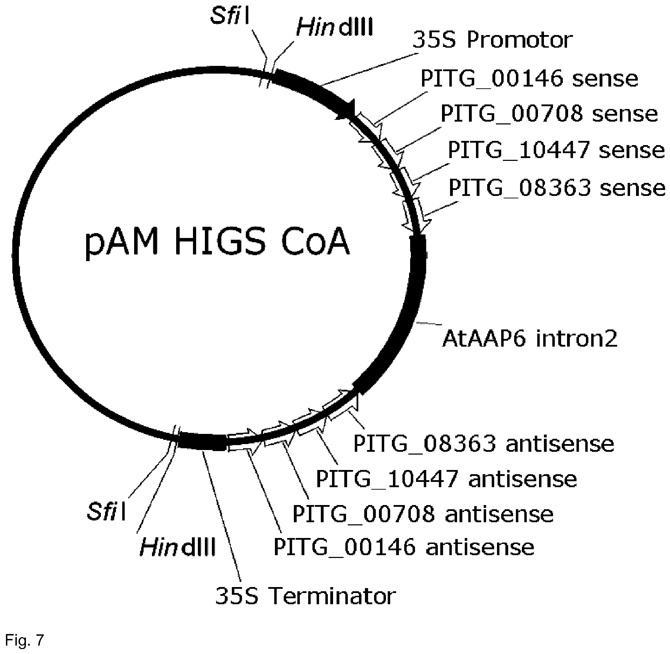

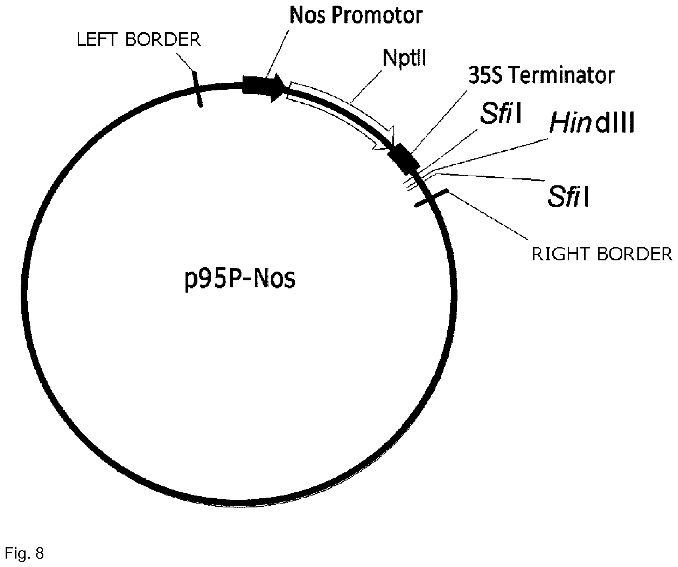

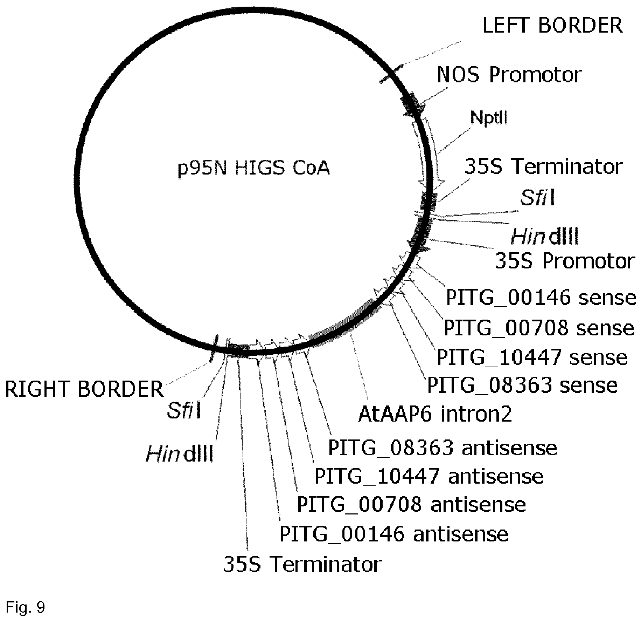

Definierte Sequenzbereiche der ausgewählten Zielgene wurden durch PCR amplifiziert und sowohl in sense- als auch in antisense-Richtung in einen Vektor pRNAi kloniert, der zur Synthese von hairpin-Strukturen geeignet ist (Fig. 2). Dabei können auch mehrere Fragmente mit Sequenzbereichen verschiedener Zielgene in einen Vektor kloniert werden, um ein Kombinations-hairpin-Konstrukt zu generieren (Fig. 3).
Ausgehend von genomischer DNA von *P. infestans* wurde mittels PCR ein Sequenzbereich von 290 bp aus dem kodierenden Bereich des Gens PITG_03410 amplifiziert, über die durch die Primersequenzen angehängten Restriktionsenzymschnittstellen Xhol und Smal ausgeschnitten und in den Vektor pRNAi kloniert (Primer 1: cgctcgaggctggatctcgcgctgaggt, Primer 2: ttgatatcgcggaaggcgagagacatcg). Dieser Vektor enthält einen CaMV 35S-Promotor, eine multiple Klonierungsstelle, ein Intron aus dem Gen AtAAP6, das in *Arabidopsis thaliana* für eine Aminosäurepermease kodiert, eine weitere multiple Klonierungsstelle sowie einen CaMV 35S-Terminator. Dieser wurde mit Xhol und Ecl136II geschnitten und der 4.098 kb große Vektoranteil über Agarosegelelektrophorese aufgetrennt und anschließend isoliert. Der Ligationsansatz wurde in *E. coli* Stamm XL1-blue (Stratagene, LaJolla, CA) transformiert. In das Plasmid pRNAi_PITG_03410_sense wurde das gleiche PITG_03410-Fragment nun in antisense Orientierung kloniert. Dazu wurde das Fragment erneut aus genomischer DNA von *P*. *infestans* wurde mittels PCR amplifiziert, über die durch die Primersequenzen angehängten Restriktionsenzymschnittstellen Xhol und Smal geschnitten und in den mit Smal - Sall geschnittenen und damit folglich linearisierten Vektor pRNAi_PITG_03410_sense ligiert (Fig. 1). Das Genfragment sense-Intron-antisense (RNAi-PITG_03410) wurde aus dem Vektor pRNAigeschnitten und in den Vektor pGBTV/EcoRI_kan kloniert (Fig. 4). Dazu wurden sowohl pGBTV/EcoRI_kan als auch pRNAi_PITG_03410 mit HindIII geschnitten und ligiert, so dass das Plasmid pGBTV/EcoRI_kan_PITG_03410 entsteht (Fig. 5). Alternativ wurden HIGS-RNAi-Konstrukte wie beispielsweise HIGS-CoA zunächst in den Vektor pAM (DNA Cloning Service e.K., Hamburg) kloniert (Fig. 6). Dazu wurden sowohl pAM als auch pRNAi_PITG_03410 mit HindIII geschnitten und ligiert, so dass das Plasmid pAM_HIGS_CoA entsteht (Fig. 7). Aus dem Vektor pAM wurde das HIGS_CoA-Fragment mittels Sfil-Verdau und Ligation in der Vektor p95P-Nos (DNA Cloning Service e.K., Hamburg) integriert (Fig. 8), so dass das Plasmid p95N_HIGS_CoA entsteht (Fig. 9), das zur Kartoffeltransformation genutzt wurde.

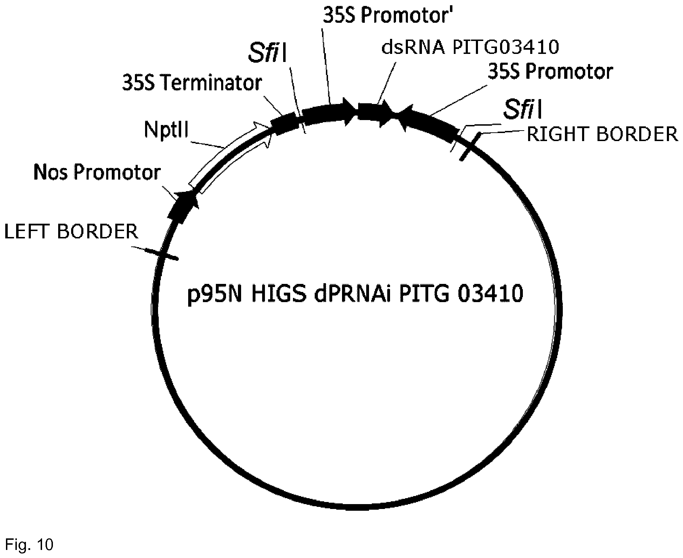

Alternativ zu einem oben beschriebenen Vektor, der zur Synthese von hairpin-Strukturen geeignet ist, kann ein Abschnitt des kodierenden Zielgenbereiches in der Kartoffelpflanze mit Hilfe zweier gegenläufig (invers) orientierter Promotoren zur Expression gebracht werden (Fig. 10). Außerdem kann ein Gen-Silencing auch über artifizielle microRNA-Konstrukte (amiRNA) gemäß dem Protokoll des Web microRNA Designers (WMD3) erzielt werden. Artifizielle miRNAs sind 21mer einzelsträngige RNAs, die synthetisiert werden können, um gewünschte Gene in Pflanzen gezielt negativ zu regulieren. Die Regulation erfolgt - wie bei siRNAs- über mRNASpaltung. Diese RNAi-Konstrukte werden dann in einen binären Vektor kloniert und durch *Agrobacterium tumefaciens* vermittelte Transformation in Kartoffeln transformiert.

### Transformation und Regeneration

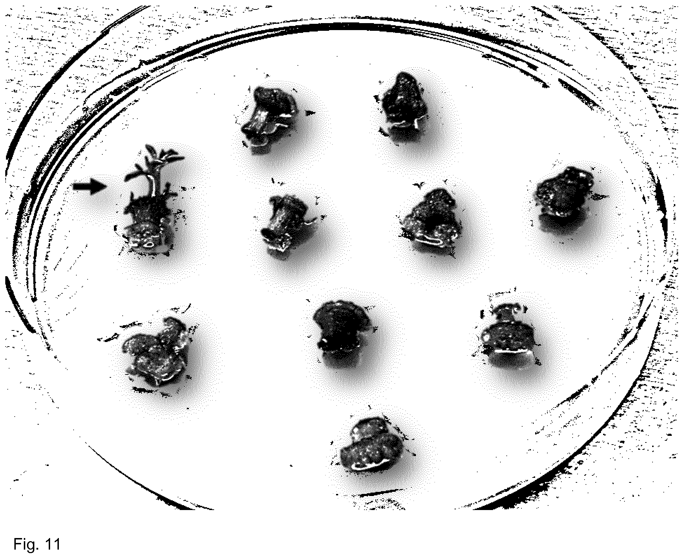

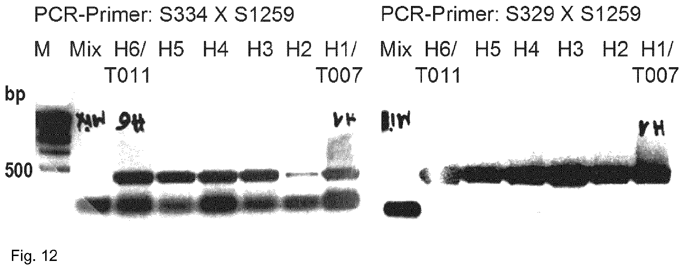

Die Transformation der Kartoffeln erfolgte nach einem modifizierten Protokoll von Pel et al. (2009) unter Verwendung des Antibiotikums Kanamycin. Die Anzucht des Spendermaterials erfolgte in 80 ml MS(D) (25°C; 16 h Tag / 8 h Nacht; 2000 Lux) für 3-4 Wochen. Für die Transformation (C1) wurden aus dem Spendermaterial die Internodien in ca. 0,5 cm große Explantate geschnitten. Diese wurden in Petrischalen mit 10 ml MS(D) (15-20 Explantate/Schale) mit 70 µl einer über Nacht bei 28°C angezogenen *Agrobacterium tumefaciens*-Kultur, die zuvor mit dem in die Pflanzen zu transformierenden HIGS-RNAi Konstrukt als Teil eines binären Vektors wie beispielsweise p95N transformiert wurde, bei 25°C für 2 Tage im Dunkeln inkubiert. Anschließend wurden die Explantate auf Filterpapier getrocknet und in Petrischalen auf MSW-Medium mit Selektionsantibiotikum (400 mg/l Timentin+ 75 Kanamycin mg/l) gesetzt, welche luftdicht abgeklebt und für 2 Wochen (25°C; 16 h Tag / 8 h Nacht; 2000 Lux) kultiviert wurden (C2). Dieser Selektionsschritt wurde im 2-wöchentlichen Rhythmus bis zur Sprossregeneration (ab C3) wiederholt. Regenerierte Sprosse (Fig. 11) wurden zur Bewurzelung auf MS (30 g/l Saccharose) mit Selektionsantibiotikum (250 Timentin mg/l + 100 Kanamycin mg/l) inkubiert und mittels PCR auf die Integration des zu transformierenden Konstruktes getestet und so auf das Vorhandensein der Nukleinsäuren der Erfindung überprüft. Die Verwendung der Primer Bo2299 (5'-GTGGAGAGGCTATTCGGTA-3') und Bo2300 (5'-CCACCATGATATTCGGCAAG-3') führte zu der Amplifikation eines 553 bp großen DNA-Fragments aus dem bakteriellen *NPTII*-Gen, das für die Neomycinphosphotransferase kodiert. Außerdem wurden noch das sense sowie das antisense Fragment mittel PCR nachgewiesen, um die Vollständigkeit des Konstruktes zu gewährleisten (Fig. 12). Die PCR wurde unter Verwendung von 10 ng genomischer DNA, einer Primerkonzentration von 0,2 µM bei einer Annealingtemperatur von 55°C in einem Multicycler PTC-200 (MJ Research, Watertown, USA) durchgeführt. Die Vermehrung der nach PCR positivgetesteten Sprosse erfolgte auf MS + 30 g/l Saccharose + 400 mg/l Ampicillin.

### Detektion der prozessierten doppelsträngigen RNA und siRNAs

In den transformierten Pflanzen werden die exprimierten hairpin- oder doppelsträngigen RNAs über die natürlichen pflanzlichen RNAi-Mechanismen so prozessiert, dass diese RNA-Moleküle zu kleinen einzelsträngigen RNAs abgebaut werden. Diese siRNAs lagern sich an die mRNA des entsprechenden Zielgens im Oomyzeten und bewirken so ein Silencing dieses Gens. Die Pflanze wird somit in die Lage versetzt, sich selbst gegen angreifende Pathogene zu schützen. Durch dieses Konzept können transgene Kartoffelpflanzen erstellt werden, die eine erhöhte Resistenz gegen *P. infestans* aufweisen.

Die Transformation von Kartoffelpflanzen mit Konstrukten zur Expression von hairpin- oder doppelsträngigen RNAs soll dazu führen, dass die daraus resultierenden dsRNA über die natürlichen pflanzlichen RNAi-Mechanismen zu siRNAs prozessiert werden. Um die Fragmentierung der dsRNA zu messen, wurde Gesamt-RNA aus den transgenen Pflanzen mittels der Trizol-Methode (Chomczynski und Sacchi, 1987) isoliert. 15 µg Gesamt-RNA / Probe wurden mit Formamid versetzt, denaturiert und in einem 1% Agarosegel mit 10% Formaldehyd in 1 X MOPS-Puffer (0.2 M MOPS (Natriumsalz), 0.05 M NaOAc, 0.01 M EDTA in DEPC dH₂O. pH 7.0 mit NaOH) elektrophoretisch aufgetrennt. Die aufgetrennte RNA wurde aus dem Gel mittels des Northern Blot-Verfahrens in 20 x SSC-Puffer (Saline-Sodiumcitrat Puffer) auf eine Nylon-Membran (positiv geladen) transferiert. Diese wurde mit einer radioaktiv markierten Sonde, die komplementär zur Sequenz des Zielgenfragments ist, welches in sense bzw. in antisense Richtung in dem in die Pflanzen transformierten Konstrukt vorliegt, hybridisiert. So konnten RNA-Fragmente, die komplementär zur Sequenz des dsRNA-Fragments sind, markiert und mittels eines Phosphoimagers detektiert werden.

Die Transformation von Kartoffelpflanzen mit Konstrukten zur Expression von hairpin- oder doppelsträngigen RNAs soll dazu führen, dass die daraus resultierenden dsRNA über die natürlichen pflanzlichen RNAi-Mechanismen zu siRNAs prozessiert werden. Um die Fragmentierung der dsRNA zu siRNAs zu messen, wurde Gesamt-RNA aus den transgenen Pflanzen mittels der Trizol-Methode (Chomczynski und Sacchi, 1987) isoliert. 15 µg Gesamt-RNA / Probe wurden mit Formamid versetzt, denaturiert und in einem Polyacrylamid-Gel mit 15% Tris/Borsäure/EDTA (TBE) und Harnsäure in 0,5 x TBE elektrophoretisch aufgetrennt. Die aufgetrennte RNA wurde aus dem Gel mittels des Tankblot-Verfahrens in 0,5 x TBE auf eine Nylon-Membran (neutral) transferiert. Diese wurde mit einer radioaktiv markierten Sonde, die komplementär zur Sequenz des Zielgenfragments ist, welches in sense bzw. in antisense Richtung in dem in die Pflanzen transformierten Konstrukt vorliegt, hybridisiert. So konnten siRNAs, die komplementär zu Abschnitten der Sequenz des dsRNA-Fragments sind, markiert und mittels eines Phosphoimagers detektiert werden.
In verschiedenen transgenen Kartoffellinien wie beispielsweise PR-H4-Linien oder PR-H2-Linien konnten solche siRNAs nachgewiesen werden (Fig. 13A, B). Dies zeigt, dass die in die Pflanzen transformierten Konstrukte durch pflanzliche RNAi-Mechanismen so erkannt und prozessiert werden, dass siRNAs gegen HIGS-Zielgene aus *P. infestans* entstehen können, die ein Silencing dieser Gene im Pathogen bewirken sollen.

### Messung der Resistenz in transgenen Kartoffelpflanzen im Einzelblatt-Biotestsvstem

Für den Resistenztest der transgenen Kartoffelblätter wurden die transgenen Pflanzen aus *in vitro* Pflanzen im Gewächshaus in 5 l -Töpfen angezogen. Nach 6-8 Wochen wurden 2 Fieder pro Pflanze abgeschnitten und in einer geschlossenen Plastikbox, so auf eine feuchte Grodanmatten gelegt, dass der Blattstiel im feuchten Grodanmaterial steckt. Eine hohe Luftfeuchtigkeit ist damit gewährleistet. Die Boxen wurden bei 18°C mit Tag/Nacht-Rhythmus (Sonnenlicht, Februar-September) inkubiert. Die Fiederblättchen wurden mit Tropfen einer Zoosporensuspension (10 µl; 10⁴ Zoosporen / ml) von *P. infestans* inokuliert. Nach 24 h wurden die Deckel der Boxen etwas geöffnet, um einen leichte Luftzirkulation in den Boxen zu ermöglichen. Die optische Bonitur sowie die Quantifizierung der Zoosporen erfolgte nach 6 Tagen. Die optische Bonitur bewertet den Infektionsgrad und die Zersetzung des Fiederblattes durch *P. infestans.* Die Auszählung der Sporangien ermöglicht die Quantifizierung der Reproduktionsfähigkeit des Pathogens auf der Pflanze. Dabei wurden die zuvor infizierten Blättchen eines Fieders in 5 ml Wasser in Falconröhrchen auf einem Schüttler für 2 h inkubiert, so dass sich die Sporangien vom Blatt lösen. Die Sporangien wurden dann mit Hilfe der Thoma-Zählkammer unter dem Mikroskop ausgezählt.

In verschiedenen HIGS-Kartoffellinien, die durch Transformationen unterschiedlicher Kartoffelgenotypen entstanden sind, konnte eine reduzierte Sporangienzahl nach Infektion mit *P. infestans* (6 dpi) bestimmt werden (Fig. 15 B -18 B). Dies weist darauf hin, dass die Reproduktionsfähigkeit des Pathogens auf den transgenen Pflanzen beeinträchtigt ist.

Das Gen PITG_03410 wurde als Zielgen für HIGS im genetischen Hintergrund der Kartoffelsorten Baltica, Hermes und Desiree als auch der Sorte Russet Burbank unter Verwendung eines Vektors nach Fig. 5 getestet. Das Einzelblatt-Biotestsystem angewendet auf diese transgenen Pflanzen der Sorte Russet Burbank zeigt, dass die Reproduktionsfähigkeit des Pathogens auf den transgenen Pflanzen im Vergleich zu nicht-transgenen Kontrollpflanzen beeinträchtigt ist (Fig. 20).
Alternativ zu Vektoren, die zur Synthese von hairpin Strukturen geeignet sind, wurde ein Vektor nach Fig. 10 mit dem Zielgen PITG_03410 in Kartoffelpflanzen der Sorte Hermes eingebracht und die transgenen Linien im Einzelblatt-Biotestsystem untersucht (Fig. 21). Auch hier zeigt sich, dass die Reproduktionsfähigkeit von *P. infestans* auf den transgenen Pflanzen im Vergleich zu nicht-transgenen Kontrollpflanzen beeinträchtigt ist.
Es wurden auch Kartoffelpflanzen der Sorte Russet Burbank, die mit einem Kombinationsvektor gegen die Gene PITG_00146, PITG_00708, PITG_10447 und PITG_08363 nach Fig. 3 transformiert wurden, im Einzelblatt-Biotestsystem getestet. Es zeigt sich, dass die Reproduktionsfähigkeit von *P. infestans* auf diesen transgenen Pflanzen im Vergleich zu nicht-transgenen Kontrollpflanzen beeinträchtigt ist (Fig. 22).
Ebenso zeigt sich im Einzelblatt-Biotestsystem, dass die Reproduktionsfähigkeit von *P*. *infestans* auf transgenen Pflanzen der Sorte Russet Burbank beeinträchtigt ist, die mit einem Vektor nach Fig. 29, nach Fig. 30, nach Fig. 31 oder nach Fig. 32 transformiert wurden, der gegen die Gene PITG_06748 (Fig. 23), PITG_09306 (Fig. 24), PITG_09193 (Fig. 25) bzw. PITG_19177 (Fig. 27) gerichtet ist.
Auch auf transgenen Pflanzen der Sorte Desiree, welche mit einem Vektor nach Fig. 31 oder nach Fig. 32 transformiert wurden, der gegen das Gen PITG_09193 (Fig. 25) bzw. PITG_19177 (Fig. 27) gerichtet ist, zeigte sich im Einzelblatt-Biotestsystem, dass die Reproduktionsfähigkeit von *P. infestans* im Vergleich zu nicht-transgenen Kontrollpflanzen beeinträchtigt ist.

### Transientes Testsystem für die RNAi-Vektoren in Kartoffelblättern

Es wurde ein transientes Testsystem nach Birch et al. (2010) entwickelt, um die Funktionalität der RNAi-Vektoren gegen ausgewählte Zielgensequenzen von *P. Infestans* zu überprüfen. Mittels Co-Bombardement wurden ein auf ein Zielgen abzielender RNAi-Vektor zusammen mit einem Fusion-Konstrukt bestehend aus einem Luciferase-Reportergen und dem zu testenden Zielgen-Fragment transient in Kartoffelblättern exprimiert. Wird das im RNAi-Vektor kodierte dsRNA-Konstrukt prozessiert, sollte die Bildung von dsRNA und daraus resultierend die Bildung von siRNAs gewährleistet sein. Diese siRNAs sollen nicht nur den Abbau des Zielgen-Fragment-Transkripts sondern auch des daran fusionierten Reportergen-Transkripts bewirken, so dass bei einem funktionalen RNAi-Konstrukt eine Reduktion der Luciferaseaktivität beobachtet werden kann. Das Plasmid pABM-70Sluci umfasst einen doppelten CaMV 35S-Promotor, eine multiple Klonierungsstelle, die kodierende Sequenz des Gens *luc* aus *Photinus pyralis,* das für eine Luciferase kodiert, getrennt von einem modifizierten Intron PIV2 aus dem Kartoffel-Gen *St-LS1* (Eckes et al. 1986, Vancanneyt et al. 1990), eine weitere multiple Klonierungsstelle sowie einen Nos-Terminator des Nopalin Synthase-Gens aus Agrobacterium tumefaciens. In dieses Plasmid pABM-70Sluci wurde das mittels PCR amplifizierte Fragment des kodierenden Sequenzbereiches beispielsweise des PITG_03410-Gens kloniert, das auch zur Herstellung des dsRNA-Konstruktes in den pRNAi-Vektor kloniert wurde (Fig. 14 A) Dieses transiente Testsystem kann nicht nur zur Validierung der generellen Funktionalität der RNAi-Konstrukte dienen, sondern außerdem genutzt werden, um verschiedene Sequenzbereiche eines Genes auf deren Silencing-Effekt zu untersuchen und schließlich die besten Sequenzbereiche eines Gens für ein optimales Silencing auszuwählen. Außerdem kann der Beschuss transgener stabil mit einem RNAi-Konstrukt transformierter Pflanzen dazu dienen, die Silencing-Effizienz der einzelnen transgenen HIGS-Kartoffellinien zu bestimmen, die sich beispielsweise abhängig vom Integrationsort des Konstruktes stark unterscheiden kann. In verschiedenen transgenen HIGS-Kartoffellinien, die durch Transformationen in unterschiedliche Kartoffelgenotypen entstanden sind, und die eine reduzierte Sporangienzahl nach Infektion mit *P. infestans* zeigten, konnte auch eine Reduktion der Luciferaseaktivität gemessen werden (Fig. 15 A - 18 A). Dies zeigt die Funktionalität der zu siRNAs prozessierten HIGS-Konstrukte in Bezug auf ein Silencing der Zielgensequenz in den transgenen Pflanzen.
Wenn die kodierenden Gensequencen, die von einem Kombinationskonstrukt wie beispielsweise pRNAi_HIGS-CoA gesilenced werden sollen (Zielgene: PITG_08393, PITG_00146, PITG_10447, PITG_00708), jeweils in den Vektor pABM_70Sluci hinter die kodierende Sequenz des Gens *luc* aus *Photinus pyralis* kloniert werden (pABM_70Sluci_PITG_08393, pABM_70Sluci_PITG_00146, pABM_70Sluci_PITG_10447, pABM_70Sluci_PITG_00708) und zusammen mit dem Vektor pRNAi_HIGS_CoA in Kartoffelblättern transient exprimiert werden, kann die Silencing-Effizienz des Kombinationskonstrukts auf die verschiedenen Zielgene analysiert werden. Dies ist auch möglich durch den Beschuss transgener stabil mit dem RNAi-Kombinationskonstrukt transformierter Pflanzen mit den einzelnen Fusion-Konstrukten bestehend aus dem Luciferase-Reportergen und den zu testenden kodierenden Sequenzen der verschiedenen Zielgene. Die Luciferase-Aktivitätsbestimmungen wurden mit Hilfe des Dual Luciferase® Reporter Assays (Promega, Mannheim) durchgeführt (Schmidt et al. 2004).

### Messung der Resistenz in transgenen Kartoffelpflanzen unter Freilandbedinaunaen

Für den Resistenztest der transgenen Kartoffelpflanzen unter Freilandbedingungen wurden die transgenen Pflanzen im Frühjahr (März) zunächst aus *in vitro* Pflanzen im Gewächshaus für 3 Wochen in Multitopfplatten angezogen. Danach wurden diese Pflanzen in ein Gewächshaus mit Maschendrahtdach in gewachsenem Boden ausgepflanzt, so dass die Pflanzen Umweltbedingungen beeinflusst beispielsweise durch Temperatur, Sonneneinstrahlung, Niederschlag und Luftfeuchtigkeit, welche mit natürlichen Feldbedingungen vergleichbar sind, ausgesetzt waren. Die Pflanzen wurden in je 3 Parzellen mit jeweils 6 Pflanzen ausgepflanzt. Nach 8 Wochen wurde jeweils ein Fieder von 2 Pflanzen einer Parzelle mittels Sprühinokulation (750 µl; 10⁴ Zoosporen / ml) von *P. infestans* inokuliert. Über diese Fieder wurden Plastiktüten gestülpt, um eine hohe Luftfeuchtigkeit zu gewährleisten, die die Infektion begünstigt. Nach zwei Tagen wurden diese Tüten entfernt. Die Ausbreitung der Krautfäule durch *P. infestans* im Gewächshaus wurde wöchentlich optisch bonitiert und photographisch dokumentiert. Die Kriterien zur Bonitur der Infektion beziehen sich zunächst nur auf das infizierte Fiederblatt (0: keine Infektion, 1: leichte Infektion (1/2 Anzahl der infizierten Fiederblättchen befallen), 2: Infektion auf mehr als 1/2 Blättchen des Fieders, 3: Infektion auf allen Blättchen des Fieders) und bewerten dann die Ausbreitung der Infektion auf der Pflanze und in der gesamten Parzelle (4: Infektion breitet sich auch auf einige andere Blätter der Pflanze aus, 6: Infektion breitet sich auch auf andere Pflanzen aus, 8: Infektion breitet sich auch auf andere Pflanzen stark aus, 10: 10% der Pflanze infiziert / zerstört, 20: 20% der Pflanzen infiziert / zerstört, 100: 100% der Pflanze infiziert / zerstört).
In verschiedenen transgenen HIGS-Kartoffellinien (PR-H-4-7, PR-H-4-11), die durch Transformationen in den Kartoffelgenotypen Hermes entstanden sind, konnte eine stark reduzierter Infektionsgrad dieser Pflanzen im Verlauf der Infektion mit *P. infestans* im Vergleich zum Transformationsgenotyp Hermes, der als Kontrolle genau wie die transgenen Pflanzen angezogen, ausgepflanzt und infiziert worden war, bestimmt werden. Der reduzierte Infektionsgrad spiegelte sich zunächst durch eine stark verringerte Infektionsfähigkeit des Pathogens auf den inokulierten Fiedern (Boniturnoten 21 Tage nach Infektion: PR-H-4-7: 3,3; PR-H-4-11: 3,2; Hermes: 7,6) und zu späteren Zeitpunkten durch eine deutlich verringerte Ausbreitungsfähigkeit des Pathogens auf diesen Pflanzen wider (Boniturnoten 32 Tage nach Infektion: PR-H-4-7: 26; PR-H-4-11: 15; Hermes: 80) (Fig. 19 A, B).

Durch die beschriebenen Versuche konnte nicht nur die Prozessierung der HIGS-Konstrukte in transgenen Kartoffelpflanzen zu siRNAs, sondern auch die Funktionalität dieser Konstrukte in Bezug auf das Silencing der Zielgensequenz in diesen transgenen Pflanzen sowie eine erhöhte Resistenz dieser Pflanzen gegenüber *P. infestans* quantifizierbar durch eine reduzierte Sporangienproduktion des Pathogens auf diesen Wirtspflanzen demonstriert werden. Da die Identifizierung funktionaler HIGS-Zielgene nicht ohne sorgfältige Überprüfung ihrer Funktionalität möglich ist, eignen sich diese hier beschriebenen ausführlichen Analysen besonders gut, um für das HIGS-Konzept wirksame Gene zu definieren und resistente HIGS-Pflanzen zu generieren.

Von der Erfindung eingeschlossen sind:
[1] Transgene Pflanze der Art *Solanum tuberosum* oder deren Teile, in deren Genom eine doppelsträngige erste DNA und eine doppelsträngige zweite DNA zur Ausbildung einer Pathogenresistenz gegenüber einem Oomyzeten der Gattung *Phytophthora* stabil integriert sind, wobei die Nukleotidsequenzen des kodierenden Stranges der ersten und zweiten DNA zueinander ganz oder teilweise invers komplementär sind, so dass ausgehend hiervon eine doppelsträngige RNA gebildet werden kann, dadurch gekennzeichnet, dass die erste DNA
   (a) eine Nukleotidsequenz gemäß den SEQ ID NOS: 1 - 43, oder
   (b) ein Fragment von mindestens 15 aufeinanderfolgenden Nukleotiden einer Nukleotidsequenz gemäß den SEQ ID NOS: 1 - 43, oder
   (c) eine Nukleotidsequenz, die komplementär zu einer der Nukleotidsequenzen nach (a) oder (b) ist, oder
   (d) eine Nukleotidsequenz, die mit einer der Nukleotidsequenzen nach (a), (b) oder (c) unter stringenten Bedingungen hybridisiert,
   umfasst.
[2] Transgene Pflanze nach [1] dadurch gekennzeichnet, dass die Pflanze eine Resistenz gegenüber *Phytophthora infestans* aufweist.
[3] Transgene Pflanze nach [1], dadurch gekennzeichnet, dass es sich bei der doppelsträngigen RNA um miRNA oder siRNA handelt.
[4] Transgene Pflanze nach [1], dadurch gekennzeichnet, dass die erste DNA und die zweite DNA mit mindestens einem Promotor operativ verknüpft sind.
[5] Teile einer Pflanze nach [1], dadurch gekennzeichnet, dass es sich um Samen oder Zellen handelt.
[6] Verfahren zur Herstellung einer transgenen Pflanze der Art *Solanum tuberosum,* die eine Resistenz gegenüber einem Oomyzeten der Gattung *Phytophthora* aufweist, umfassend die Schritte:
   (i) Herstellen einer transformierten ersten Elternpflanze enthaltend eine erste doppelsträngige DNA, die stabil in das Genom der Elternpflanze integriert ist und die
      (a) eine Nukleotidseauenz gemäß den SEQ ID NOS: 1 - 43, oder
      (b) ein Fragment von mindestens 15 aufeinanderfolgenden Nukleotiden einer Nukleotidseauenz gemäß den SEQ ID NOS: 1 - 43, oder
      (c) eine Nukleotidsequenz, die komplementär zu einer der Nukleotidseauenzen nach (a) oder (b) ist, oder
      (d) eine Nukleotidsequenz, die mit einer der Nukleotidseauenzen nach (a), (b) oder (c) unter stringenten Bedingungen hybridisiert, umfasst:
   (ii) Herstellen einer transformierten zweiten Elternpflanze enthaltend eine zweite doppelsträngige DNA, die stabil in das Genom der Elternpflanze integriert ist, wobei die Nukleotidseauenzen des kodierenden Stranges der ersten und zweiten DNA zueinander ganz oder teilweise invers komplementär sind;
   (iii) Kreuzen der ersten Elternpflanze mit der zweiten Elternpflanze;
   (iv) Auswählen einer solchen Pflanze, in deren Genom die erste DNA und die zweite DNA zur Ausbildung einer Pathogenresistenz gegenüber einem Oomyzeten der Gattung *Phytophthora* integriert sind, so dass ausgehend hiervon eine doppelsträngige RNA gebildet werden kann.
[7] Verfahren nach [6] dadurch gekennzeichnet, dass es sich bei der Resistenz um eine Resistenz gegenüber *Phytophthora infestans* handelt.
[8] Verfahren nach [6], dadurch gekennzeichnet, dass es sich bei der doppelsträngigen RNA um miRNA oder siRNA handelt.
[9] Mittel zur äußeren Applikation bei Pflanzen enthaltend doppelsträngige RNA, wobei ein Strang dieser RNA dem Transkript einer doppelsträngigen DNA umfassend
   (a) eine Nukleotidseauenz gemäß den SEQ ID NOS: 1 - 43, oder
   (b) ein Fragment von mindestens 15 aufeinanderfolgenden Nukleotiden einer Nukleotidseauenz gemäß den SEQ ID NOS: 1 - 43, oder
   (c) eine Nukleotidsequenz, die komplementär zu einer der Nukleotidsequenzen nach (a) oder (b) ist, oder
   (d) eine Nukleotidsequenz, die mit einer der Nukleotidsequenzen nach (a), (b) oder (c) unter stringenten Bedingungen hybridisiert,
   entspricht.
[10] Mittel nach [9] zur Erzeugung einer Resistenz gegenüber einem Oomyzeten der Gattung *Phytophthora,* insbesondere gegenüber *Phytophthora infestans.*

### Referenzen

Avrova AO, Boevink PC, Young V, Grenville-Briggs LJ, van West P, Birch PR, Whisson SC (2008) A novel Phytophthora infestans haustorium-specific membrane protein is required for infection of potato. Cell Microbiol. 10(11):2271-84.
Birch RG, Shen B, Sawyer BJ, Huttner E, Tucker WQ, Betzner AS (2010) Evaluation and application of a luciferase fusion system for rapid in vivo analysis of RNAi targets and constructs in plants. Plant Biotechnol J. May 1;8(4):465-75. Epub 2010 Jan 19
Blackman LM, Arikawa M, Yamada S, Suzaki T, Hardham AR (2011) Identification of a mastigoneme protein from Phytophthora nicotianae. Protist. 162(1):100-14.
Benfey, P. N., Ren, L., and Chua, N.-H. (1990). Combinatorial and synergistic properties of CaMV 35S enhancer subdomains. EMBO J. (9), 1685-1696.
Chomczynski P, Sacchi N. (1987) Single-step method of RNA isolation by acid guanidinium thiocyanate-phenol-chloroform extraction. Anal Biochem.162(1):156-9
Eckes P., Rosahl S., Schell J., Willmitzer L. (1986) Isolation and characterization of a lightinducible, organ-specific gene from potato and analysis of its expression after tagging and transfer into tobacco and potato shoots. Molecular and General Genetics 205 (1) 14-22, DOI: 10.1007/BF02428027
Fire A, Xu S, Montgomery MK, Kostas SA, Driver SE, Mello CC. (1998) Potent and specific genetic interference by double-stranded RNA in Caenorhabditis elegans. Nature Feb 19;391(6669):806-11
Grenville-Briggs LJ, Avrova AO, Bruce CR, Williams A, Whisson SC, Birch PR, van West P (2005) Elevated amino acid biosynthesis in Phytophthora infestans during appressorium formation and potato infection. Fungal Genet Biol. 42(3):244-56.
Inoue SB, Takewaki N, Takasuka T, Mio T, Adachi M, Fujii Y, Miyamoto C, Arisawa M, Furuichi Y, Watanabe T (1995) Characterization and gene cloning of 1,3-beta-D-glucan synthase from Saccharomyces cerevisiae. Eur J Biochem 231(3):845-54.
Judelson HS, Narayan RD, Ah-Fong AM, Kim KS (2009a) Gene expression changes during asexual sporulation by the late blight agent Phytophthora infestans occur in discrete temporal stages. Mol Genet Genomics. 281(2):193-206.
Judelson HS, Tani S, Narayan RD (2009b) Metabolic adaptation of Phytophthora infestans during growth on leaves, tubers and artificial media. Mol Plant Pathol. 10(6):843-55.
Kamoun S, van West P, Vleeshouwers VG, de Groot KE, Govers F. (1998) . Resistance of nicotiana benthamiana to phytophthora infestans is mediated by the recognition of the elicitor protein INF1. Plant Cell. Sep;10(9):1413-26.
Lesage G, Sdicu AM, Menard P, Shapiro J, Hussein S, Bussey H (2004) Analysis of beta-1,3-glucan assembly in Saccharomyces cerevisiae using a synthetic interaction network and altered sensitivity to caspofungin. Genetics. May; 167(1):35-49
Li A, Wang Y, Tao K, Dong S, Huang Q, Dai T, Zheng X, Wang Y (2010) PsSAK1, a Stress-Activated MAP Kinase of Phytophthora sojae, Is Required for Zoospore Viability and Infection of Soybean. Mol Plant Microbe Interact. 23(8):1022-31.
Mazur P, Morin N, Baginsky W, el-Sherbeini M, Clemas JA, Nielsen JB, Foor F (1995) Differential expression and function of two homologous subunits of yeast 1,3-beta-D-glucan synthase. Mol Cell Biol. 15(10):5671-81.
Pel MA, Foster SJ, Park TH, Rietman H, van Arkel G, Jones JDG, Van Eck HJ, Jacobsen E, Visser RGF, Van der Vossen EAG (2009) Mapping and cloning of late blight resistance genes from Solanum venturii using an interspecific candidate gene approach. MPMI 22:601-615
Roemer T, Paravicini G, Payton MA, Bussey H (1994) Characterization of the yeast (1-->6)-beta-glucan biosynthetic components, Kre6p and Skn1p, and genetic interactions between the PKC1 pathway and extracellular matrix assembly. J Cell Biol. 127(2):567-79.
Saito, K., Yamazaki, M., Kaneko, H., Murakoshi, I., Fukuda, Y., and van Montagu, M. (1991). Tissue-specific and stress-enhancing expression of the TR promoter for mannopine synthase in transgenic medicinal plants. Planta 184, 40-46.
Schmidt K., Heberle B., Kurrasch J., Nehls R., Stahl D.J. (2004) Suppression of phenylalanine ammonia lyase expression in sugar beet by the fungal pathogen Cercospora beticola is mediated at the core promoter of the gene. Plant Mol. Biol., 55: 835-852.
Stahl D. J., Kloos, D. U., and Hehl, R. (2004). A sugar beet chlorophyll a/b binding protein void of G-box like elements confer strong and leaf specific reporter gene expression in transgenic sugar beet. BMC Biotechnology 4;31: 12
Vancanneyt G., Schmidt R., O'Connor-Sanchez A., Willmitzer L., Rocha-Sosa M. (1990) Construction of an intron-containing marker gene: splicing of the intron in transgenic plants and its use in monitoring early events in Agrobacterium-mediated plant transformation. Mol Gen Genet. 220(2):245-50
Van West P, Kamoun S, van 't Klooster JW, Govers F (1999) Internuclear gene silencing in Phytophthora infestans. Mol Cell. Mar;3(3):339-48.
Wang Y, Dou D, Wang X, Li A, Sheng Y, Hua C, Cheng B, Chen X, Zheng X, Wang Y (2009) The PsCZF1 gene encoding a C2H2 zinc finger protein is required for growth, development and pathogenesis in Phytophthora sojae. Microb Pathog. 47(2):78-86.
Wang Y, Li A, Wang X, Zhang X, Zhao W, Dou D, Zheng X, Wang Y (2010) GPR11, a putative seven-transmembrane G protein-coupled receptor, controls zoospore development and virulence of Phytophthora sojae. Eukaryot Cell 9(2):242-50.
Yin C, Jurgenson J E, Hulbert S H (2011) Development of a Host-Induced RNAi System in the Wheat Stripe Rust Fungus Puccinia striiformis f. sp. Tritici. MPMI 24(5): 554-561. doi:10.1094/MPMI -10-10-0229. © 2011 The American Phytopathological Society
Zhang M, Wang Q, Xu K, Meng Y, Quan J, et al. (2011) Production of dsRNA Sequences in the Host Plant Is Not Sufficient to Initiate Gene Silencing in theColonizing Oomyzete Pathogen Phytophthora parasitica. PLoS ONE 6(11): e28114.
EP 1716238 (Bayer S.A.S.) METHOD FOR MODIFYING GENE EXPRESSION OF A PHYTOPATHOGENIC FUNGUS
WO 2006/070227 (Devgen N.V.) METHOD FOR DOWN-REGULATING GENE EXPRESSION IN FUNGI
WO 2009/112270 (Leibniz-Institut für Pflanzengenetik und Kulturpflanzenforschung) METHOD FOR CREATING BROAD-SPECTRUM RESISTANCE TO FUNGI IN TRANSGENIC PLANTS
US 2010/0257634 (Venganza Inc.) BIOASSAY FOR GENE SILENCING CONSTRUCTS
WO 2006/047495 (Venganza Inc.) METHODS AND MATERIALS FOR CONFERRING RESISTANCE TO PESTS AND PATHOGENS OF PLANTS

## Patentansprüche

1. Transgene Pflanze der Art *Solanum tuberosum* oder deren Teile, in deren Genom eine doppelsträngige erste DNA und eine doppelsträngige zweite DNA zur Ausbildung einer Pathogenresistenz gegenüber einem Oomyzeten der Gattung *Phytophthora* stabil integriert sind, wobei die Nukleotidsequenzen des kodierenden Stranges der ersten und zweiten DNA zueinander ganz oder teilweise invers komplementär sind, so dass ausgehend hiervon eine doppelsträngige RNA gebildet werden kann, **dadurch gekennzeichnet, dass** die erste DNA
(a) eine Nukleotidsequenz gemäß der SEQ ID NO: 38, oder
(b) ein Fragment von mindestens 15 aufeinanderfolgenden Nukleotiden einer Nukleotidsequenz gemäß der SEQ ID NO: 38, oder
(c) eine Nukleotidsequenz, die komplementär zu einer der Nukleotidsequenzen nach (a) oder (b) ist, oder
(d) eine Nukleotidsequenz, die mit einer der Nukleotidsequenzen nach (a), (b) oder (c) unter stringenten Bedingungen hybridisiert,
umfasst.

2. Transgene Pflanze nach Anspruch 1 **dadurch gekennzeichnet, dass** die Pflanze eine Resistenz gegenüber *Phytophthora infestans* aufweist.

3. Transgene Pflanze nach Anspruch 1, **dadurch gekennzeichnet, dass** es sich bei der doppelsträngigen RNA um miRNA oder siRNA handelt.

4. Transgene Pflanze nach Anspruch 1, **dadurch gekennzeichnet, dass** die erste DNA und die zweite DNA mit mindestens einem Promotor operativ verknüpft sind.

5. Teile einer Pflanze nach Anspruch 1, **dadurch gekennzeichnet, dass** es sich um Samen oder Zellen handelt.

6. Verfahren zur Herstellung einer transgenen Pflanze der Art *Solanum tuberosum,* die eine Resistenz gegenüber einem Oomyzeten der Gattung *Phytophthora* aufweist, umfassend die Schritte:
(i) Herstellen einer transformierten ersten Elternpflanze enthaltend eine erste doppelsträngige DNA, die stabil in das Genom der Elternpflanze integriert ist und die
(a) eine Nukleotidsequenz gemäß der SEQ ID NOS: 38, oder
(b) ein Fragment von mindestens 15 aufeinanderfolgenden Nukleotiden einer Nukleotidsequenz gemäß der SEQ ID NOS: 38, oder
(c) eine Nukleotidsequenz, die komplementär zu einer der Nukleotidsequenzen nach (a) oder (b) ist, oder
(d) eine Nukleotidsequenz, die mit einer der Nukleotidsequenzen nach (a), (b) oder (c) unter stringenten Bedingungen hybridisiert, umfasst;
(ii) Herstellen einer transformierten zweiten Elternpflanze enthaltend eine zweite doppelsträngige DNA, die stabil in das Genom der Elternpflanze integriert ist, wobei die Nukleotidsequenzen des kodierenden Stranges der ersten und zweiten DNA zueinander ganz oder teilweise invers komplementär sind;
(iii) Kreuzen der ersten Elternpflanze mit der zweiten Elternpflanze;
(iv) Auswählen einer solchen Pflanze, in deren Genom die erste DNA und die zweite DNA zur Ausbildung einer Pathogenresistenz gegenüber einem Oomyzeten der Gattung *Phytophthora* integriert sind, so dass ausgehend hiervon eine doppelsträngige RNA gebildet werden kann.

7. Verfahren nach Anspruch 6 **dadurch gekennzeichnet, dass** es sich bei der Resistenz um eine Resistenz gegenüber *Phytophthora infestans* handelt.

8. Verfahren nach Anspruch 6, **dadurch gekennzeichnet, dass** es sich bei der doppelsträngigen RNA um miRNA oder siRNA handelt.

9. Mittel zur äußeren Applikation bei Pflanzen enthaltend doppelsträngige RNA, wobei ein Strang dieser RNA dem Transkript einer doppelsträngigen DNA umfassend
(a) eine Nukleotidsequenz gemäß der SEQ ID NO: 38, oder
(b) ein Fragment von mindestens 15 aufeinanderfolgenden Nukleotiden einer Nukleotidsequenz gemäß der SEQ ID NO: 38, oder
(c) eine Nukleotidsequenz, die komplementär zu einer der Nukleotidsequenzen nach (a) oder (b) ist, oder
(d) eine Nukleotidsequenz, die mit einer der Nukleotidsequenzen nach (a), (b) oder (c) unter stringenten Bedingungen hybridisiert,
entspricht.

10. Mittel nach Anspruch 9 zur Erzeugung einer Resistenz gegenüber einem Oomyzeten der Gattung *Phytophthora,* insbesondere gegenüber *Phytophthora infestans.*
